# EUROPEAN PATENT APPLICATION

(11) **EP 3 904 352 A1**
(43) Date of publication of application: **03.11.2021**
(21) Application number: 19903101.4
(22) Date of filing: 19.12.2019
(51) Int. Cl.: C07D 405/14, C07D 498/04, C07D 471/04, C07D 451/02, A61K 31/444, A61K 31/4545, A61K 31/5377, A61K 31/53, A61K 31/5383, A61K 31/506, A61P 35/00

(54) **SUBSTITUTED ARYL COMPOUND AND PREPARATION METHOD THEREFOR AND USE THEREOF**

(30) Priority: 28.12.2018 CN 201811616858
(71) Applicant: Sichuan Kelun-Biotech Biopharmaceutical Co., Ltd., Chengdu, Sichuan 611138 (CN)
(72) Inventor: LIU, Jinming, CHENGDU, Sichuan 611138 (CN); HE, Ting, CHENGDU, Sichuan 611138 (CN); CAI, Jiaqiang, CHENGDU, Sichuan 611138 (CN); LI, Xiaoyong, CHENGDU, Sichuan 611138 (CN); LUO, Xiaoyong, CHENGDU, Sichuan 611138 (CN); TIAN, Qiang, CHENGDU, Sichuan 611138 (CN); SONG, Hongmei, CHENGDU, Sichuan 611138 (CN); XUE, Tongtong, CHENGDU, Sichuan 611138 (CN); WANG, Lichun, CHENGDU, Sichuan 611138 (CN); WANG, Jingyi, CHENGDU, Sichuan 611138 (CN)
(74) Representative: Nony
(86) International application number: PCT/CN2019/126555
(87) International publication number: WO 2020/135210

(57) **Abstract**

The present application relates to a substituted aryl compound or a pharmaceutically acceptable salt, a stereoisomer, a polymorph, a solvate, a *N*-oxide, an isotope-labeled compound, a metabolite or a prodrug thereof, and a preparation method therefor and use thereof, also relates to a pharmaceutical composition containing the compound and a therapeutic use thereof. The compound or a pharmaceutical composition thereof can inhibit the activity of adenosine A2a receptor, and can be used for treating or preventing a disease related to adenosine A2a receptor, especially for treating a tumor.

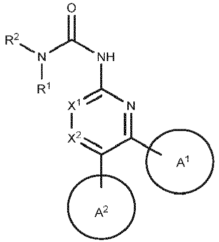

## Description

The present application is based on and claims the benefit of priority from Chinese application No. 201811616858.5, filed on December 28, 2018, the disclosures of which are incorporated herein by reference in its entirety.

### Technical Field

The present application relates to a substituted aryl compound or a pharmaceutically acceptable salt, a stereoisomer, a polymorph, a solvate, a N-oxide, an isotope-labeled compound, a metabolite or a prodrug thereof, a method for preparing the same and a use thereof. The present application also relates to a pharmaceutical composition containing the compound and a therapeutic use thereof.

### Background Art

Adenosine is a signal molecule for inhibiting inflammation and immune response *in vivo*. Two main sources of extracellular adenosine are the transport of intracellular adenosine and the hydrolysis of extracellular adenosine. Adenosine receptors are a kind of G protein-coupled receptors (GPCRs), and this receptor family mainly includes four receptors: A1, A2a, A2b and A3, in which the A2a and A2b receptors are coupled to a Gs protein that activates adenylate cyclase to stimulate the production of intracellular cyclic adenosine monophosphate (cAMP).

The adenosine A2a receptor is expressed on the surface of some cells in the immune system, such as T cells, NK cells, macrophages and dendritic cells. The adenosine produced by tumors can interact with the adenosine A2a receptor on the surface of tumor-infiltrating immunocytes to increase the amount of cAMP in the immunocytes, inhibit the ability of the immunocytes to attack the tumor, and make the body generate immune tolerance, thereby make the tumor cells can escape the body's immune surveillance, which are mainly showed in two aspects: (1) blocking the activation and function of immunocytes that can kill tumor cells; (2) increasing the number of regulatory T cells (T-regs) that can inhibit responses of immunocytes to tumor cells. In the process of tumor cells evolving into cancer cells, they use these mechanisms to escape the surveillance and attack of the immune system to increase their survival rate. The knockout of adenosine A2a receptor gene in mice can enhance the immunity of CD8+ T cells against tumor cells, and significantly inhibit the proliferation of tumor cells. The melanoma or lymphoma cells transplanted into the wild-type mice are easier to grow than those transplanted into the mice with knockout of adenosine A2a receptor gene, and the mice with knockout of adenosine A2a receptor gene have better response to tumor vaccines.

The adenosine A2a receptor is expressed at a high level on immunocytes, the activation of adenosine A2a receptor can promote the body to generate immune tolerance, promote the formation of "immune escape" or "immune suppression" of tumor cells, thereby creating advantageous conditions for tumor development. The adenosine A2a receptor antagonist directly targets the adenosine A2a receptor on the surface of immunocytes, inhibit the activation of the receptor and thereby inhibit the production of cAMP in immunocytes and eliminate the immune suppression of T-cell mediated by the activation of adenosine A2a receptor, and achieve the treatment effect of tumor. Therefore, the adenosine A2a receptor antagonists as tumor treatment drugs have good application prospects in the pharmaceutical industry.

CPI-444 of Corvus Pharmaceuticals is a compound that has an antagonistic effect on adenosine A2a receptor, and its indication is tumor. CPI-444 was used in clinical trials for the treatment of central nervous system diseases heretofore. WO01/62233 discloses that an aminopyridine compound has an antagonistic effect on adenosine A2a receptor, and also discloses that it can be used as a therapeutic agent for Parkinson's disease or senile dementia. WO2011/095625 A1 discloses that an aminotriazine compound has an antagonistic effect on adenosine A2a receptor, and also discloses that it can be used as a therapeutic agent for dyskinesia, stroke, or Parkinson's disease.

Therefore, adenosine A2a receptor antagonists as drugs have good application prospects in the pharmaceutical industry. In order to achieve better tumor treatment effects and meet market needs, there is an urgent need to develop an adenosine A2a receptor antagonist for tumor treatment, especially that with low central nervous system toxic side effects.

### Contents of the Invention

The present application relates to a compound represented by Formula I, a pharmaceutically acceptable salt, a stereoisomer, a polymorph, a solvate, a N-oxide, an isotope-labeled compound, a metabolite or a prodrug thereof, 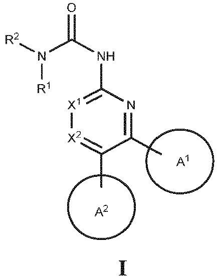 wherein:
X¹ is N or CR³_{;}
X² is N or CR⁴;
and when X¹ is CR³, X² is not N;
ring A¹ is selected from the group consisting of C₃₋₁₀ cycloalkyl, 4- to 12-membered heterocyclyl, C₆₋₁₀ aryl and 5- to 10-membered heteroaryl, the ring A¹ is optionally substituted by one or more substituents independently selected from the group consisting of: halogen, cyano, nitro, C₁₋₆ alkyl, C₁₋₆ haloalkyl, C₃₋₁₀ cycloalkyl, hydroxy, C₁₋₆ alkoxy, amino, C₁₋₆ alkyl-amino-, di(C₁₋₆ alkyl)-amino- and 4- to 12-membered heterocyclyl;
ring A² is selected from the group consisting of C₃₋₁₀ cycloalkyl, 4- to 12-membered heterocyclyl, C₆₋₁₀ aryl and 5- to 10-membered heteroaryl (preferably, the 5- to 10-membered heteroaryl is 5- to 10-membered nitrogen-containing heteroaryl), the ring A² is optionally substituted by one or more substituents independently selected from the group consisting of: halogen, cyano, nitro, C₁₋₆ alkyl, C₁₋₆ haloalkyl, C₃₋₁₀ cycloalkyl, hydroxy, C₁₋₆ alkoxy, amino, C₁₋₆ alkyl-amino-, di(C₁₋₆ alkyl)-amino- and 4- to 12-membered heterocyclyl, and ring A² is not 1-isopropyl-6-oxo-1,6-dihydropyridin-3-yl;
R¹ and R² each are independently selected from the group consisting of hydrogen, C₁₋₆ alkyl, C₃₋₁₀ cycloalkyl, 4- to 12-membered heterocyclyl, C₆₋₁₀ aryl, 5- to 10-membered heteroaryl, 4- to 12-membered heterocyclyl-C₁₋₆ alkyl- and 5- to 10-membered heteroaryl-C₁₋₆ alkyl-, wherein the C₁₋₆ alkyl, C₃₋₁₀ cycloalkyl, 4- to 12-membered heterocyclyl, C₆₋₁₀ aryl, 5- to 10-membered heteroaryl, 4- to 12-membered heterocyclyl-C₁₋₆ alkyl- or 5- to 10-membered heteroaryl-C₁₋₆ alkyl- is optionally substituted by one or more substituents independently selected from the group consisting of: halogen, cyano, hydroxy, C₁₋₆ alkyl, C₁₋₆ haloalkyl, C₁₋₆ alkoxy, C₃₋₁₀ cycloalkyl and 4- to 12-membered heterocyclyl; or,
R¹ and R² together with the nitrogen atom linked to them form a 4- to 12-membered nitrogen-containing heterocyclyl (preferably, the 4- to 12-membered nitrogen-containing heterocyclyl comprises one or more heteroatoms selected from the group consisting of nitrogen atom and oxygen atom), the 4- to 12-membered nitrogen-containing heterocyclyl is optionally substituted by one or more substituents independently selected from the group consisting of: halogen, cyano, hydroxy, C₁₋₆ alkyl, C₁₋₆ haloalkyl, C₁₋₆ alkoxy, C₁₋₆ alkoxy-C₁₋₆ alkyl-, C₁₋₆ alkoxy-C₁₋₆ alkoxy-, C₃₋₁₀ cycloalkyl and 4- to 12-membered heterocyclyl;
R³ and R⁴ each are independently selected from the group consisting of hydrogen, halogen, cyano, hydroxy, amino, C₁₋₆ alkyl, C₁₋₆ haloalkyl, C₁₋₆ alkoxy, C₃₋₆ cycloalkyl, C₁₋₆ alkyl-amino- and di(C₁₋₆ alkyl)-amino-, and The compound is not the following compounds:
   1-(5,6-diphenyl-1,2,4-triazin-3-yl)-3-phenylurea,
   1-(5-(2-bromo-5-hydroxyphenyl)-4-ethyl-6-phenylpyrimidin-2-yl)urea,
   1-(5-(2-chloro-5-hydroxyphenyl)-4-ethyl-6-phenylpyrimidin-2-yl)urea,
   1-(3,5-di(trifluoromethyl)phenyl)-3-(4-(3-hydroxy-5-methylphenyl)-5-(pyridin-4-yl)pyrimidin-2-yl)urea,
   1-(4-chloro-3-(trifluoromethyl)phenyl)-3-(4-(3-hydroxy-5-methylphenyl)-5-(pyridin-4-yl)pyrimidin-2-yl)urea,
   1-(3,5-di(trifluoromethyl)phenyl)-3-(4-(3-methoxy-5-metliylphenyl)-5-(pyridin-4-yl)pyrimidin-2-yl)urea,
   1-(4-chloro-3-(trifluoromethyl)phenyl)-3-(4-(3-methoxy-5-methylphenyl)-5-(pyridin-4-yl)pyrimidin-2-yl)urea,
   1-(4-chloro-3-(trifluoromethyl)phenyl)-3-(4-(2-hydroxyphenyl)-5-(4-methoxyphenyl)pyrimidin-2-yl)urea.

In some embodiments, in the compound represented by Formula I, a pharmaceutically acceptable salt, a stereoisomer, a polymorph, a solvate, a *N*-oxide, an isotope-labeled compound, a metabolite or a prodrug thereof according to the present application, R³ and R⁴ each are independently selected from the group consisting of hydrogen, halogen, cyano, hydroxy, amino, C₁₋₆ alkyl, C₁₋₆ haloalkyl, C₁₋₆ alkoxy, C₁₋₆ alkyl-amino- and di(C₁₋₆ alkyl)-amino-.

In some embodiments, in the compound represented by Formula I, a pharmaceutically acceptable salt, a stereoisomer, a polymorph, a solvate, a N-oxide, an isotope-labeled compound, a metabolite or a prodrug thereof according to the present application, the compound is not 1-(6-(2-chloro-6-methylpyridin-4-yl)-5-(4-fluorophenyl)-1,2,4-triazin-3-yl)urea.

In some embodiments, the compound according to the present application has the structure represented by Formula IIa, Formula IIb or Formula IIc, 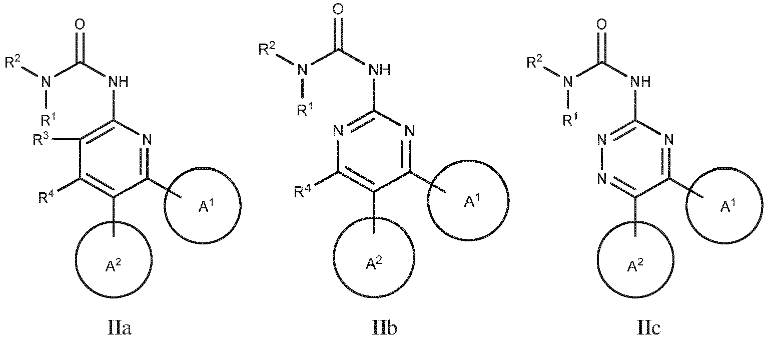 wherein: ring A¹, ring A², R¹, R², R³ and R⁴ have the definitions as described in the present application.

In some embodiments, in the compound according to the present application has the structure represented by Formula IIa, Formula IIb or Formula IIc, ring A¹, ring A², R³ and R⁴ have the definitions as described in the present application,

R¹ and R² each are independently selected from the group consisting of hydrogen, C₁₋₆ alkyl, C₃₋₈ cycloalkyl, 4- to 8-membered heterocyclyl, phenyl, 5- to 6-membered heteroaryl, 4- to 7-membered heterocyclyl-C₁₋₆ alkyl-or 5- to 6-membered heteroaryl-C₁₋₆ alkyl-, wherein the C₁₋₆ alkyl, C₃₋₈ cycloalkyl, 4- to 8-membered heterocyclyl, phenyl, 5- to 6-membered heteroaryl, 4- to 7-membered heterocyclyl-C₁₋₆ alkyl- or 5- to 6-membered heteroaryl-C₁₋₆ alkyl- is optionally substituted by one or more substituents independently selected from the group consisting of: halogen, cyano, hydroxy, C₁₋₆ alkyl, C₁₋₆ haloalkyl, C₁₋₆ alkoxy, C₃₋₆ cycloalkyl and 4- to 7-membered heterocyclyl; or,

R¹ and R² together with the nitrogen atom linked to them form a 4- to 12-membered nitrogen-containing heterocyclyl (e.g., 4- to 6-membered nitrogen-containing heterocyclyl), the 4- to 12-membered nitrogen-containing heterocyclyl (e.g., 4- to 6-membered nitrogen-containing heterocyclyl) is optionally substituted by one or more substituents independently selected from the group consisting of: halogen, cyano, hydroxy, C₁₋₆ alkyl, C₁₋₆ haloalkyl, C₁₋₆ alkoxy, C₁₋₆ alkoxy-C₁₋₆ alkyl-, C₁₋₆ alkoxy-C₁₋₆ alkoxy-, C₃₋₁₀ cycloalkyl and 4- to 12-membered heterocyclyl.

In some embodiments, R¹ and R² together with the nitrogen atom linked to them form a 4- to 12-membered nitrogen-containing heterocyclyl (e.g., 4- to 6-membered nitrogen-containing heterocyclyl), the 4- to 12-membered nitrogen-containing heterocyclyl comprises one or more heteroatoms selected from the group consisting of nitrogen atom and oxygen atom, the 4- to 12-membered nitrogen-containing heterocyclyl (e.g., 4-to 6-membered nitrogen-containing heterocyclyl) is optionally substituted by one or more substituents independently selected from the group consisting of: halogen, cyano, hydroxy, C₁₋₆ alkyl, C₁₋₆ haloalkyl, C₁₋₆ alkoxy, C₁₋₆ alkoxy-C₁₋₆ alkyl-, C₁₋₆ alkoxy-C₁₋₆ alkoxy-, C₃₋₁₀ cycloalkyl and 4- to 12-membered heterocyclyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A¹ is selected from the group consisting of phenyl and 5- to 6-membered heteroaryl, the ring A¹ is optionally substituted by one or more substituents independently selected from the group consisting of: halogen, cyano, nitro, C₁₋₆ alkyl, C₁₋₆ haloalkyl, C₃₋₆ cycloalkyl and 4- to 7-membered heterocyclyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A¹ is selected from the group consisting of phenyl, furan-2-yl and furan-3-yl, the ring A¹ is optionally substituted by one or more substituents independently selected from the group consisting of: fluoro, chloro, bromo, cyano, nitro, methyl, ethyl, n-propyl, isopropyl, trifluoromethyl, cyclopropyl, cyclobutyl, cyclopentyl.

In some embodiments, in the compound, a pharmaceutically acceptable salt, a stereoisomer, a polymorph, a solvate, a N-oxide, an isotope-labeled compound, a metabolite or a prodrug thereof according to the present application, ring A¹ is selected from the group consisting of phenyl and furan-2-yl, the ring A¹ is optionally substituted by one or more substituents independently selected from the group consisting of: fluoro, chloro, bromo, cyano, nitro, methyl, ethyl, n-propyl, isopropyl, trifluoromethyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A¹ is selected from the group consisting of phenyl, 4-fluorophenyl, 4-ethylphenyl, 4-nitrophenyl, 2-fluorophenyl, 3-fluorophenyl, 4-methylphenyl, 2-methylphenyl, 3-methylphenyl, 5-methyl-furan-2-yl, 3-methyl-furan-2-yl and 4-methyl-furan-2-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A¹ is selected from the group consisting of phenyl, 4-fluorophenyl, 4-ethylphenyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A¹ is selected from the group consisting of 4-nitrophenyl, 2-fluorophenyl, 3-fluorophenyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A¹ is selected from the group consisting of 4-methylphenyl, 2-methylphenyl, 3-methylphenyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A¹ is selected from the group consisting of 5-methyl-furan-2-yl, 3-methyl-furan-2-yl and 4-methyl-furan-2-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A¹ is selected from the group consisting of 4-fluorophenyl and 5-methyl-furan-2-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A¹ is 4-fluorophenyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A¹ is 5-methyl-furan-2-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is selected from the group consisting of C₆₋₁₀ aryl and 5- to 10-membered heteroaryl, the ring A² is optionally substituted by one or more substituents independently selected from the group consisting of: halogen, cyano, nitro, C₁₋₆ alkyl, C₁₋₆ haloalkyl, C₃₋₆ cycloalkyl and 4- to 7-membered heterocyclyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is selected from the group consisting of C₆₋₁₀ aryl and 5- to 10-membered nitrogen-containing heteroaryl, the ring A² is optionally substituted by one or more substituents independently selected from the group consisting of: halogen, cyano, nitro, C₁₋₆ alkyl, C₁₋₆ haloalkyl, C₃₋₆ cycloalkyl and 4- to 7-membered heterocyclyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is selected from the group consisting of phenyl, pyridyl and quinazolinyl, the ring A² is optionally substituted by one or more substituents independently selected from the group consisting of: halogen, cyano, nitro, C₁₋₆ alkyl, C₁₋₆ haloalkyl, C₃₋₆ cycloalkyl and 4- to 7-membered heterocyclyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is selected from the group consisting of phenyl, pyridin-2-yl, pyridin-3-yl, pyridin-4-yl, quinazolin-2-yl, quinazolin-4-yl, quinazolin-5-yl, quinazolin-6-yl, quinazolin-7-yl and quinazolin-8-yl, the ring A² is optionally substituted by one or more substituents independently selected from the group consisting of: halogen, cyano, nitro, C₁₋₆ alkyl, C₁₋₆ haloalkyl, C₃₋₆ cycloalkyl and 4- to 7-membered heterocyclyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is selected from the group consisting of phenyl, pyridin-4-yl and quinazolin-6-yl, the ring A² is optionally substituted by one or more substituents independently selected from the group consisting of: fluoro, chloro, bromo, cyano, nitro, methyl, ethyl, n-propyl, isopropyl, and trifluoromethyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is selected from the group consisting of phenyl, pyridin-4-yl and quinazolin-6-yl, the ring A² is optionally substituted by one or more substituents independently selected from the group consisting of: fluoro, chloro, bromo, methyl, ethyl, n-propyl, isopropyl, and trifluoromethyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is selected from the group consisting of phenyl, pyridin-4-yl and quinazolin-6-yl, the ring A² is optionally substituted by one or more substituents independently selected from the group consisting of: chloro, methyl, and trifluoromethyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is selected from the group consisting of pyridin-4-yl and quinazolin-6-yl, the ring A² is optionally substituted by one or more substituents independently selected from the group consisting of: chloro and methyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is phenyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is pyridin-4-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is quinazolin-6-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, the ring A² is optionally substituted by one or more substituents independently selected from the group consisting of: fluoro and chloro.

In some embodiments, in the compound, a pharmaceutically acceptable salt, a stereoisomer, a polymorph, a solvate, a *N*-oxide, an isotope-labeled compound, a metabolite or a prodrug thereof according to the present application, the ring A² is optionally substituted by one or more substituents independently selected from the group consisting of: bromo, cyano, nitro and methyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, the ring A² is optionally substituted by one or more substituents independently selected from the group consisting of: ethyl and n-propyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, the ring A² is optionally substituted by one or more substituents independently selected from the group consisting of: isopropyl and trifluoromethyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is selected from the group consisting of 3-chloro-5-methylphenyl, 2-chloro-6-methylpyridin-4-yl, 2-chloro-6-(trifluoromethyl)-pyridin-4-yl and 4-methylquinazolin-6-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is 4-methylquinazolin-6-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is 2-chloro-6-metliylpyridin-4-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, ring A² is 2-chloro-6-(trifluoromethyl)-pyridin-4-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² each are independently selected from the group consisting of hydrogen, C₁₋₆ alkyl, C₃₋₆ cycloalkyl, 4- to 7-membered heterocyclyl, phenyl, 5- to 6-membered heteroaryl, 4- to 7-membered heterocyclyl-C₁₋₆ alkyl- and 5- to 6-membered heteroaryl-C₁₋₆ alkyl-, wherein the C₁₋₆ alkyl, C₃₋₆ cycloalkyl, 4-to 7-membered heterocyclyl, phenyl, 5- to 6-membered heteroaryl, 4- to 7-membered heterocyclyl-C₁₋₆ alkyl- or 5- to 6-membered heteroaryl-C₁₋₆ alkyl- is optionally substituted by one or more substituents independently selected from the group consisting of: halogen, cyano, hydroxy, C₁₋₆ alkyl, C₁₋₆ haloalkyl, C₁₋₆ alkoxy, C₃₋₆ cycloalkyl and 4- to 7-membered heterocyclyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² each are independently selected from the group consisting of hydrogen, C₁₋₆ alkyl, C₃₋₆ cycloalkyl, 4- to 7-membered heterocyclyl, phenyl, 5- to 6-membered heteroaryl, 4- to 7-membered heterocyclyl-C₁₋₆ alkyl- and 5- to 6-membered heteroaryl-C₁₋₆ alkyl-, wherein the C₁₋₆ alkyl, C₃₋₆ cycloalkyl, 4-to 7-membered heterocyclyl, phenyl, 5- to 6-membered heteroaryl, 4- to 7-membered heterocyclyl-C₁₋₆ alkyl- or 5- to 6-membered heteroaryl-C₁₋₆ alkyl- is optionally substituted by one or more substituents independently selected from the group consisting of: fluoro, chloro, bromo, cyano, hydroxy, methyl, ethyl, n-propyl, isopropyl, trifluoromethyl, methoxy, ethoxy, propoxy, cyclopropyl, cyclobutyl, cyclopentyl, cyclohexyl, piperidin-1-yl, piperidin-2-yl, piperidin-3-yl and piperidin-4-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² each are independently selected from the group consisting of hydrogen, C₁₋₆ alkyl, phenyl and 5- to 6-membered heteroaryl, wherein the C₁₋₆ alkyl, phenyl or 5- to 6-membered heteroaryl is optionally substituted by one or more substituents independently selected from the group consisting of: fluoro, chloro, bromo, cyano, hydroxy, methyl, ethyl, n-propyl, isopropyl, trifluoromethyl, methoxy, ethoxy, propoxy, cyclopropyl, cyclobutyl, cyclopentyl, cyclohexyl, piperidin-1-yl, piperidin-2-yl, piperidin-3-yl and piperidin-4-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² each are independently selected from the group consisting of hydrogen, methyl, ethyl, n-propyl, isopropyl, n-butyl, sec-butyl, isobutyl, tert-butyl, cyclopropyl, cyclobutyl, cyclopentyl, cyclohexyl, piperidinyl, phenyl, pyridyl, pyridin-2-ylmethyl, pyridin-2-ylethyl, pyridin-2-ylpropyl, methoxyethyl and 2-hydroxy-2-methyl-propyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² each are independently selected from the group consisting of hydrogen, methyl, methoxyethyl, 2-hydroxy-2-methyl-propyl, phenyl and pyridin-2-ylmethyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² each are independently selected from the group consisting of 2-hydroxy-2-methyl-propyl, phenyl and pyridin-2-ylmethyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² each are independently hydrogen, methyl or 2-methoxyethyl.

In some embodiments, R¹ is hydrogen or methyl, and R² is hydrogen, methyl, phenyl, pyridin-2-ylmethyl, 2-hydroxy-2-methyl-propyl or 2-methoxyethyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ is hydrogen or methyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, wherein: R² is methyl, phenyl, pyridin-2-ylmethyl, 2-hydroxy-2-methyl-propyl or 2-methoxyethyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ is hydrogen or methyl, and R² is hydrogen, methyl, phenyl, pyridin-2-ylmethyl, 2-hydroxy-2-methyl-propyl or 2-methoxyethyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ is hydrogen or methyl, and R² is methyl, pyridin-2-ylmethyl, 2-hydroxy-2-methyl-propyl or 2-methoxyethyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a 4- to 7-membered nitrogen-containing heterocyclyl, the 4- to 7-membered nitrogen-containing heterocyclyl is optionally substituted by one or more substituents independently selected from the group consisting of: halogen, cyano, hydroxy, C₁₋₆ alkyl, C₁₋₆ haloalkyl, C₁₋₆ alkoxy, C₁₋₆ alkoxy-C₁₋₆ alkyl-, C₁₋₆ alkoxy-C₁₋₆ alkoxy-, C₃₋₆ cycloalkyl and 4- to 7-membered heterocyclyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a 4- to 7-membered nitrogen-containing heterocyclyl (e.g., the 4- to 7-membered nitrogen-containing heterocyclyl comprises one or more heteroatoms selected from the group consisting of oxygen atom and nitrogen atom), the 4- to 7-membered nitrogen-containing heterocyclyl is optionally substituted by one or more substituents independently selected from the group consisting of: halogen, cyano, hydroxy, C₁₋₆ alkyl, C₁₋₆ haloalkyl, C₁₋₆ alkoxy, C₁₋₆ alkoxy-C₁₋₆ alkyl-, C₁₋₆ alkoxy-C₁₋₆ alkoxy- and 4- to 7-membered heterocyclyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a piperidinyl, morpholinyl or piperazinyl, the piperidinyl, morpholinyl or piperazinyl is optionally substituted by one or more substituents independently selected from the group consisting of: fluoro, chloro, bromo, cyano, hydroxy, methyl, ethyl, n-propyl, isopropyl, n-butyl, sec-butyl, isobutyl, tert-butyl, cyclopropyl, cyclobutyl, cyclopentyl, cyclohexyl, trifluoromethyl, methoxy, ethoxy, propoxy, piperidin-1-yl, piperidin-1-yl, piperidin-2-yl, piperidin-3-yl and piperidin-4-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a piperidinyl or morpholinyl, the piperidinyl or morpholinyl is optionally substituted by one or more substituents independently selected from the group consisting of: fluoro, chloro, bromo, cyano, hydroxy, methyl, ethyl, n-propyl, isopropyl, n-butyl, sec-butyl, isobutyl, tert-butyl, cyclopropyl, cyclobutyl, cyclopentyl, cyclohexyl, trifluoromethyl, methoxy, ethoxy, propoxy, piperidin-1-yl, piperidin-1-yl, piperidin-2-yl, piperidin-3-yl and piperidin-4-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom liked to them form a piperazinyl, the piperazinyl is optionally substituted by one or more substituents independently selected from the group consisting of: fluoro, chloro, bromo, cyano, hydroxy, methyl, ethyl, n-propyl, isopropyl, n-butyl, sec-butyl, isobutyl, tert-butyl, cyclopropyl, cyclobutyl, cyclopentyl, cyclohexyl, trifluoromethyl, methoxy, ethoxy, propoxy, piperidin-1-yl, piperidin-1-yl, piperidin-2-yl, piperidin-3-yl and piperidin-4-yl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a piperidinyl, morpholinyl, 4-cyanopiperidinyl, 4-methoxypiperidinyl or 1,4'-bipiperidinyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a 4-methylpiperazinyl, 2-methylmorpholinyl, or 2,6-dimethylmorpholinyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a piperidinyl, 4-cyanopiperidinyl, or 4-methoxypiperidinyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a 1,4'-bipiperidinyl, or 4-methylpiperazinyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a morpholinyl, the morpholinyl is optionally substituted by one or more substituents independently selected from the group consisting of: methyl, ethyl, n-propyl, isopropyl, n-butyl, sec-butyl, isobutyl and tert-butyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a morpholinyl, 2-methylmorpholinyl or 2,6-dimethylmorpholinyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a morpholinyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a 2-methylmorpholinyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R¹ and R² together with the nitrogen atom linked to them form a 2,6-dimethylmorpholinyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ and R⁴ each are independently selected from the group consisting of hydrogen, halogen, cyano, hydroxy, amino, C₁₋₆ alkyl, C₁₋₆ haloalkyl, C₁₋₆ alkoxy, C₃₋₆ cycloalkyl, C₁₋₆ alkyl-amino- and di(C₁₋₆ alkyl)-amino-.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ and R⁴ each are independently selected from the group consisting of hydrogen, halogen, cyano, hydroxy, amino, C₁₋₆ alkyl, C₁₋₆ haloalkyl, C₁₋₆ alkoxy, C₁₋₆ alkyl-amino- and di(C₁₋₆ alkyl)-amino-.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ and R⁴ each are independently selected from the group consisting of hydrogen, fluoro, chloro, bromo, cyano, hydroxy, amino, methyl, ethyl, n-propyl, isopropyl, trifluoromethyl, methoxy, ethoxy, propoxy, methylamino, ethylamino, dimethylamino, diethylamino, cyclopropyl, cyclobutyl, cyclopentyl and cyclohexyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ and R⁴ each are independently selected from the group consisting of hydrogen, fluoro, chloro, bromo, cyano, hydroxy, amino, methyl, ethyl, n-propyl, isopropyl, trifluoromethyl, methoxy, ethoxy, propoxy, methylamino, ethylamino, dimethylamino and diethylamino.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ is selected from the group consisting of hydrogen, fluoro, chloro, bromo, cyano, hydroxy, amino, methyl, ethyl, n-propyl, isopropyl, trifluoromethyl, methoxy, ethoxy, propoxy, methylamino, ethylamino, dimethylamino, diethylamino, cyclopropyl, cyclobutyl, cyclopentyl and cyclohexyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ is selected from the group consisting of hydrogen, fluoro, chloro, bromo, cyano, hydroxy, amino, methyl, ethyl, n-propyl, isopropyl, trifluoromethyl, methoxy, ethoxy, propoxy, methylamino, ethylamino, dimethylamino and diethylamino.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ is selected from the group consisting of hydrogen, fluoro, chloro, bromo, methyl, ethyl, n-propyl, isopropyl, trifluoromethyl, methoxy, ethoxy, propoxy, methylamino, ethylamino, dimethylamino and diethylamino.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ is hydrogen, fluoro or chloro.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ is selected from the group consisting of bromo, methyl, ethyl and n-propyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ is selected from the group consisting of isopropyl, trifluoromethyl, methoxy and ethoxy.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ is selected from the group consisting of propoxy, methylamino and ethylamino.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ is selected from the group consisting of dimethylamino and diethylamino.

In some embodiments, in the compound, a pharmaceutically acceptable salt, a stereoisomer, a polymorph, a solvate, a *N*-oxide, an isotope-labeled compound, a metabolite or a prodrug thereof according to the present application, R³ is fluoro.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is selected from the group consisting of hydrogen, fluoro, chloro, bromo, cyano, hydroxy, amino, methyl, ethyl, n-propyl, isopropyl, trifluoromethyl, methoxy, ethoxy, propoxy, methylamino, ethylamino, dimethylamino, diethylamino, cyclopropyl, cyclobutyl, cyclopentyl and cyclohexyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is selected from the group consisting of hydrogen, fluoro, chloro, bromo, cyano, hydroxy, amino, methyl, ethyl, n-propyl, isopropyl, trifluoromethyl, methoxy, ethoxy, propoxy, methylamino, ethylamino, dimethylamino and diethylamino.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is selected from the group consisting of hydrogen, fluoro, chloro, bromo, methyl, ethyl, n-propyl, isopropyl, trifluoromethyl, methoxy, ethoxy, propoxy, methylamino, ethylamino, dimethylamino, diethylamino, cyclopropyl, cyclobutyl, cyclopentyl and cyclohexyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is selected from the group consisting of hydrogen, fluoro, chloro, bromo, methyl, ethyl, n-propyl, isopropyl, trifluoromethyl, methoxy, ethoxy, propoxy, methylamino, ethylamino, dimethylamino and diethylamino.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is selected from the group consisting of hydrogen, fluoro and chloro.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is selected from the group consisting of bromo, methyl, ethyl and n-propyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is selected from the group consisting of isopropyl, trifluoromethyl, methoxy and ethoxy.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is selected from the group consisting of propoxy, methylamino and ethylamino.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is selected from the group consisting of dimethylamino and diethylamino.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is hydrogen, chloro, cyano or cyclopropyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is hydrogen.

In some embodiments, in the compound, a pharmaceutically acceptable salt, a stereoisomer, a polymorph, a solvate, a *N*-oxide, an isotope-labeled compound, a metabolite or a prodrug thereof according to the present application, R⁴ is chloro, cyano or cyclopropyl.

In some embodiments, in the compound, a pharmaceutically acceptable salt, a stereoisomer, a polymorph, a solvate, a *N*-oxide, an isotope-labeled compound, a metabolite or a prodrug thereof according to the present application, the compound is selected from the group consisting of:

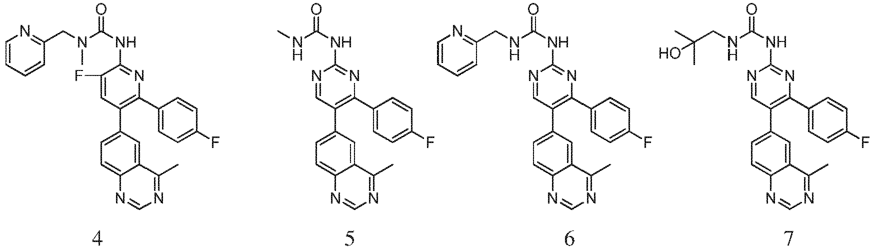

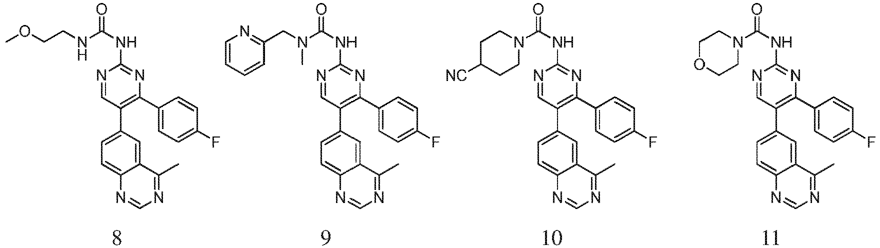

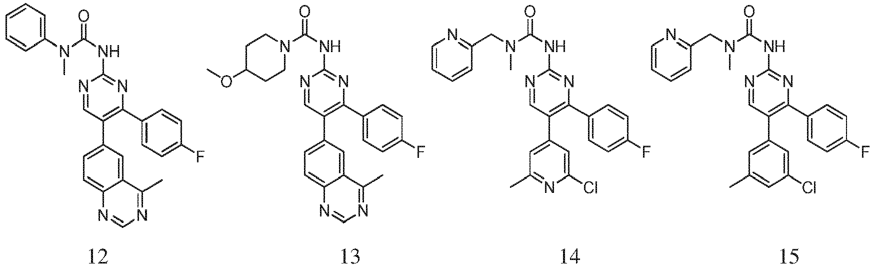

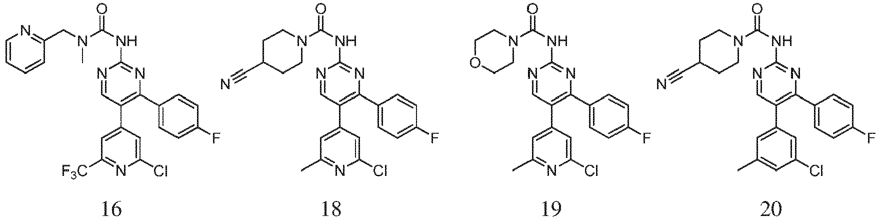

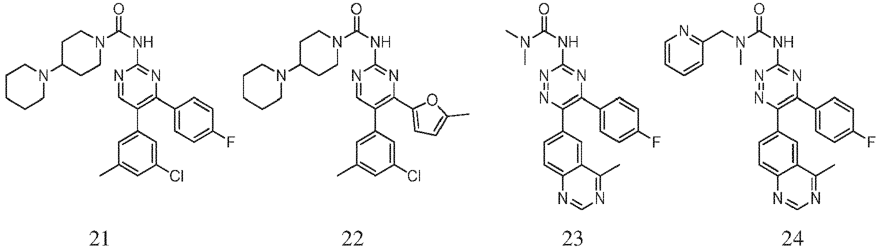

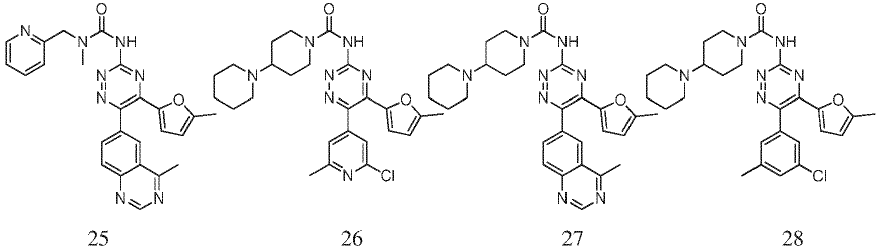

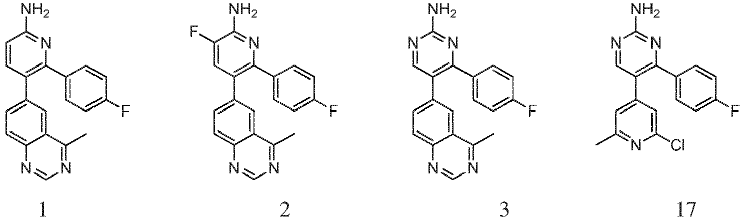

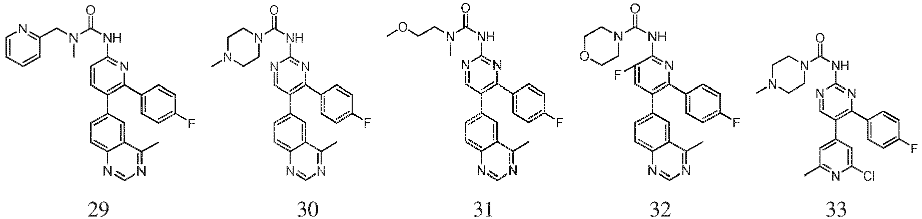

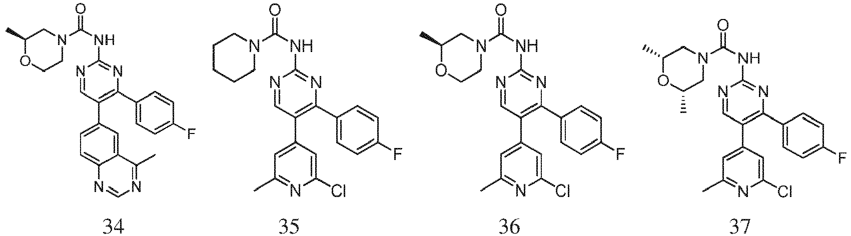

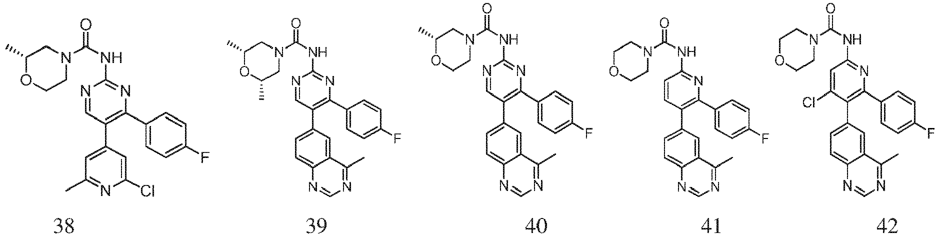

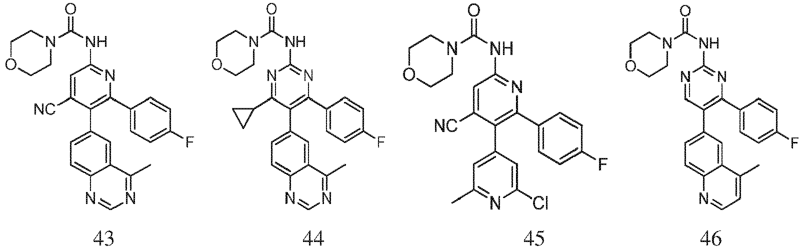

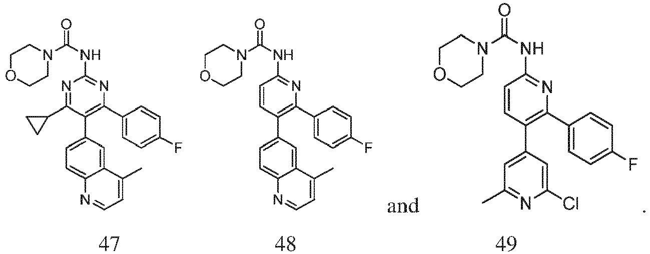

The atom in the compound of the present application can be replaced by its isotope. For example, ¹²C can be replaced by its isotope ¹³C or ¹⁴C; ¹H can be replaced by ²H (D, deuterium) or ³H (T, tritium), etc. The present application comprises the compound represented by Formula I and the isotope-labeled compound obtained after any atom in the compound represented by Formula I is replaced by its isotope.

The present application also relates to a method for preparing the compound, which comprises the following synthetic routes: 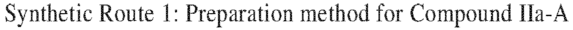 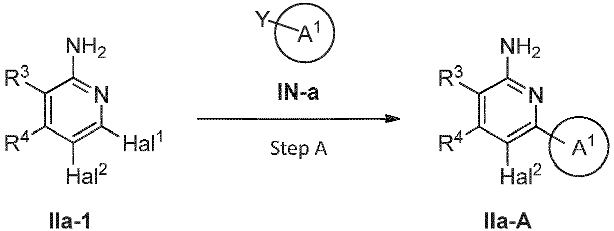 wherein: Hal¹ and Hal² each independently are the same or different halogen, such as Cl, Br or I; Y is a boric acid or boric acid ester group, preferably -B(OH)₂ or 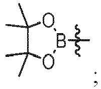 the remaining groups are as defined above in the present application.

### Step A: Reaction of Compound IIa-1 and Compound IN-a to obtain Compound IIa-A

Compound IIa-1 and Compound IN-a undergo a coupling reaction to obtain Compound IIa-A. The coupling reaction is preferably carried out in the presence of a metal catalyst and a base. Preferably, the metal catalyst is a palladium metal catalyst, such as tetrakis(triphenylphosphine)palladium, [1,1'-bis(diphenylphosphino)ferrocene]dichloropalladium, [1,1'-bis(diphenylphosphino)ferrocene] palladium dichloride dichloromethane complex, bis(triphenylphosphine)palladium dichloride, palladium acetate, preferably tetrakis(triphenylphosphine)palladium. The base is an inorganic base, such as potassium carbonate, cesium carbonate, sodium carbonate, sodium bicarbonate, potassium bicarbonate, preferably sodium carbonate. Preferably, the coupling reaction is carried out in a suitable organic solvent or a mixed solvent of an organic solvent and water. The organic solvent can be 1,4-dioxane, *N,N*-dimethylformamide, methanol, ethanol, toluene or a mixed solvent of the above-mentioned organic solvent and water, for example a mixed solvent of toluene, methanol and water. Preferably, the coupling reaction is carried out in a suitable protective atmosphere (e.g., a nitrogen atmosphere). Preferably, the coupling reaction is carried out at a suitable temperature, and the temperature can be 70-100°C, preferably 95°C. Preferably, the coupling reaction is carried out for a suitable time, and the time can be 1-24 hours, For example, 11 hours. 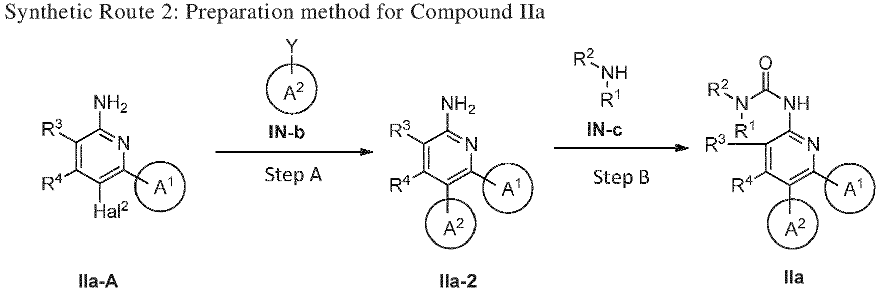 wherein: Hal² is halogen, such as Cl, Br or I, preferably Br; Y is a boric acid or boric acid ester group, preferably -B(OH)₂ or 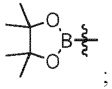 the remaining groups are as defined above in the present application.

### Step A: Reaction of Compound IIa-A and Compound IN-b to obtain Compound IIa-2

Compound IIa and Compound IN-b undergo a coupling reaction to obtain Compound IIa-2. The coupling reaction is preferably carried out in the presence of a metal catalyst and a base. Preferably, the metal catalyst is a palladium metal catalyst, such as tetrakis(triphenylphosphine)palladium, [1,1'-bis(diphenylphosphino)ferrocene]dichloropalladium, [1,1'-bis(diphenylphosphino)ferrocene] palladium dichloride dichloromethane complex, bis(triphenylphosphine)palladium dichloride, palladium acetate, preferably tetrakis(triphenylphosphine)palladium. The base is an inorganic base, such as potassium carbonate, cesium carbonate, sodium carbonate, sodium bicarbonate, potassium bicarbonate, preferably potassium carbonate. Preferably, the coupling reaction is carried out in a suitable organic solvent, the organic solvent can be 1,4-dioxane, *N,N*-dimethylformamide, methanol, ethanol, toluene or a mixed solvent of the above-mentioned organic solvent and water, for example, a mixed solvent of 1,4-dioxane and water. Preferably, the coupling reaction is carried out in a suitable protective atmosphere (e.g., a nitrogen atmosphere). Preferably, the coupling reaction is carried out at a suitable temperature, and the temperature can be 70-100°C, preferably 95°C. Preferably, the coupling reaction is carried out for a suitable time, and the time may be 1-24 hours, preferably 12 hours.

### Step B: Reaction of Compound IIa-2 and Compound IN-c to obtain Compound of Formula IIa

Compound IIa-2 can react with triphosgene to convert amino into isocyanate, and then react with compound IN-c to obtain the compound of Formula IIa. The reaction is preferably carried out in a suitable organic solvent and a suitable base. The organic solvent can be selected from the group consisting of linear or cyclic ethers (e.g., tetrahydrofuran or diethyl ether, etc.), 1,4-dioxane, dichloromethane, dimethyl sulfoxide and any combination thereof, preferably tetrahydrofuran. The base is an organic base, such as *N,N*-diisopropylethylamine, triethylamine, pyridine, 4-dimethylaminopyridine, preferably *N,N-*diisopropylethylamine. The reaction is preferably carried out at a suitable temperature. The temperature can be -40°C to 60°C, such as 0-25°C. The reaction is preferably carried out for a suitable time, such as 0.5-2 hours.

Compound IIa-2 can also first react with phenyl chloroformate in a solvent and in the presence of a base, and then react with compound IN-c to obtain the compound of Formula IIa. The used base can be any base as long as it does not inhibit the reaction, and is usually an organic base, such as *N,N-*diisopropylethylamine, triethylamine, pyridine and 4-dimethylaminopyridine, preferably *N,N-*diisopropylethylamine. The used solvent can be any solvent as long as it does not inhibit the reaction and can dissolve the raw materials to a certain extent, such as dichloromethane, tetrahydrofuran, 1,4-dioxane, diethyl ether, acetonitrile, etc., preferably tetrahydrofuran. The reaction temperature is usually 0°C to 60°C, preferably room temperature. The reaction time is usually 1-12 hours, preferably 4 hours. 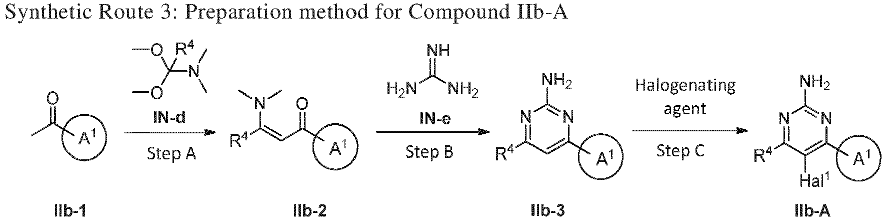 wherein: Hal¹ is halogen, such as Cl, Br or I, preferably Br; the remaining groups are as defined above in the present application.

### Step A: Reaction of Compound IIb-1 and Compound IN-d to obtain Compound IIb-2

Compound IIb-1 reacts with Compound IN-d to obtain Compound IIb-2. This reaction is preferably carried out under a solvent-free condition, and this reaction can also be carried out in a solvent that does not inhibit the reaction, such as 1,4-dioxane, *N,N*-dimethylformamide, tetrahydrofuran, N-methylpyrrolidone, benzene or toluene, etc. The reaction temperature is usually room temperature to 120°C, preferably 80°C. The reaction time is usually 6-24 hours, preferably 12 hours.

### Step B: Reaction of Compound IIb-2 and IN-e to obtain Compound IIb-3

Compound IIb-2 reacts with Compound IN-e in a solvent and in the presence of a base to obtain Compound IIb-3. The used guanidine (IN-e) and an acid such as hydrochloric acid, hydrobromic acid, sulfuric acid or acetic acid can form a salt, preferably a hydrochloride. The used base can be any base as long as it does not inhibit the reaction, including alkali metal carbonates such as potassium carbonate, cesium carbonate, sodium carbonate, or alkali metal alkoxides such as sodium methoxide, sodium ethoxide, potassium tert-butoxide, preferably potassium carbonate. The used solvent can be any solvent as long as it does not inhibit the reaction and can dissolve the raw materials and bases to a certain extent, such as *N,N*-dimethylformamide, N-methylpyrrolidone, dimethylsulfoxide and ethanol, etc., preferably ethanol. The reaction temperature is usually room temperature to 120°C, preferably 80°C. The reaction time is usually 12-36 hours, preferably 20 hours.

### Step C: Reaction of Compound IIb-3 and halogenating agent to obtain Compound IIb-A

Compound IIb-3 reacts with a halogenating agent in a solvent to obtain Compound IIb-A. The used halogenating agent can be *N*-bromosuccinimide or bromine, preferably *N*-bromosuccinimide. The used solvent can be any solvent as long as it does not inhibit the reaction and can dissolve the raw materials to a certain extent, such as *N,N*-dimethylformamide, N-methylpyrrolidone, methanol, ethanol, tetrahydrofuran, 1,4-dioxane and dimethoxyethane, preferably *N,N*-dimethylformamide. The reaction temperature is usually -20°C to room temperature, preferably 0°C to room temperature. The reaction time is usually 1-24 hours, preferably 12 hours. 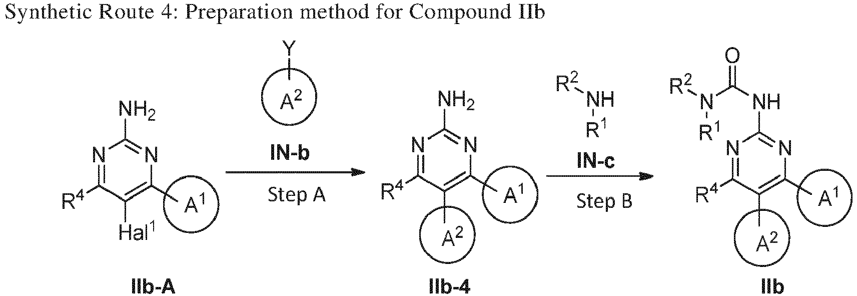 wherein: Hal¹ is halogen, such as Cl, Br or I, preferably Br; Y is a boric acid or boric acid ester group, preferably -B(OH)₂ or 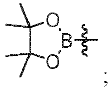 the remaining groups are as defined above in the present application.

### Step A: Reaction of Compound IIb-A and Compound IN-b to obtain Compound IIb-4

Compound IIb-A and Compound IN-b undergo a coupling reaction to obtain Compound IIb-4. The coupling reaction is preferably carried out in the presence of a metal catalyst and a base. Preferably, the metal catalyst is a palladium metal catalyst, such as tetrakis(triphenylphosphine)palladium, [1,1'-bis(diphenylphosphino)ferrocene]dichloropalladium, [1,1'-bis(diphenylphosphino)ferrocene] palladium dichloride dichloromethane complex, bis(triphenylphosphine)palladium dichloride, palladium acetate, preferably [1,1'-bis(diphenylphosphino)ferrocene]dichloropalladium. The base is an inorganic base, such as potassium carbonate, cesium carbonate, sodium carbonate, sodium bicarbonate, potassium bicarbonate, preferably potassium carbonate. Preferably, the coupling reaction is carried out in a suitable organic solvent or a mixed solvent of an organic solvent and water, the organic solvent can be 1,4-dioxane, *N,N-*dimethylformamide or a mixed solvent of the above-mentioned organic solvent and water, for example, a mixed solvent of 1,4-dioxane and water. Preferably, the coupling reaction is carried out in a suitable protective atmosphere (e.g., a nitrogen atmosphere). Preferably, the coupling reaction is carried out at a suitable temperature, the temperature can be 70-100°C, preferably 100°C. The reaction time is usually 1-8 hours, preferably 4 hours.

### Step B: Reaction of Compound IIb-4 with compound IN-c to obtain Compound of Formula IIb

Compound IIb-4 can react with triphosgene to convert amino into isocyanate, and then react with compound IN-c to obtain a compound of Formula IIb. The reaction is preferably carried out in a suitable organic solvent and a suitable base. The organic solvent can be selected from the group consisting of linear or cyclic ethers (e.g., tetrahydrofuran or diethyl ether, etc.), 1,4-dioxane, dichloromethane, dimethyl sulfoxide and any combination thereof, preferably tetrahydrofuran. The base is an organic base, such as *N,N*-diisopropylethylamine, triethylamine, pyridine, 4-dimethylaminopyridine, preferably *N,N-*diisopropylethylamine. The reaction is preferably carried out at a suitable temperature. The temperature can be -40°C to 60°C, such as 0-25°C. The reaction is preferably carried out for a suitable time, such as 0.5-2 hours.

Compound IIb-4 can also first react with phenyl chloroformate in a solvent and in the presence of a base, and then react with compound IN-c to obtain a compound of Formula IIb. The used base can be any base as long as it does not inhibit the reaction, and can usually be an organic base, such as *N,N-*diisopropylethylamine, triethylamine, pyridine and 4-dimethylaminopyridine, preferably *N,N-*diisopropylethylamine. The used solvent can be any solvent as long as it does not inhibit the reaction and can dissolve the raw materials to a certain extent, such as dichloromethane, tetrahydrofuran, 1,4-dioxane, diethyl ether, acetonitrile, etc., preferably tetrahydrofuran. The reaction temperature is usually 0°C to 60°C, preferably room temperature. The reaction time is usually 1-12 hours, preferably 4 hours. 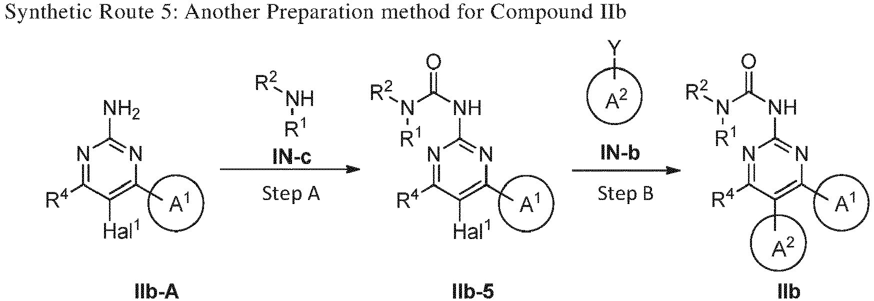 wherein: Hal¹ is halogen, such as Cl, Br or I, preferably Br; Y is a boric acid or boric acid ester group, preferably -B(OH)₂ or 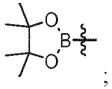 the remaining groups are as defined above in the present application.

### Step A: Reaction of Compound IIb-A with Compound IN-c to obtain Compound IIb-5

Compound IIb-A can react with triphosgene to convert amino into isocyanate, and then react with compound IN-c to obtain Compound IIb-5. The reaction is preferably carried out in a suitable organic solvent and a suitable base. The organic solvent can be selected from the group consisting of linear or cyclic ethers (e.g., tetrahydrofuran or diethyl ether, etc.), 1,4-dioxane, dichloromethane, dimethyl sulfoxide and any combination thereof, preferably tetrahydrofuran. The base is an organic base, such as *N,N*-diisopropylethylamine, triethylamine, pyridine, 4-dimethylaminopyridine, preferably *N,N-*diisopropylethylamine. The reaction is preferably carried out at a suitable temperature. The temperature can be -40°C to 60°C, such as 0-25°C. The reaction is preferably carried out for a suitable time, such as 0.5-2 hours.

Compound IIb-A can also first react with phenyl chloroformate in a solvent and in the presence of a base, and then react with compound IN-c to obtain Compound IIb-5. The used base can be any base as long as it does not inhibit the reaction, and can usually be an organic base, such as *N,N-*diisopropylethylamine, triethylamine, pyridine and 4-dimethylaminopyridine, preferably *N,N-*diisopropylethylamine. The used solvent can be any solvent as long as it does not inhibit the reaction and can dissolve the raw materials to a certain extent, such as dichloromethane, tetrahydrofuran, 1,4-dioxane, diethyl ether, acetonitrile, etc., preferably tetrahydrofuran. The reaction temperature is usually 0°C to 60°C, preferably room temperature. The reaction time is usually 1-12 hours, preferably 4 hours.

### Step B: Reaction of Compound IIb-5 with Compound IN-b to obtain Compound of Formula IIb

Compound IIb-5 and Compound IN-b undergo a coupling reaction to obtain a compound of Formula IIb. The coupling reaction is preferably carried out in the presence of a metal catalyst and a base. Preferably, the metal catalyst is a palladium metal catalyst, such as tetrakis(triphenylphosphine)palladium, [1,1'-bis(diphenylphosphino)ferrocene]dichloropalladium, [1,1'-bis(diphenylphosphino)ferrocene] palladium dichloride dichloromethane complex, bis(triphenylphosphine)palladium dichloride, palladium acetate, preferably [1,1'-bis(diphenylphosphino)ferrocene]dichloropalladium. The base is an inorganic base, such as potassium carbonate, cesium carbonate, sodium carbonate, sodium bicarbonate, potassium bicarbonate, preferably potassium carbonate. Preferably, the coupling reaction is carried out in a suitable organic solvent or a mixed solvent of an organic solvent and water, the organic solvent can be 1,4-dioxane, *N,N*-dimethylformamide or a mixed solvent of the above-mentioned organic solvent and water, for example, a mixed solvent of 1,4-dioxane and water. Preferably, the coupling reaction is carried out in a suitable protective atmosphere (e.g., a nitrogen atmosphere). Preferably, the coupling reaction is carried out at a suitable temperature, the temperature can be 70-100°C, preferably 80°C. Preferably, the coupling reaction is carried out for a suitable time, and the time can be 8-24 hours, such as 12 hours. 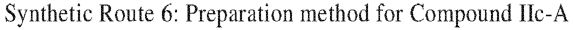 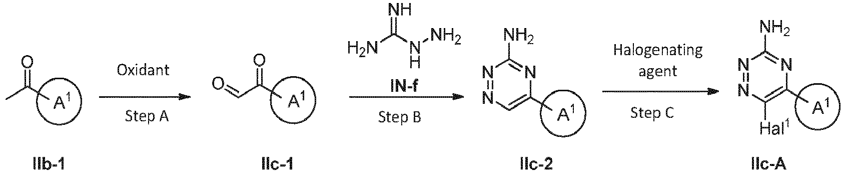 wherein: Hal¹ is halogen, such as Cl, Br or I, preferably Br; the remaining groups are as defined above in the present application.

### Step A: Reaction of Compound IIb-1 with oxidant to obtain Compound IIc-1

Compound IIb-1 reacts with an oxidant in a solvent to obtain Compound IIc-1. The used oxidant can be selenium dioxide, potassium permanganate, sodium periodate, hydrogen peroxide, etc., preferably selenium dioxide. The reaction can be carried out in a solvent that does not inhibit the reaction, such as 1,4-dioxane, *N,N*-dimethylformamide, tetrahydrofuran, N-methylpyrrolidone or a mixed solvent of the above-mentioned organic solvent and water, preferably a mixed solvent of 1,4-dioxane and water. The reaction temperature is usually from room temperature to 120°C, preferably 110°C. The reaction time is usually 3-14 hours, preferably 5 hours.

### Step B: Reaction of Compound IIc-1 with Compound IN-f to obtain Compound IIc-2

Compound IIc-1 reacts with Compound IN-f in the presence of an acid to obtain Compound IIc-2. The used aminoguanidine (IN-f) and an acid such as hydrochloric acid, carbonic acid, hydroiodic acid, sulfuric acid or acetic acid can form a salt, preferably a bicarbonate. The used acid can be any acid as long as it does not inhibit the reaction, including hydrochloric acid, acetic acid, sulfuric acid, trifluoroacetic acid, preferably acetic acid. The reaction is preferably carried out under a solvent-free condition. However, the reaction can also be carried out in a solvent that can dissolve the raw materials to a certain extent but does not inhibit the reaction, for example, 1,4-dioxane, ethanol, tetrahydrofuran, benzene or toluene, etc. The reaction temperature is usually room temperature to 120°C, preferably 100°C. The reaction time is usually 1-12 hours, preferably 6 hours.

### Step C: Reaction of Compound IIc-2 with halogenating agent to obtain Compound IIc-A

Compound IIc-2 reacts with a halogenating agent in a solvent to obtain Compound IIc-A. The used halogenating agent can be N-bromosuccinimide or bromine, preferably N-bromosuccinimide. The used solvent can be any solvent as long as it does not inhibit the reaction and can dissolve the raw materials to a certain extent, such as *N,N*-dimethylformamide, *N*-methylpyrrolidone, methanol, ethanol, tetrahydrofuran, 1,4-dioxane and dimethoxyethane, preferably *N,N*-dimethylformamide. The reaction temperature is usually -20°C to room temperature, preferably room temperature. The reaction time is usually 1-8 hours, preferably 2 hours. 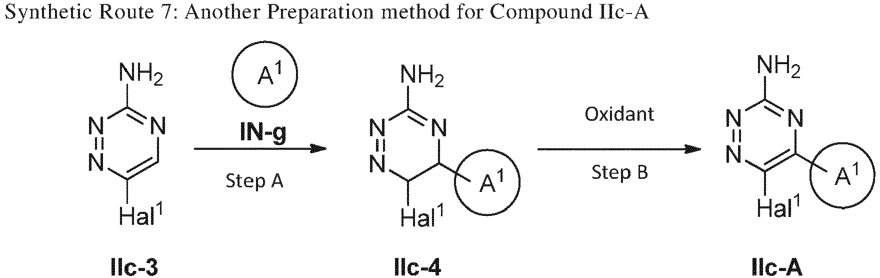 wherein: Hal¹ is halogen, such as Cl, Br or I, preferably Br; the remaining groups are as defined above in the present application.

### Step A: Reaction of Compound IIc-3 with Compound IN-g to obtain Compound IIc-4

Compound IIc-3 and Compound IN-g react in the presence of an acid to obtain Compound IIc-4. The used acid can be any acid as long as it does not inhibit the reaction, including hydrochloric acid, acetic acid, sulfuric acid, trifluoroacetic acid, preferably trifluoroacetic acid. The reaction can be carried out in a solvent that can dissolve the raw materials to a certain extent but does not inhibit the reaction, such as dichloromethane, chloroform, 1,4-dioxane, tetrahydrofuran, *N*-methylpyrrolidone, preferably chloroform. The reaction temperature is usually room temperature to 80°C, preferably room temperature. The reaction time is usually 1-8 hours, preferably 2 hours.

### Step B: Reaction of Compound IIc-4 with oxidant to obtain Compound IIc-A

Compound IIc-4 reacts with an oxidizing agent in a solvent to obtain Compound IIc-A. The used oxidizing agent can be K₃[Fe(CN)₆], DDQ, manganese dioxide, potassium permanganate, etc., preferably K₃[Fe(CN)₆]. The reaction can be carried out in a solvent that can dissolve the raw materials to a certain extent but does not inhibit the reaction, such as water, 1,4-dioxane, *N,N*-dimethylformamide, tetrahydrofuran, dimethylsulfoxide or a mixed solvent of the above-mentioned organic solvent and water, preferably water. The reaction temperature is usually room temperature to 120°C, preferably room temperature. The reaction time is usually 3-14 hours, preferably 12 hours. 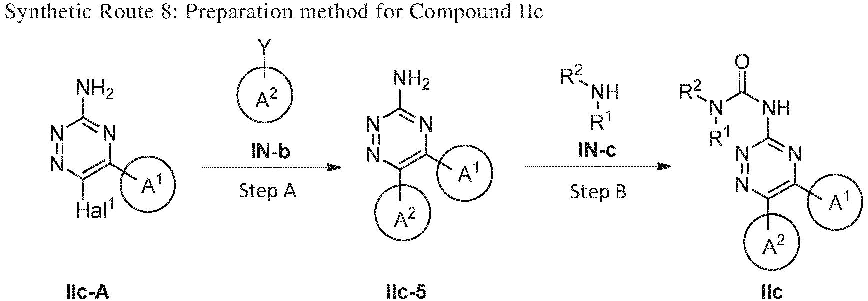 wherein: Hal¹ is halogen, such as Cl, Br or I, preferably Br; Y is a boric acid or boric acid ester group, preferably -B(OH)₂ or 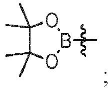 the remaining groups are as defined above in the present application.

### Step A: Reaction of Compound IIc-A with Compound IN-b to obtain Compound IIc-5

Compound IIc-A and Compound IN-b undergo a coupling reaction to obtain Compound IIc-5. The coupling reaction is preferably carried out in the presence of a metal catalyst and a base. Preferably, the metal catalyst is a palladium metal catalyst, such as tetrakis(triphenylphosphine)palladium, [1,1'-bis(diphenylphosphino)ferrocene]dichloropalladium, [1,1'-bis(diphenylphosphino)ferrocene] palladium dichloride dichloromethane complex, bis(triphenylphosphine)palladium dichloride, palladium acetate, preferably tetrakis(triphenylphosphine)palladium. The base is an inorganic base, such as cesium carbonate, potassium carbonate, sodium carbonate, potassium bicarbonate, sodium bicarbonate, preferably potassium carbonate. Preferably, the coupling reaction is carried out in a suitable organic solvent or a mixed solvent of an organic solvent and water, the organic solvent can be 1,4-dioxane, *N,N-*dimethylformamide or a mixed solvent of the above-mentioned organic solvent and water, for example, a mixed solvent of 1,4-dioxane and water. Preferably, the coupling reaction is carried out in a suitable protective atmosphere (e.g., a nitrogen atmosphere). Preferably, the coupling reaction is carried out at a suitable temperature, and the temperature can be 70-100°C, preferably 80°C. Preferably, the coupling reaction is carried out for a suitable time, and the time can be 1-24 hours, for example, 6 hours.

### Step B: Reaction of Compound IIc-5 with Compound IN-c to obtain Compound of Formula IIc

Compound IIc-5 can react with triphosgene to convert amino into isocyanate, and then react with Compound IN-c to obtain a compound of Formula IIc. The reaction is preferably carried out in a suitable organic solvent and a suitable base. The organic solvent can be selected from the group consisting of linear or cyclic ethers (e.g., tetrahydrofuran or diethyl ether, etc.), 1,4-dioxane, dichloromethane, dimethyl sulfoxide and any combination thereof, preferably tetrahydrofuran. The base is an organic base, such as *N,N*-diisopropylethylamine, triethylamine, pyridine, 4-dimethylaminopyridine, preferably *N,N-*diisopropylethylamine. The reaction is preferably carried out at a suitable temperature. The temperature can be -40°C to 60°C, such as 0-25°C. The reaction is preferably carried out for a suitable time, such as 0.5-2 hours.

Compound IIc-5 can also first react with phenyl chloroformate in a solvent and in the presence of a base, and then react with IN-c to obtain a compound of Formula (IIc). The used base can be any base as long as it does not inhibit the reaction, and usually can be an organic base, such as *N,N-*diisopropylethylamine, triethylamine, pyridine and 4-dimethylaminopyridine, preferably *N,N-*diisopropylethylamine. The used solvent can be any solvent as long as it does not inhibit the reaction but dissolve the raw materials to a certain extent, such as dichloromethane, tetrahydrofuran, 1,4-dioxane, diethyl ether, acetonitrile, etc., preferably tetrahydrofuran. The reaction temperature is usually 0°C to 60°C, preferably room temperature. The reaction time is usually 1-12 hours, preferably 4 hours. 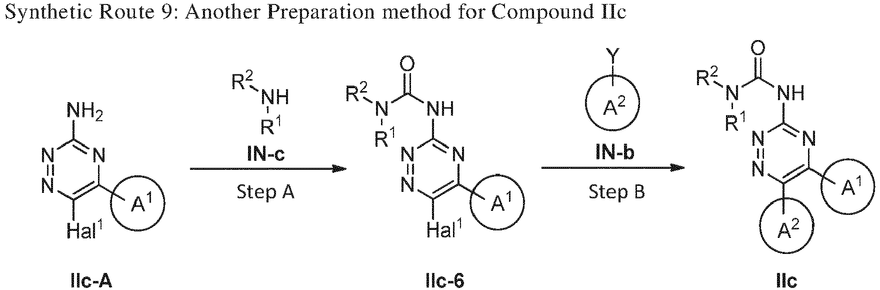 wherein: Hal¹ is halogen, such as Cl, Br or I, preferably Br; Y is a boric acid or boric acid ester group, preferably -B(OH)₂ or 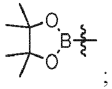 the remaining groups are as defined above in the present application.

### Step A: Reaction of Compound IIc-A with Compound IN-c to obtain Compound IIc-6

Compound IIc-A can react with triphosgene to convert amino into isocyanate, and then react with Compound IN-c to obtain Compound IIb-6. The reaction is preferably carried out in a suitable organic solvent and a suitable base. The organic solvent can be selected from the group consisting of linear or cyclic ethers (e.g., tetrahydrofuran or diethyl ether, etc.), 1,4-dioxane, dichloromethane, dimethyl sulfoxide and any combination thereof, preferably tetrahydrofuran. The base is an organic base, such as *N,N*-diisopropylethylamine, triethylamine, pyridine, 4-dimethylaminopyridine, preferably *N,N-*diisopropylethylamine. The reaction is preferably carried out at a suitable temperature. The temperature can be -40°C to 60°C, such as 0-25°C. The reaction is preferably carried out for a suitable time, such as 0.5-2 hours.

Compound IIc-A can also first react with phenyl chloroformate in a solvent and in the presence of a base, and then react with Compound IN-c to obtain Compound IIb-6. The used base can be any base as long as it does not inhibit the reaction, and usually can be an organic base, such as *N,N-*diisopropylethylamine, triethylamine, pyridine and 4-dimethylaminopyridine, preferably *N,N-*diisopropylethylamine. The used solvent can be any solvent as long as it does not inhibit the reaction but dissolve the raw materials to a certain extent, such as dichloromethane, tetrahydrofuran, 1,4-dioxane, diethyl ether, acetonitrile, etc., preferably tetrahydrofuran. The reaction temperature is usually 0°C to 60°C, preferably room temperature. The reaction time is usually 1-12 hours, preferably 4 hours.

### Step B: Reaction of Compound IIc-6 with Compound IN-b to obtain Compound of Formula (IIc)

Compound IIc-6 and Compound IN-b undergo a coupling reaction to obtain a compound of Formula (IIc). The coupling reaction is preferably carried out in the presence of a metal catalyst and a base. Preferably, the metal catalyst is a palladium metal catalyst, such as tetrakis(triphenylphosphine)palladium, [1,1'-bis(diphenylphosphino)ferrocene]dichloropalladium, [1,1'-bis(diphenylphosphine)ferrocene] palladium dichloride dichloromethane complex, bis(triphenylphosphine)palladium dichloride, palladium acetate, preferably [1,1'-bis(diphenylphosphino)ferrocene]dichloropalladium. The base is an inorganic base, such as potassium carbonate, cesium carbonate, sodium carbonate, sodium bicarbonate, potassium bicarbonate, preferably potassium carbonate. Preferably, the coupling reaction is carried out in a suitable organic solvent or a mixed solvent of organic solvent and water, the organic solvent can be 1,4-dioxane, *N,N*-dimethylformamide or a mixed solvent of the above-mentioned organic solvent and water, for example, a mixed solvent of 1,4-dioxane and water. Preferably, the coupling reaction is carried out under a suitable protective atmosphere (e.g., a nitrogen atmosphere). Preferably, the coupling reaction is carried out at a suitable temperature, and the temperature can be 70-100°C, preferably 80°C. Preferably, the coupling reaction is carried out for a suitable time, and the time can be 8-24 hours, such as 12 hours.

The specific conditions of each of the above-mentioned reaction steps are well-known in the art, and are not specifically limited in the present application. According to the teachings of the present application in combination with common knowledge in the art, those skilled in the art can select and replace each substituent in the general formula to prepare different compounds, and these choices and substitutions are all within the scope of protection of the present application.

The present application also relates to a pharmaceutical composition comprising the compound represented by Formula I, a pharmaceutically acceptable salt, a stereoisomer, a polymorph, a solvate, a N-oxide, an isotope-labeled compound, a metabolite or a prodrug thereof according to the present application, and optionally one or more pharmaceutically acceptable carriers or excipients.

The present application also relates to a use of the compound represented by Formula I, a pharmaceutically acceptable salt, a stereoisomer, a polymorph, a solvate, a *N*-oxide, an isotope-labeled compound, a metabolite or a prodrug thereof, or the pharmaceutical composition thereof in the manufacture of a medicament as an adenosine A2a receptor antagonist.

The present application also relates to a use of the compound represented by Formula I, a pharmaceutically acceptable salt, a stereoisomer, a polymorph, a solvate, a N-oxide, an isotope-labeled compound, a metabolite or a prodrug thereof, or the pharmaceutical composition thereof in the manufacture of a medicament for the treatment or prevention of a disease related to adenosine A2a receptor.

The present application also relates to a method for the treatment and/or prevention of a disease related to adenosine A2a receptorcomprising administering to a subject in need an effective amount of the compound represented by Formula I, a pharmaceutically acceptable salt, a stereoisomer, a polymorph, a solvate, a N-oxide, an isotope-labeled compound, a metabolite or a prodrug thereof, or the pharmaceutical composition thereof according to the present application.

The present application also relates to the compound represented by Formula I, a pharmaceutically acceptable salt, a stereoisomer, a polymorph, a solvate, a N-oxide, an isotope-labeled compound, a metabolite or a prodrug thereof, or the pharmaceutical composition thereof, for use in the inhibition of the activity of adenosine A2a receptor.

The present application also relates to the compound represented by Formula I, a pharmaceutically acceptable salt, a stereoisomer, a polymorph, a solvate, a *N*-oxide, an isotope-labeled compound, a metabolite or a prodrug thereof, or the pharmaceutical composition thereof, for use in the treatment and/or prevention of a disease related to adenosine A2a receptor.

In some embodiments, the disease related to adenosine A2a receptor is a tumor. The tumor includes but is not limited to: breast cancer, ovarian cancer, colorectal cancer, melanoma, non-small cell lung cancer, small cell lung cancer, gastrointestinal stromal tumor, cervical cancer, pancreatic cancer, prostate cancer, gastric cancer, chronic myeloid leukocytosis, liver cancer, lymphoma, peritoneal cancer and soft tissue sarcoma.

In the present application, the purpose of the pharmaceutical composition is to promote the administration to a biological body, which is conducive to the absorption of an active ingredient so as to exert biological activity thereof, wherein the carrier includes but is not limited to: ion exchanger, aluminum oxide, aluminum stearate, lecithin, serum protein such as human albumin, buffer substance such as phosphate, glycerin, sorbic acid, potassium sorbate, partial glyceride mixture of saturated plant fatty acid, water, salt or electrolyte such as protamine sulfate, disodium hydrogen phosphate, potassium hydrogen phosphate, sodium chloride, zinc salt, colloidal silica, magnesium trisilicate, polyvinylpyrrolidone, cellulose material, polyethylene glycol, sodium carboxymethylcellulose, polyacrylate, beeswax, wool fat.

The excipient refers to an additive besides main active ingredient in the pharmaceutical preparation. It is stable in nature, has no contraindication with the main active ingredient, does not produce side effects, does not affect the efficacy, is not easy to deform, crack, mildew or worm-eaten at room temperature, is harmless to the human body, has no physiological effect, does not produce chemical or physical effects with the main active ingredient, and does not affect the content determination of the main active ingredient. For example, binder, filler, disintegrant, lubricant in tablets; alcohol, vinegar, concoction and so on in Chinese medicine pills; base substance in semi-solid preparation ointment or cream; preservative, antioxidant, corrigent, flavoring agent, cosolvent, emulsifier, solubilizer, osmotic pressure regulator, coloring agent and the like in liquid preparation can all be called excipients.

The compound, a pharmaceutically acceptable salt, a stereoisomer, a polymorph, a solvate, a *N-*oxide, an isotope-labeled compound, a metabolite or a prodrug thereof according to the present application can be administered through the following routes: parenteral, topical, intravenous, oral, subcutaneous, intraarterial, intradermal, percutaneous, rectal, intracranial, intraperitoneal, intranasal or intramuscular routes, or as an inhalant. The pharmaceutical composition can optionally be administered in combination with an additional agent that has at least a certain effect in the treatment of various diseases.

The compound, a pharmaceutically acceptable salt, a stereoisomer, a polymorph, a solvate, a *N-*oxide, an isotope-labeled compound, a metabolite or a prodrug thereof according to the present application can be prepared into a suitable preparation form according to administration route.

The pharmaceutical composition or suitable preparation form thereof according to the present application can contain 0.01 mg to 1000 mg of the compound of the present application.

When administered orally, the compound, a pharmaceutically acceptable salt, a stereoisomer, a polymorph, a solvate, a *N*-oxide, an isotope-labeled compound, a metabolite or a prodrug thereof according to the present application can be prepared into any orally acceptable preparation form, including but not limited to tablet, capsule, aqueous solution or suspension.

When applied topically to skin, the compound, a pharmaceutically acceptable salt, a stereoisomer, a polymorph, a solvate, a N̅-oxide, an isotope-labeled compound, a metabolite or a prodrug thereof according to the present application can be prepared into a suitable preparation form such as ointment, lotion or cream, wherein the active ingredient is suspended or dissolved in one or more carriers. The carriers that can be used in ointment preparation include but are not limited to: mineral oil, liquid petrolatum, white petrolatum, propylene glycol, polyoxyethylene, polyoxypropylene, emulsified wax and water; the carriers that can be used in lotion or cream include but are not limited to: mineral oil, sorbitan monostearate, Tween-60, cetyl ester wax, hexadecene aromatic alcohol, 2-octyldodecanol, benzyl alcohol and water.

The compound, a pharmaceutically acceptable salt, a stereoisomer, a polymorph, a solvate, a *N-*oxide, an isotope-labeled compound, a metabolite or a prodrug thereof according to the present application can also be administered in the form of sterile injectable preparation including sterile injection water or oil suspension or sterile injection solution, wherein usable carriers and solvents include water, Ringer's solution and isotonic sodium chloride solution. In addition, the sterilized non-volatile oil can also be used as solvent or suspension medium, such as monoglyceride or diglyceride.

In an embodiment of the application, suitable in vitro or in vivo tests are performed to determine the effect of the pharmaceutical composition of the present application and whether the administration is suitable for treating a disease or medical disease state of an individual. Examples of these tests are described in a non-limiting manner in following examples in combination with specific diseases or medical treatments. Generally, the effective amount of the composition of the present application, that is sufficient to achieve preventive or therapeutic effects, is about 0.001 mg/kg body weight/day to about 10,000 mg/kg body weight/day. Under appropriate situations, the dose is about 0.01 mg/kg body weight/day to about 1000 mg/kg body weight/day. The dose range can be about 0.01 to 1000 mg/kg subject body weight, more usually 0.1 to 500 mg/kg subject body weight every day, every two days or every three days. An exemplary treatment regimen is to perform administration once every two days or once a week or once a month. Generally, the preparation is administered for multiple times, and the interval between single dosages can be a day, a week, a month or a year, or the preparation can be administered in the form of a sustained-release preparation, in which case less frequency of administration is required. The dosage and frequency vary according to the half-life of preparation in a subject. The dosage and frequency can be also different depending on whether it is a preventive treatment or a therapeutic treatment. In the preventive treatment, a relatively low dose is given at a relatively low frequency interval for a long time. In the therapeutic treatment, it is sometimes necessary to give a relatively high dose at a relatively short interval, until the progression of disease is delayed or stopped, and preferably until the individual shows a partial or complete improvement in the symptoms of the disease, after which a preventive program can be given to the individual.

The terms of the present application are explained as follows. For a specific term, if its meaning in the present application is inconsistent with its meaning commonly understood by those skilled in the art, the meaning in the present application shall prevail; if there is no definition in the present application, it has the meaning commonly understood by those skilled in the art. Unless stated to the contrary, the terms used in the present application have the following meanings:

The terms "comprising", "including", "having", "containing" or "involving" and other variant forms thereof used herein are inclusive or open-ended, and do not exclude other unlisted elements or method steps.

The term "hydrogen" or H in each group used in the present application refers to protium (H), deuterium (D) or tritium (T).

The term "halogen" used in the present application refers to fluorine, chlorine, bromine or iodine.

The term "C₁₋₆ alkyl" used in the present application refers to a linear or branched alkyl having 1 to 6 carbon atoms, such as C₁₋₄ alkyl, C₁₋₂ alkyl, C₁ alkyl, C₂ alkyl, C₃ alkyl, C₄ alkyl, C₅ alkyl or C₆ alkyl. Specific examples include but are not limited to methyl, ethyl, propyl, isopropyl, n-butyl, isobutyl, sec-butyl, tert-butyl, n-pentyl, n-hexyl, etc.

The term "C₁₋₆ alkoxy" used in the present application refers to a C₁₋₆ alkyl as defined above that is linked to the parent molecular through an oxygen atom. Representative examples of C₁₋₆ alkoxy include but are not limited to methoxy, ethoxy, n-propoxy, isopropoxy, n-butoxy, isobutoxy, tert-butoxy.

The term "C₁₋₆ haloalkyl" as used in the present application refers to a C₁₋₆ alkyl as defined above that is mono- or poly-substituted by halogens such as fluorine, chlorine, bromine or iodine. Representative examples of C₁₋₆ haloalkyl include but not limited to chloromethyl, chloroethyl, dichloroethyl, trifluoromethyl, difluoromethyl, monofluoromethyl, etc.

The term "C₁₋₆ alkyl-amino-" used in the present application refers to an amino mono-substituted by C₁₋₆ alkyl as defined above. Typical examples of "C₁₋₆ alkyl-amino" include but are not limited to methylamino, ethylamino, propylamino, butylamino, etc.

The term "di(C₁₋₆ alkyl)-amino-" used in the present application refers to an amino di-substituted by C₁₋₆ alkyl as defined above. Typical examples of "di(C₁₋₆ alkyl)-amino-" include but are not limited to dimethylamino, diethylamino, dipropylamino, dibutylamino, etc.

The term "C₃₋₁₀ cycloalkyl" as used herein refers to a saturated cyclic hydrocarbyl having 3 to 10 carbon atoms and having a monocyclic or bicyclic or multiple fused ring (including fused and bridged ring systems), for example, having 3 to 8, 5 to 8, 3 to 6, or 5 to 6 carbon atoms. Typical examples of "C₃₋₁₀ cycloalkyl" include but are not limited to monocyclic structures such as cyclopropyl, cyclobutyl, cyclopentyl, cyclohexyl, cycloheptyl, cyclooctyl, 1-methylcyclopropyl, 2-methylcyclopentyl, 2-methylcyclooctyl, etc., bicyclic structures such as bicyclo[2.2.1]heptyl, and polycyclic structures such as adamantyl, etc.

The term "4- to 12-membered heterocyclyl" used in the present application refers to a saturated or partially saturated monocyclic or bicyclic or multiple fused cyclic group that is not aromatic and contains at least one (for example, containing 1, 2, 3, 4 or 5) heteroatom and the number of ring atoms thereof is 4 to 12, in which the heteroatom is nitrogen atom, oxygen atom and/or sulfur atom. The term "4- to 12-membered heterocyclyl" can be substituted by an oxo or a sulfo. For example, the "4- to 12-membered heterocyclyl" in the present application contains 1 to 2 heteroatoms, for example, contains one nitrogen atom, oxygen atom or sulfur atom, or contains one nitrogen atom and one oxygen atom. The "4- to 12-membered heterocyclyl" includes "4- to 10-membered heterocyclyl", "4- to 8-membered heterocyclyl", "4- to 6-membered heterocyclyl", "4- to 7-membered heterocyclyl", "5- to 6-membered heterocyclyl", "4- to 6-membered saturated heterocyclyl", "5- to 6-membered saturated heterocyclyl", "4- to 8-membered oxygen-containing heterocyclyl", "4- to 6-membered oxygen-containing heterocyclyl", "5- to 6-membered oxygen-containing heterocyclyl", "4- to 6-membered saturated oxygen-containing heterocyclyl", "4- to 10-membered nitrogen-containing heterocyclyl", "4- to 7-membered nitrogen-containing heterocyclyl", "4- to 8-membered nitrogen-containing heterocyclyl", "5- to 6-membered nitrogen-containing heterocyclyl", "5- to 6-membered saturated nitrogen-containing heterocyclyl" and so on. Specific examples of "4- to 12-membered heterocyclyl" include but are not limited to: azetidinyl, 1,4-dioxanyl, 1,3-dioxanyl, 1,3-dioxolanyl, 1,4-dioxyheterocyclohexadienyl, tetrahydrofuranyl, dihydropyrrolyl, pyrrolyl, imidazolyl, 4,5-dihydroimidazolyl, pyrazolidinyl, 4,5-dihydropyrazolyl, 2,5-dihydrothienyl, tetrahydrothienyl, piperazinyl, thiazinyl, piperidinyl, morpholinyl, etc.

The term "C₆₋₁₀ aryl" used in the present application refers to an unsaturated aromatic carbocyclyl having monocyclic or bicyclic or multiple fused rings and having 6 to 10 carbon atoms. For example, the aryl has 5 to 8 or 5 to 6 carbon atoms. Typical examples of "C₆₋₁₀ aryl" include but are not limited to phenyl, naphthyl and anthracenyl, etc.

The term "5- to 10-membered heteroaryl" as used herein refers to a heteroaromatic ring group with 5-10 ring members, including monocyclic heteroaromatic ring and polycyclic aromatic ring, in which a monocyclic aromatic ring is fused with one or more other aromatic rings in a polycyclic aromatic ring. The "5- to 10-membered heterocyclyl" has one or two or more heteroatoms selected from the group consisting of O, S or N. The term "heteroaryl" used in the present application also comprises a group in which an aromatic ring is fused with one or more non-aromatic rings (carbocyclic rings or heterocyclic rings), wherein the connecting group or point is located on the aromatic or non-aromatic ring. The heteroaryl group has, for example, 5 to 6 ring members. Typical examples of "5- to 10-membered heterocyclyl" include but are not limited to furyl, imidazolyl, triazolyl, indolyl, tetrazolyl, pyridyl, pterridinyl, pyrimidinyl, triazolyl, quinolinyl, isoquinolinyl, quinazolinyl, quinoxalinyl, etc.

If a substituent is described as "optionally substituted by... ", then the substituent can be (1) unsubstituted or (2) substituted. If a carbon of the substituent is described as optionally substituted by one or more substitutions in the list, then one or more hydrogens on the carbon (to the extent of any existing hydrogens) can be individually and/or together replaced by independently selected optional substituents. If a nitrogen of the substituent is described as optionally substituted by one or more substitutions of the list, then one or more hydrogens on the nitrogen (to the extent of any existing hydrogens) can each be replaced by an independently selected optional substituent.

The term "one or more" used in the present application refers to one or more than one under reasonable conditions, such as two, three, four, five, six, seven, eight, nine or ten.

Unless specified, as used herein, the point for attaching a substituent can be from any suitable position of the substituent.

The term "stereoisomer" as used in the present application refers to an isomer formed due to at least one asymmetric center. In a compound having one or more (for example, 1, 2, 3, or 4) asymmetric centers, it can produce a racemic mixture, a single enantiomer, a diastereoisomer mixture and individual diastereomers. Specific molecules can also exist as geometric isomers (cis/trans). Similarly, the compound of the present application can exist in a mixture of two or more structurally different forms (commonly referred to as tautomers) that are in rapid equilibrium. Representative examples of tautomers include keto-enol tautomers, phenol-ketone tautomers, nitroso-oxime tautomers, imine-enamine tautomers, etc. It should be understood that the scope of the present application covers all such isomers or mixtures thereof in any ratios (e.g., 60%, 65%, 70%, 75%, 80%, 85%, 90%, 95%, 96%, 97%, 98%, 99%).

Unless otherwise specified, the compounds of the present application are intended to exist in the form of stereoisomers (which include cis and trans isomers, optical isomers (e.g., R and S enantiomers), diastereomers, geometric isomers, rotamers, conformational isomers, atropisomers and mixtures thereof). The compound of the present application can exhibit one above type of isomerism, and consists of its mixtures (for example, racemic mixture and diastereoisomer pair).

The present application covers all possible crystalline forms or polymorphs of the compound of the present application, which can be a single polymorph or a mixture of more than one polymorph in any ratio.

It should also be understood that some compounds of the present application can exist in free form for treatment, or appropriately exist in the form of its pharmaceutically acceptable derivative. In the present application, the pharmaceutically acceptable derivative includes but is not limited to pharmaceutically acceptable salt, solvate, *N*-oxide, metabolite or prodrug, after they are administered to a patient in need thereof, they can directly or indirectly provide the compound or metabolite or residue thereof according to the present application. Therefore, when referring to "the compound of the present application" herein, it is also intended to cover the above various derivative forms of the compound.

Those skilled in the art will understand that since nitrogen requires available lone pair of electrons to be oxidized into oxides, not all nitrogen-containing heterocycles can form *N*-oxides; those skilled in the art can recognize nitrogen-containing heterocycles that can form *N*-oxides. Those skilled in the art can also recognize that tertiary amines can form *N*-oxides. The synthetic methods for preparing *N*-oxides of heterocycles and tertiary amines are well known to those skilled in the art, comprising oxidizing heterocycles and tertiary amines with peroxide acids such as peracetic acid and m-chloroperoxybenzoic acid (MCPBA), hydrogen peroxide, alkyl hydroperoxide such as tert-butyl hydroperoxide, sodium perborate and dioxirane such as dimethyl dioxirane. These methods for preparing *N*-oxides have been widely described and reviewed in the literature, see for example: TL Gilchrist, Comprehensive Organic Synthesis, vol. 7, pp 748-750; AR Katritzky and AJ Boulton, Eds., Academic Press; and G. W. H Cheeseman and E. S. G. Werstiuk, Advances in Heterocyclic Chemistry, vol. 22, pp 390-392, A. R. Katritzky and A. J. Boulton, Eds., Academic Press.

The compound of the present application can exist in the form of solvate (such as hydrate), wherein the compound of the present application contains a polar solvent as a structural element of crystal lattice of the compound, especially, for example, water, methanol or ethanol. The amount of polar solvent, especially water, can exist in a stoichiometric or non-stoichiometric ratio.

The compound or pharmaceutically acceptable salt thereof according to the present application can also form a solvate, such as an alcoholate, etc.

The compound of the present application can also be a prodrug or in a form that can release the active ingredient after metabolic changes in the body. It is well-known technique to those skilled in the art to select and prepare an appropriate prodrug derivative.

The compound of the present application can also be in the form of chemical protection, in which a protecting group can protect an active group (such as amino) of the compound, and the protecting group can be metabolized in the body to release the active ingredient. It is well-known technique to those skilled in the art to select and prepare an appropriate chemical protection form.

The term "pharmaceutically acceptable" as used in the present application means that a substance or composition must be chemically and/or toxicologically compatible with other components of a preparation and/or a mammal to be treated with it.

The term "pharmaceutically acceptable salt" as used in the present application comprises conventional salts formed with pharmaceutically acceptable inorganic or organic acids, or inorganic or organic bases. Examples of suitable acid addition salts include salts formed with perchloric acid, propionic acid, succinic acid, hydroxyacetic acid, pamoic acid, malonic acid, hydroxymaleic acid, phenylacetic acid, glutamic acid, naphthalene-2-sulfonic acid, hydroxynaphthoic acid, hydroiodic acid, malic acid, tannic acid, etc. Examples of suitable base addition salts include salts formed with sodium, potassium, magnesium, lithium, aluminum, calcium, zinc, *N,N*'-dibenzylethylenediamine, chloroprocaine, choline, diethanolamine, ethylenediamine, *N-*methylglucosamine and procaine.

For a review of suitable salts, see "Handbook of Pharmaceutical Salts: Properties, Selection, and Use" by Stahl and Wermuth (Wiley-VCH, 2002). Methods for preparing pharmaceutically acceptable salts of the compounds according to the present application are well known by those skilled in the art.

The term "pharmaceutical composition" as used in the present application comprises a product containing a therapeutically effective amount of the compound of the present application represented by Formula I, and any product directly or indirectly produced by the combination of the compound represented by Formula I according to the present application.

The term "effective amount" as used in the present application refers to an amount sufficient to achieve a desired therapeutic effect, for example, an amount that achieves alleviation of a symptom associated with a disease to be treated.

The term "prevention" as used in the present application refers to prophylactic administration to reduce the chance of onset of a disease or symptom or delay the onset of a disease or symptom.

The purpose of the term "treatment" as used in the present application refers to reduction or elimination of a targeted disease state or condition. If a subject receives a therapeutic amount of the compound, an optical isomer or a pharmaceutically acceptable salt or a pharmaceutical composition thereof, one or more indications and symptoms of the subject show observable and/or detectable reduction or improvement, the subject is successfully "treated". It should also be understood that the treatment of a disease state or condition comprises not only the disease state or condition is complete treated, but also although the disease state or condition is not completed treated, some biological or medical related results are achieved.

The term "subject" as used in the present application refers to a mammal subject (e.g., dog, cat, horse, cattle, sheep, goat, monkey, etc.) and a human subject including male and female subjects, and including newborns, infants, teenagers, adolescents, adults and elderly subjects, and including various races and ethnicities including but not limited to whites, blacks, Asians, American Indians and Hispanics.

For the compound of the present application, if its name is inconsistent with its structural formula, the compound structural formula shall prevail.

### Beneficial effects of the present application

Through a lot of research, it was surprisingly found that the compound of the present application has weak inhibitory ability on adenosine A1 receptor and can selectively inhibit adenosine A2a receptor. In addition, the compound of the present application has low blood-brain barrier permeability, and has strong peripheral selectivity and good pharmacokinetic properties. Therefore, the compound of the present application can activate T cell immune function mediated by the activation of adenosine A2a receptor, has good tumor treatment activity, and can avoid toxic side effects caused by inhibition of adenosine A2a receptor in the central nervous system.

### Specific Models for Carrying Out the Invention

The embodiments of the present application will be described in detail below in combination with examples, but those skilled in the art will understand that the following examples are only used to illustrate the present application, and should not be considered as limiting the scope of the present application. If specific conditions are not given in the examples, the conventional conditions or the conditions recommended by the manufacturer will be followed. The reagents or instruments used without giving manufacturers are all conventional products that can be purchased commercially.

In the following examples, the structure of compound was determined by nuclear magnetic resonance (¹H NMR) or mass spectrometry (MS). The measurement of ¹H NMR was carried out by using JEOL Eclipse 400 nuclear magnetometer, wherein the used solvent for measurement was deuterated methanol (CD₃OD), deuterated chloroform (CDCl₃) or hexa-deuterated dimethyl sulfoxide (DMSO-*d₆*), the internal standard was tetramethylsilane (TMS), and the chemical shift (δ) was given in unit of 10⁻⁶ (ppm).

In the following examples, the MS was measured by using Agilent (ESI) mass spectrometer, its manufacturer is Agilent, and its model is Agilent 6120B.

In the following examples, when a preparative high performance liquid chromatograph was used for purification, the model of the used instrument was Agilent 1260, and the chromatographic column was Waters SunFire Prep C18 OBD (19 mm×150 mm×5.0 µm). The chromatographic conditions were as follows:
Column temperature: 25°C;
Flow rate: 20.0 mL/min;
Detection wavelength: 214 nm;
Elution gradient: (0 min: 10% (v/v) A, 90% (v/v) B; 16.0 min: 90% (v/v) A, 10% (v/v) B);
wherein the mobile phase A was acetonitrile; the mobile phase B was 0.05% (v/v) aqueous solution of formic acid.

In the following examples, an aluminum plate (20×20 cm) produced by Merck was used as the silica gel plate of thin layer chromatography (TLC), while GF 254 (thickness: 1 mm) silica plate produced in Yantai was used for TLC separation and purification.

In the following examples, the reaction was monitored by thin layer chromatography (TLC) or LC-MS, wherein the used developing solvent systems included dichloromethane and methanol system, n-hexane and ethyl acetate system, petroleum ether and ethyl acetate system, in which the volume ratios of solvents were adjusted according to the polarity of compound or adjusted by adding triethylamine.

In the following examples, Biotage Initiator+ (400W, RT~300°C) microwave reactor was used in microwave reaction.

In the following examples, 200-300 mesh silica gel was generally used as carrier in column chromatography. The eluent system comprised: dichloromethane and methanol system, petroleum ether and ethyl acetate system, in which the volume ratios of solvents were adjusted according to the polarity of compound or adjusted by adding a small amount of triethylamine.

When special instructions were not given in the following examples, the reaction temperatures were room temperature (20°C to 35°C).

In the following examples, the reagents used in the present application were purchased from companies such as Acros Organics, Aldrich Chemical Company, and Terbo Chemical.

In the conventional synthesis method and the examples for synthesis of compounds and intermediates of the present application, the meanings of abbreviations were as follows.

| Abbreviation | Meaning |
|---|---|
| DMF | *N,N*-Dimethylformamide |
| dppf | 1,1'-Bis(diphenylphosphino)ferrocene |
| TLC | Thin layer chromatography |
| Pd(dppf)Cl₂ | [1,1'-Bis(diphenylphosphino)ferrocene]dichloropalladium |
| Pd(amphos)Cl₂ | Bis(di-tert-butyl-(4-dimethylaminophenyl)phosphine dichloropalladium |
| DIPEA | *N,N*-Diisopropylethylamine |
| K₃[Fe(CN)₆] | Potassium ferricyanide |

### Preparation of intermediates:

Preparation Example 1 of Intermediate: Preparation of 6-bromo-5-(5-methylfuran-2-yl)-1,2,4-triazine-3-amine (In-1)

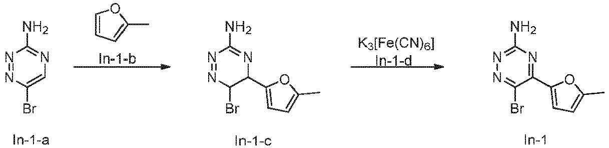

### Step 1: Preparation of 6-bromo-5-(5-methylfuran-2-yl)-4,5-dihydro-1,2,4-triazine-3-amine (In-1-c)

At 0°C, trifluoroacetic acid (50 mL) was added dropwise into a solution of 3-amino-6-bromo-1,2,4-triazine (In-1-a) (10.0 g, 114.4 mmol) and 2-methylfuran (In-1-b) (9.4 g, 114.3 mmol) in chloroform (50 mL), then the obtained mixture was heated to room temperature, and stirred continually for 2 hours. After the reaction was completed, saturated sodium carbonate solution was added to the reaction solution to adjust the pH to 8-9, the obtained mixture was stirred for 20 minutes, n-heptane was added to form a slurry, and then the slurry was filtrated, the obtained filter residue was washed with n-heptane, and dried in vacuo to obtain the title compound (In-1-c) (14 g, yield: 95 %).
MS m/z (ESI): 257.0 [M+H]⁺.

### Step 2: Preparation of 6-bromo-5-(5-methylfuran-2-yl)-1,2,4-triazine-3-amine (In-1)

Potassium hydroxide (7.3 g, 130.7 mmol) in water (90 mL) solution was added into a solution of 6-bromo-5-(5-methylfuran-2-yl)-4,5-dihydro-1,2,4-triazine-3-amine (In-1-c) (14.0 g, 54.5 mmol) and K₃[Fe(CN)₆] (In-1-d) (35.9 g, 108.9 mmol) in dichloromethane (180 mL), and reacted overnight at room temperature. After the reaction was completed, water (250 mL) and n-heptane (250 mL) were added to the reaction solution to form a slurry, the slurry was subjected to suction filtration, the obtained filter residue was washed with n-heptane, and dried in vacuo to obtain the title compound (In-1) (11 g, yield: 79%).
MS m/z (ESI): 255.0 [M+H]⁺.
Preparation Example 2 of Intermediate:
Preparation of 4-methyl-6-(4,4,5,5-tetramethyl-1,3,2-dioxaborolan-2-yl)quinazoline

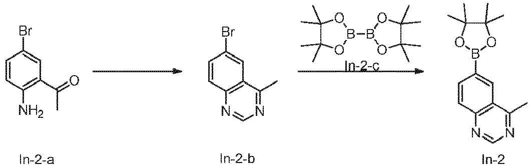

### Step 1: Preparation of 6-bromo-4-methylquinazoline (In-2-b)

To a reaction flask, 1-(2-amino-5-bromophenyl)ethanone (In-2-a) (3 g, 14 mmol), triethyl orthoformate (3.1 g, 21 mmol) and ammonium acetate (1.62 g, 21 mmol) were added, and reacted overnight at 100°C. After the reaction was completed, the reaction solution was cooled to room temperature and concentrated, the residue was diluted with water and extracted with ethyl acetate, the organic phases were combined, dried over anhydrous sodium sulfate, filtered, the filtrate was concentrated, and the obtained residue was purified by silica gel column chromatography (eluent: ethyl acetate/petroleum ether = 3/1 (v/v)) to obtain the title compound (In-2-b) (2.6 g, yield: 83%).
MS m/z (ESI): 223.0 [M+H]⁺.

### Step 2: Preparation of 4-methyl-6-(4,4,5,5-tetramethyl-1,3,2-dioxaborolan-2-yl)quinazoline (In-2)

Under a nitrogen atmosphere, bis(pinacolato)diboron (In-2-c) (3.4 g, 13.5 mmol), potassium acetate (1.76 g, 16.9 mmol) and Pd(dppf)Cl₂ (0.65 g, 0.9 mmol) were added into a solution of 6-bromo-4-methylquinazoline (In-2-b) (2 g, 8.9 mmol) in 1,4-dioxane (50 mL), reacted at 80°C for 6 hours. After the reaction was completed, the reaction solution was cooled to room temperature and concentrated, the residue was diluted with water, and extracted with ethyl acetate, the organic phases were combined, dried over anhydrous sodium sulfate, filtrated, concentrated, and the obtained residue was purified by silica gel column chromatography (eluent: ethyl acetate/petroleum ether = 2/1 (v/v)) to obtain the title compound (In-2) (2.1 g, yield: 87%).
MS m/z (ESI): 271.2 [M+H]⁺.

### Preparation Example 3 of Intermediate:

Preparation of 5-(4-fluorophenyl)-6-(4-methylquinazolin-6-yl)-1,2,4-triazine-3-amine (In-3)

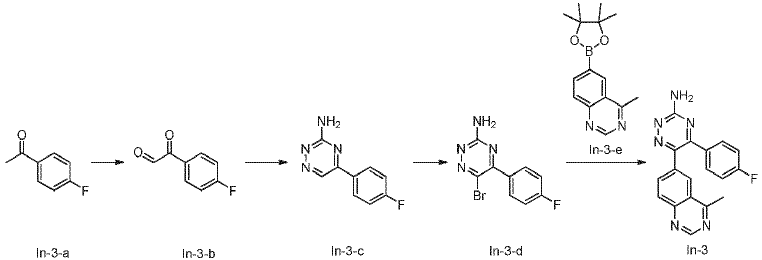

### Step 1: Preparation of 2-(4-fluorophenyl)-2-oxoacetaldehyde (In-3-b)

Under a nitrogen atmosphere, water (5 mL) was added to a solution of selenium dioxide (3.3 g, 29.7 mmol) in 1,4-dioxane (50 mL), stirred at 60°C for 0.5 hours until the selenium dioxide was completely dissolved to obtain a mixture, 1-(4-fluorophenyl)ethanone (In-3-a) (1.3 g, 9.7 mmol) was added to the mixture, and reacted at 110°C for 5 hours. The reaction solution was cooled to room temperature, and subjected to suction filtration. The filtrate was concentrated, and the residue was purified by silica gel column chromatography (eluent: ethyl acetate/petroleum ether = 1/1 (v/v)) to obtain the title compound (In-3-b) of this step (1.2 g, yield: 92%).
MS m/z (ESI): 153.0 [M+H]⁺.

### Step 2: Preparation of 5-(4-fluorophenyl)-1,2,4-triazine-3-amine (In-3-c)

2-(4-Fluorophenyl)-2-oxoacetaldehyde (In-3-b) (1.2 g, 7.9 mmol) was dissolved in acetic acid (20 mL), aminoguanidine (1.2 g, 15.8 mmol) was added in batches, and reacted at 100°C for 6 hours. The reaction solution was cooled to room temperature, the residue was diluted with water, and extracted with ethyl acetate. The organic phases were combined, dried over anhydrous sodium sulfate, and filtrated, the filtrate was concentrated, and the residue was purified by silica gel column chromatography (eluent: ethyl acetate/petroleum ether = 1/1 (v/v)) to obtain the title compound (In-3-c) of this step (0.9 g, yield: 60%).
MS m/z (ESI): 191.1 [M+H]⁺.

### Step 3: Preparation of 6-bromo-5-(4-fluorophenyl)-1,2,4-triazine-3-amine (In-3-d)

Under ice bath, *N*-bromosuccinimide (1.1 g, 6.1 mmol) was added into a solution of 5-(4-fluorophenyl)-1,2,4-triazine-3-amine (In-3-c) (0.9 g, 4.7 mmol) in DMF (10 mL) in batches and reacted at room temperature for 2 hours. The reaction solution was poured into water, and then extracted with ethyl acetate, the organic phases were combined, dried over anhydrous sodium sulfate, and filtered, and the filtrate was concentrated to obtain the title compound (In-3-d) of this step (1 g, yield: 80%).
MS m/z (ESI): 269.0 [M+H]⁺.

### Step 4: Preparation of 5-(4-fluorophenyl)-6-(4-methylquinazolin-6-yl)-1,2,4-triazine-3-amine (In-3)

6-Bromo-5-(4-fluorophenyl)-1,2,4-triazine-3-amine (In-3-d) (1 g, 3.7 mmol), 4-methyl-6-(4,4,5,5-tetramethyl-1,3,2-dioxaborolan-2-yl)quinazoline (In-3-e) (1.2 g, 4.4 mmol) and potassium carbonate (1.1 g, 7.4 mmol) were added to a mixed solvent of 1,4-dioxane (20 mL) and water (4 mL), the atmosphere of which was replaced with nitrogen gas for three times, and tetrakis(triphenylphosphine)palladium (0.4 g, 0.4 mmol) was added, the obtained mixture was reacted at 80°C for 6 hours. After the reaction was completed, the reaction solution was cooled to room temperature, and concentrated, and the residue was purified by silica gel column chromatography (eluent: methanol/dichloromethane = 1/10 (v/v)) to obtain the title compound (In-3) (0.9 g, yield: 75%).
MS m/z (ESI): 333.1 [M+H]⁺.

### Preparation Example 4 of Intermediate:

Preparation of 2-chloro-6-methyl-4-(4,4,5,5-tetramethyl-1,3,2-dioxaborolan-2-yl)pyridine (In-4)

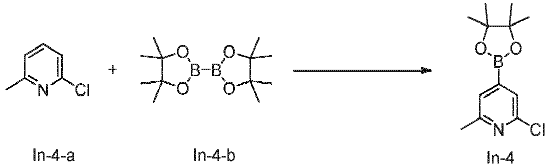

Under nitrogen atmosphere, bis(pinacolato)diborate (In-4-b) (3.0 g, 11.8 mmol), 4,4'-di-tert-butyl-2,2'-bipyridine (11 mg, 0.78 mmol) and (1,5-cyclooctadiene)(methoxy)iridium(I) dimer (520 mg, 0.78 mmol) were sequentially added into a solution of 2-methyl-6-chloropyridine (In-4-a) (1.0 g, 7.8 mmol) in cyclohexane (40 mL), and reacted at 80°C for 6 hours. After the reaction was completed, the reaction solution was cooled to room temperature, and concentrated, and the residue was purified by silica gel column chromatography (eluent: ethyl acetate/petroleum ether = 1/2 (v/v)) to obtain the title compound (In-4) (1.9 g, yield: 96%).
MS m/z (ESI): 254.1 [M+H]⁺.

### Preparation Example 5 of Intermediate:

Preparation of 2-(3-chloro-5-methylphenyl)-4,4,5,5-tetramethyl-1,3,2-dioxaborolane (In-5)

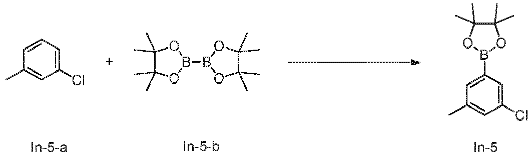

1-Chloro-3-toluene (In-5-a) was used to replace 2-methyl-6-chloropyridine in Preparation Example 4 of Intermediate, and a method similar to that of Preparation Example 4 of Intermediate was adopted to obtain the title compound (In-5) (3.91 g, yield: 81.1%).
MS m/z (ESI): 253.1 [M+H]⁺.

### Preparation Example 6 of Intermediate:

Preparation of 2-chloro-6-trifluoromethyl-4-(4,4,5,5-tetramethyl-1,3,2-dioxaborolan-2-yl)pyridine (In-6) 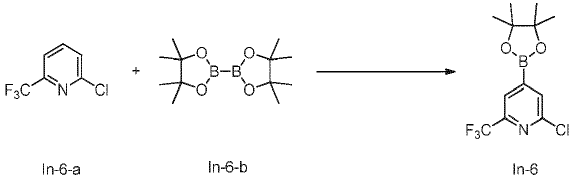 2-Chloro-6-trifluoromethylpyridine (In-6-a) was used to replace 2-methyl-6-chloropyridine in Preparation Example 4 of Intermediate, and a method similar to that of Preparation Example 4 of Intermediate was adopted to obtain the title compound (In-6) (1.5 g, yield: 88%).
MS m/z (ESI): 308.1 [M+H]⁺.

### Preparation Example 7 of Intermediate:

Preparation of 3-(5-bromo-4-(4-fluorophenyl)pyrimidin-2-yl)-1-methyl-1-(2-pyridylmethyl)urea (In-7)

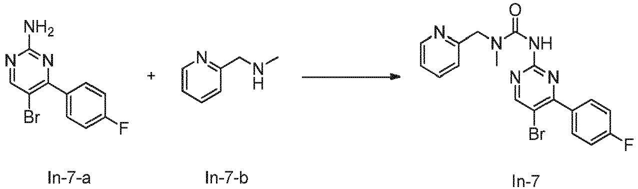

5-Bromo-4-(4-fluorophenyl)pyrimidin-2-amine (In-7-a) (300 mg, 1.12 mmol) was dissolved in a mixed solvent of tetrahydrofuran (5 mL) and DIPEA (1 mL), triphosgene (332 mg, 1.12 mmol) was added at 0°C, the obtained mixture was stirred for 0.5 hours at 0°C, and then *N*-methyl-1-pyridin-2-methylamine (In-7-b) (342 mg, 2.80 mmol) was added, the obtained mixture was reacted at 0°C for 1.5 hours. After the reaction was completed, methanol was added to quench the reaction, then the reaction solution was concentrated, and the residue was purified by preparative high performance liquid chromatography to obtain the title compound (In-7) (400 mg, yield: 86%).
MS m/z (ESI): 416.0 [M+H]⁺.

### Preparation Example 8 of Intermediate:

Preparation of *N*-(6-bromo-5-(5-methylfuran-2-yl)-1,2,4-triazin-3-yl)-[1,4'-bipiperidine]-1'-carboxamide (In-8)

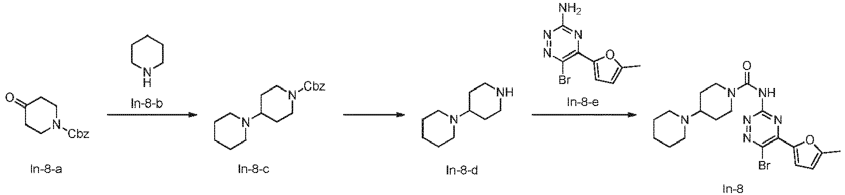

### Step 1: Preparation of benzyl [1,4'-bipiperidine]-1'-carboxylate (In-8-c)

At 0°C, piperidine (In-8- b) (1.83 g, 21.44 mmol) and tetraisopropyl titanate (6.70 g, 23.58 mmol) were added to a solution of benzyl 4-oxopiperidine-1-carboxylate (In-8-a) (5 g, 21.44 mmol) in dichloromethane (100 mL), and stirred at room temperature for 2 hours, then sodium cyanoborohydride (1.35 g, 21.44 mmol) was added in batches, and the obtained mixture was reacted at 25°C for 12 hours. Water was added to quench the reaction, and stirred for 10 minutes, then the obtained mixture stood still, and was subjected to suction filtration, the resulting filtrate was concentrated, the residue was dissolved in ethyl acetate, and subjected to suction filtration, the filtrate was concentrated, and the residue was purified by silica gel column chromatography (eluent: ethyl acetate/petroleum ether=1/3 (v/v)) to obtain the title compound (In-8-c) of this step (3.2 g, yield: 49.4%).
MS m/z (ESI): 303.2 [M+H]⁺.

### Step 2: Preparation of 1,4'-bipiperidine (In-8-d)

Benzyl [1,4'-bipiperidine]-1'-carboxylate (In-8-c) (3.2 g, 10.58 mmol) was dissolved in methanol (30 mL), palladium on carbon (0.3 g, 1.06 mmol) was added, the atmosphere of which was replaced with hydrogen gas three times, then the reaction was carried out for 12 hours under the hydrogen atmosphere. The reaction solution was filtered through diatomite, the filter cake was washed with a small amount of methanol, the resulting filtrate and methanol solution were combined, the resulting mixture was concentrated to obtain the title compound (In-8-d) (1.4 g, yield: 78.6%).
MS m/z (ESI): 169.2 [M+H]⁺.

### Step 3: Preparation of N-(6-bromo-5-(5-methylfuran-2-yl)-1,2,4-triazin-3-yl)-[1,4'-bipiperidine]-1'-carboxamide (In-8)

At 0°C, DIPEA (304 mg, 2.35 mmol) and phenyl chloroformate (245 mg, 1.57 mmol) were added into a solution of 6-bromo-5-(5-methylfuran-2-yl)-1,2,4-triazine-3-amine (In-8-e) (200 mg, 0.78 mmol) in tetrahydrofuran (10 mL), stirred at room temperature for 1.5 hours, then 1,4'-bipiperidine (In-8-d) (395 mg, 2.35 mmol) was added, the obtained mixture was reacted at room temperature for 4 hours. After the reaction was completed, water was added to quench the reaction, the reaction solution was concentrated, and the residue was purified by silica gel column chromatography (eluent: ethyl acetate/petroleum ether=1/3 (v/v)) to obtain the title compound (In-8) (250 mg, yield: 71%).
MS m/z (ESI): 449.1 [M+H]⁺.

### Preparation Example 9 of Intermediate:

Preparation of 5-(5-methylfuran-2-yl)-6-(4-methylquinazolin-6-yl)-1,2,4-triazine-3-amine (In-9)

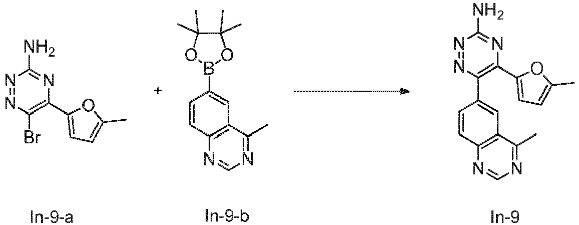

6-Bromo-5-(5-methylfuran-2-yl)-1,2,4-triazine-3-amine (In-9-a) (0.5 g, 1.9 mmol), 4-methyl- 6-(4,4,5,5-tetramethyl-1,3,2-dioxaborolan-2-yl)quinazoline (In-9-b) (0.65 g, 2.4 mmol) and potassium carbonate (0.53 g, 3.8 mmol) were added to a mixed solvent of 1,4-dioxane (10 mL) and water (4 mL), the atmosphere of which was replaced with nitrogen for three times, tetrakis(triphenylphosphine)palladium (0.1 g, 0.1 mmol) was added, and the obtained mixture was reacted at 80°C for 6 hours. After the reaction was completed, the reaction solution was cooled to room temperature, and concentrated, and the residue was purified by silica gel column chromatography (eluent: methanol/dichloromethane=1/10 (v/v)) to obtain the title compound (In-9) (0.45 g, yield: 71%).
MS m/z (ESI): 319.1 [M+H]⁺.
¹H NMR (400 MHz, DMSO-*d₆*) δ: 9.17 (s, 1H), 8.44 (s, 1H), 8.04 (d, *J* = 1.2 Hz, 2H), 7.46 (br, 2H), 6.34 (d, *J* = 3.6 Hz, 1H), 6.19 (d, *J* = 3.2 Hz, 1H), 2.91 (s, 3H), 2.18 (s, 3H).

### Preparation Example 10 of Intermediate:

Preparation of 5-(3-chloro-5-methylphenyl)-4-(4-fluorophenyl)pyrimidin-2-amine (In-10)

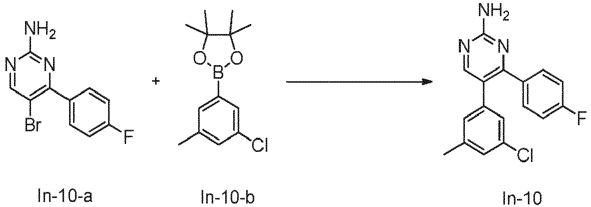

2-(3-Chloro-5-methylphenyl)-4,4,5,5-tetramethyl-1,3,2-dioxaborolane (In-10-b) was used to replace 4-methyl-6-(4,4,5,5-tetramethyl-1,3,2-dioxaborolan-2-yl)quinazoline of Preparation Example 9 of Intermediate, 5-bromo-4-(4-fluorophenyl)pyrimidin-2-amine (In-10-a) was used to replace 6-bromo-5-(5-methylfuran-2-yl)-1,2,4-triazine-3-amine of Preparation Example 9 of Intermediate, and a method similar to that of Preparation Example 9 of Intermediate was adopted to synthesize and obtain the title compound (In-10) (170 mg, yield: 72.6%).
MS m/z (ESI): 314.1 [M+H]⁺.

### Preparation Example 11 of Intermediate:

Preparation of 5-(3-chloro-5-methylphenyl)-4-(5-methylfuran-2-yl)pyrimidin-2-amine (In-11)

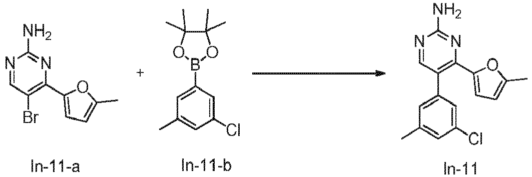

5-Bromo-4-(5-methylfuran-2-yl)pyrimidin-2-amine (In-11-a) was used to replace 6-bromo-5-(5-methylfuran-2-yl)-1,2.4-triazine-3-amine of of Preparation Example 9 of Intermediate, 2-(3-chloro-5-methylphenyl)-4,4,5,5-tetramethyl-1,3,2-dioxaborolane (In-11-b) was used to replace 4-methyl-6-(4,4,5,5-tetramethyl-1,3,2-dioxaborolan-2-yl)quinazoline of Preparation Example 9 of Intermediate, and a method similar to that of of Preparation Example 9 of Intermediate was adopted to synthesize and obtain the title compound (In-11) (220 mg, yield: 93.2%).
MS m/z (ESI): 300.1 [M+H]⁺.

### Preparation Example 12 of Intermediate:

Preparation of 6-(2-chloro-6-methylpyridin-4-yl)-5-(5-methylfuran-2-yl)-1,2,4-triazine-3-amine (In-12)

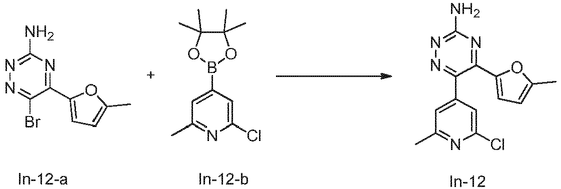

2-Chloro-6-methyl-4-(4,4,5,5-tetramethyl-1,3,2-dioxaborolan-2-yl)pyridine (In-12-b) was used to replace 4-methyl-6-(4,4,5,5-tetramethyl-1,3.2-dioxaborolan-2-yl)quinazoline of of Preparation Example 9 of Intermediate, and a method similar to that of of Preparation Example 9 of Intermediate was adopted to synthesize and obtain the title compound (In-12) (0.3 g, yield: 80%).
MS m/z (ESI): 302.1 [M+H]⁺.

### Example 1: Preparation of 6-(4-fluorophenyl)-5-(4-methylquinazolin-6-yl)pyridin-2-amine (Compound 1)

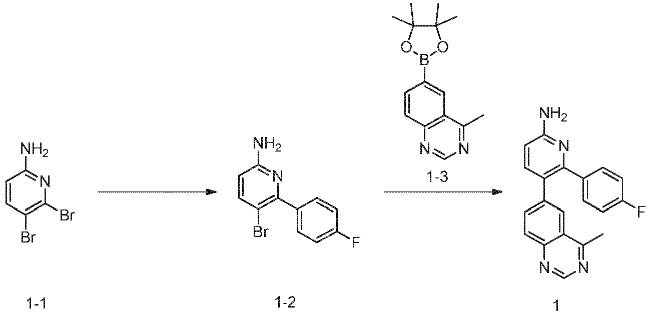

### Step 1: Preparation of 5-bromo-6-(4-fluorophenyl)pyridin-2-amine (1-2)

5,6-Dibromopyridin-2-amine (1-1) (2 g, 7.94 mmol), 4-fluorophenylboronic acid (1.11 g, 7.94 mmol) and sodium carbonate (1.68 g, 15.88 mmol) were added to a mixed solvent of toluene (40 mL), methanol (4 mL) and water (8 mL), the atmosphere of which was replaced with nitrogen gas for three times, tetrakis(triphenylphosphine) palladium (459 mg, 0.4 mmol) was added, and the obtained mixutre was reacted at 95°C for 11 hours. After the reaction was completed, the reaction solution was cooled to room temperature, and concentrated, and the residue was purified by silica gel column chromatography (eluent: ethyl acetate/petroleum ether = 1/3 (v/v)) to obtain the title compound (1-2) (2 g, yield: 94.31%).
MS m/z (ESI): 267.0 [M+H]⁺.

### Step 2: Preparation of 6-(4-fluorophenyl)-5-(4-methylquinazolin-6-yl)pyridin-2-amine (1)

5-Bromo-6-(4-fluorophenyl)pyridin-2-amine (1-2) (1.14 g, 4.27 mmol), 4-methyl-6-(4,4,5,5-tetramethyl-1,3,2-dioxaborolan-2-yl)quinazoline (1-3) (1.5 g, 5.55 mmol) and potassium carbonate (1.18 g, 8.54 mmol) were added to a mixed solvent of 1,4-dioxane (20 mL) and water (2 mL), the atmosphere of which was replaced with nitrogen gas for three times, tetrakis(triphenylphosphine)palladium (246.9 mg, 0.21 mmol) was added, and the obtained mixture was reacted at 95°C for 12 hours. After the reaction was complete, the reaction solution was cooled to room temperature, and filtered, the filtrate was concentrated, and the residue was purified by silica gel column chromatography (eluent: ethyl acetate/petroleum ether=3/2 (v/v)) to obtain the title compound (1) (1.15 g, yield: 81.5%).
MS m/z (ESI): 331.1 [M+H]⁺.
¹H NMR (400 MHz, DMSO-*d₆*) δ: 9.05 (s, 1H), 8.01 (d, *J* = 1.6 Hz, 1H), 7.75 (d, *J* = 8.4 Hz, 1H), 7.68 (d, *J* = 8.4 Hz, 1H), 7.53 (dd, *J* = 8.8, 2 Hz, 1H), 7.30-7.27 (m, 2H), 7.06 (t, *J* = 9.2 Hz, 2H), 6.60 (d, *J* = 8.4 Hz, 1H), 6.31 (s, 2H), 2.79 (s, 3H).

### Example 2: Preparation of 3-fluoro-6-(4-fluorophenyl)-5-(4-methylquinazolin-6-yl)pyridin-2-amine (Compound 2)

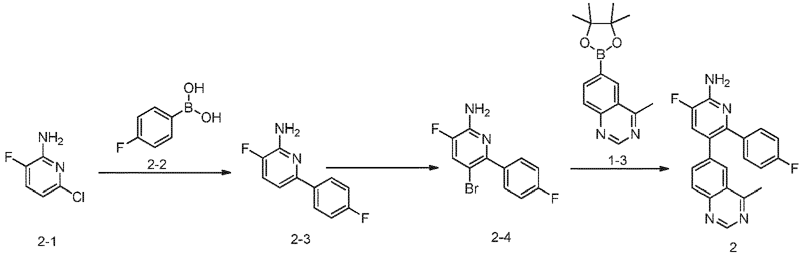

### Step 1: Preparation of 3-fluoro-6-(4-fluorophenyl)pyridin-2-amine (2-3)

6-Chloro-3-fluoropyridin-2-amine (2-1) (220 mg, 1.5 mmol) was added to a mixed solvent of 1,4-dioxane (5 mL) and water (1 mL), 4-fluorophenylboronic acid (315 mg, 2.3 mmol), potassium phosphate (637 mg, 3.0 mmol) and Pd(amphos)Cl₂ (53 mg, 0.08 mmol) were added sequentially, the atmosphere of which was replaced with nitrogen gas, the reaction solution was heated to 120°C with microwave, and reacted for 2 hours. After the reaction was completed, the reaction solution was cooled to room temperature, poured into water, and extracted with ethyl acetate. The organic phases were combined, dried over anhydrous sodium sulfate and filtered. The filtrate was concentrated, and the residue was purified by silica gel column chromatography (eluent: ethyl acetate/petroleum ether = 1/2 (v/v)) to obtain the title compound (2-3) of this step (240 mg, yield: 78%).
MS m/z (ESI): 207.1 [M+H]⁺.

### Step 2: Preparation of 5-bromo-3-fluoro-6-(4-fluorophenyl)pyridin-2-amine (2-4)

3-Fluoro-6-(4-fluorophenyl)pyridin-2-amine (2-3) (220 mg, 1.1 mmol) was added to anhydrous DMF (5 mL), cooled to 0°C, *N*-bromo-succinimide (285 mg, 1.6 mmol) was added, warmed to room temperature and reacted for 2 hours. The reaction solution was diluted with water, and extracted with ethyl acetate. The organic phases were combined, dried over anhydrous sodium sulfate, and filtered. The filtrate was concentrated, and the residue was purified by silica gel column chromatography (eluent: ethyl acetate/petroleum ether = 1/1 (v/v)) to obtain the title compound (2-4) of this step (150 mg, yield: 49%).
MS m/z (ESI): 285.0 [M+H]⁺.

### Step 3: Preparation of 3-fluoro-6-(4-fluorophenyl)-5-(4-methylquinazolin-6-yl)pyridin-2-amine (2)

5-Bromo-3-fluoro-6-(4-fluorophenyl)pyridin-2-amine (2-4) (150 mg, 0.53 mmol) was dissolved in a mixed solvent of 1,4-dioxane (5 mL) and water (1 mL), 4-methyl-6-(4,4,5,5-tetramethyl-1,3,2-dioxaborolan-2-yl)quinazoline (1-3) (213 mg, 0.79 mmol), potassium carbonate (146 mg, 1.1 mmol) and Pd(dppf)Cl₂ (22 mg, 0.026 mmol) were added sequentially, the atmosphere of which was replaced with nitrogen for three times. The obtained mixture was reacted at 80°C overnight. After the reaction was complete, the reaction solution was cooled to room temperature, poured into water, and extracted with ethyl acetate. The organic phases were combined, dried over anhydrous sodium sulfate, and filtered. The filtrate was concentrated, and the residue was purified by silica gel column chromatography (eluent: ethyl acetate/petroleum ether) =3/1 (v/v)) to obtain the title compound (2) (120 mg, yield: 62%).
MS m/z (ESI): 349.1 [M+H]⁺.
¹H NMR (400 MHz, DMSO-*d₆*) δ: 9.06 (s, 1H), 8.09 (d, *J* = 2.0 Hz, 1H), 7.77-7.61 (m, 2H), 7.55-7.53 (m, 1H), 7.27-7.25 (m, 2H), 7.06 (t, *J* = 8.8 Hz, 2H), 6.61 (s, 2H), 2.81 (s, 3H).

### Example 3: Preparation of 4-(4-fluorophenyl)-5-(4-methylquinazolin-6-yl)pyrimidin-2-amine (Compound 3)

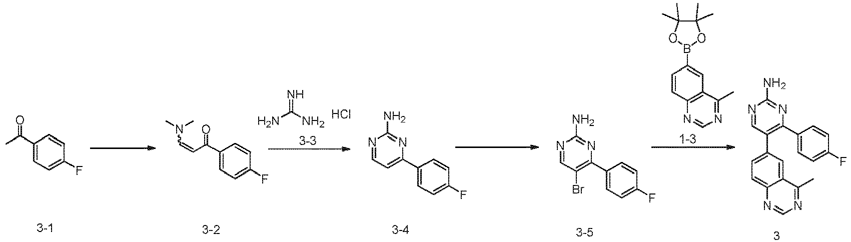

### Step 1: Preparation of 3-(dimetliylamino)-1-(4-fluorophenyl)prop-2-en-1-one (3-2)

1-(4-Fluorophenyl)ethanone (3-1) (5.0 g, 40.3 mmol) was added to *N*,*N*-dimethylformamide dimethyl acetal (50 mL), the atmosphere of which was replaced with nitrogen gas, and the obtained mixture was reacted overnight at 80°C. After the reaction was completed, the reaction solution was cooled to room temperature, and concentrated. The residue was slurried in anhydrous ether, filtered with suction. The filter cake was washed with anhydrous ether, and dried in vacuo to obtain the title compound (3-2) (2.1 g, yield: 30%).
MS m/z (ESI): 194.1 [M+H]⁺.

### Step 2: Preparation of 4-(4-fluorophenyl)pyrimidin-2-amine (3-4)

3-(Dimethylamino)-1-(4-fluorophenyl)prop-2-en-1-one (3-2) (2.1 g, 10.9 mmol), guanidine hydrochloride (3-3) (1.2 g, 12.0 mmol) and potassium carbonate (3.0 g, 21.8 mmol) were added to anhydrous ethanol (20 mL), and reacted at 80°C for 20 hours. The reaction solution was concentrated. The residue was diluted with water, filtered with suction. The filter cake was washed with anhydrous ether, and dried in vacuo to obtain the title compound (3-4) of this step (1.9 g, yield: 92%).
MS m/z (ESI): 190.1 [M+H]⁺.

### Step 3: Preparation of 5-bromo-4-(4-fluorophenyl)pyrimidin-2-amine (3-5)

4-(4-Fluorophenyl)pyrimidin-2-amine (3-4) (1.9 g, 10.1 mmol) was added to anhydrous DMF (20 mL), cooled to 0°C, *N*-bromo-succinimide (2.0 g, 11.1 mmol) was added, and stirred at 0°C for 1 hour, then naturally warmed to room temperature and stirred overnight. The reaction solution was concentrated, the residue was diluted with water, filtered with suction. The filter cake was washed with anhydrous ether, and dried in vacuo to obtain the title compound (3-5) of this step (2.6 g, yield: 95%).
MS m/z (ESI): 268.0 [M+H]⁺.

### Step 4: Preparation of 4-(4-fluorophenyl)-5-(4-methylquinazolin-6-yl)pyrimidin-2-amine (3)

5-Bromo-4-(4-fluorophenyl)pyrimidin-2-amine (3-5) (1.0 g, 3.7 mmol) was added to a mixed solvent of 1,4-dioxane (100 mL) and water (4 mL), then 4-methyl-6-(4,4,5,5-tetramethyl-1,3,2-dioxaborolan-2-yl)quinazoline (1-3) (1.2 g, 4.5 mmol), potassium carbonate (1.1 g, 7.5 mmol) and Pd(dppf)Cl₂ (0.3 g, 0.4 mmol) were add sequentially, the atmosphere of which was replaced with nitrogen gas for three times, and the obtained mixture was reacted at 100°C for 4 hours. After the reaction was complete, the reaction solution was cooled to room temperature, poured into water, and extracted with ethyl acetate. The organic phases were combined, dried over anhydrous sodium sulfate, and filtered. The filtrate was concentrated, and the residue was purified by silica gel column chromatography (eluent: ethyl acetate/petroleum ether=3 /1 (v/v)) to obtain the title compound (3) (0.9 g, yield: 73%).
MS m/z (ESI): 332.1 [M+H]⁺.
¹H NMR (400 MHz, DMSO-*d₆*) δ: 9.08 (s, 1H), 8.51 (s, 1H), 8.18 (d, *J* = 1.6 Hz, 1H), 7.79 (d, *J* = 8.8 Hz, 1H), 7.53-7.50 (m, 1H), 7.42-7.31 (m, 2H), 7.14 (t, *J* = 8.8 Hz, 2H), 7.02 (s, 2H), 2.86 (s, 3H).

### Example 4: Preparation of 3-(3-fluoro-6-(4-fluorophenyl)-5-(4-methylquinazolin-6-yl)pyridin-2-yl)-1-methyl-1-(pyridin-2-ylmethyl)urea (Compound 4)

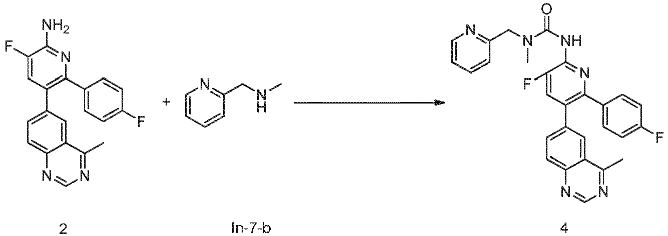

3-Fluoro-6-(4-fluorophenyl)-5-(4-methylquinazolin-6-yl)pyridin-2-amine (2) (50 mg, 0.14 mmol) was dissolved in a mixed solvent of tetrahydrofuran (2 mL) and DIPEA (0.5 mL), then triphosgene (43 mg, 0.14 mmol) was added at 0°C. The mixture was stirred at 0°C for 0.5 hours, *N*-methyl-1-pyridine-2-methylamine (In-7-b) (35 mg, 0.28 mmol) was added. The mixture was reacted at 0°C for 1.5 hours. After the reaction was completed, methanol was added to quench the reaction. The reaction solution was concentrated, and the residue was purified by preparative high performance liquid chromatography to obtain the title compound (4) (25 mg, yield: 30 %).
MS m/z (ESI): 497.2 [M+H]⁺.
¹H NMR (400 MHz, DMSO-*d₆*) δ: 9.57 (s, 1H), 9.11 (s, 1H), 8.58 (d, *J* = 4.4 Hz, 1H), 8.27 (d, *J* = 1.6 Hz, 1H), 8.06 (d, *J* = 10.4 Hz, 1H), 7.85-7.82 (m, 2H), 7.64-7.61 (m, 1H), 7.36-7.32 (m, 4H), 7.13-7.09 (m, 2H), 4.68 (s, 2H), 3.04 (s, 3H), 2.86 (s, 3H).

### Example 5: Preparation of 1-(4-(4-fluorophenyl)-5-(4-methylquinazolin-6-yl)pyrimidin-2-yl)-3-methylurea (Compound 5)

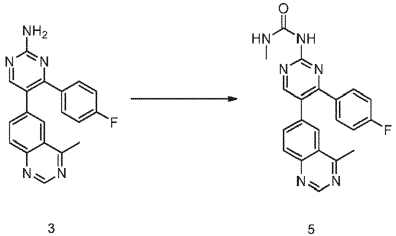

4-(4-Fluorophenyl)-5-(4-methylquinazolin-6-yl)pyrimidin-2-amine (3) (100.0 mg, 0.3 mmol) was dissolved in tetrahydrofuran (15 mL), DIPEA (0.6 mL, 3.2 mmol) and triphosgene (89.0 mg, 0.3 mmol) were added at -40°C, stirred at -40°C for 1 hour, then a solution of methylamine in tetrahydrofuran (0.3 mL, 2 mol/L, 0.6 mmol) was added, and reacted at 0°C for 2 hours. After the reaction was completed, water was added to the reaction solution, the obtained mixture was extracted with ethyl acetate. The organic phases were combined, dried over anhydrous sodium sulfate, and filtered. The filtrate was concentrated, and the residue was purified by silica gel column chromatography (eluent: ethyl acetate) to obtain the title compound (5) (20.0 mg, yield: 17%).
MS m/z (ESI): 389.1 [M+H]⁺.
¹H NMR (400 MHz, DMSO-*d₆*) δ: 10.06 (s, 1H), 9.12 (s, 1H), 8.91 (d, *J* = 4.8 Hz, 1H), 8.81 (s, 1H), 8.32 (d, *J* = 1.6 Hz, 1H), 7.86 (d, *J* = 8.4 Hz, 1H), 7.60-7.58 (m, 1H), 7.52-7.37 (m, 2H), 7.21-7.17 (m, 2H), 2.88 (s, 3H), 2.83 (d, *J* = 4.4 Hz, 3H).

### Example 6: Preparation of 1-(4-(4-fluorophenyl)-5-(4-methylquinazolin-6-yl)pyrimidin-2-yl)-3-(pyridine-2-methyl)urea (Compound 6)

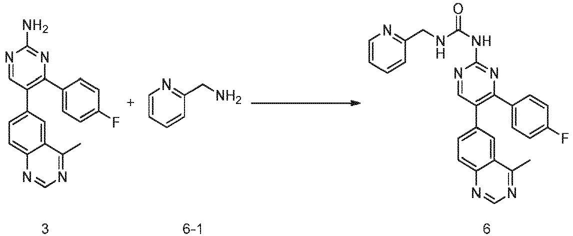

2-Aminomethylpyridine (6-1) was used to replace the solution of methylamine in tetrahydrofuran in Example 5, and a method similar to that of Example 5 was adopted to synthesize and obtain the title compound (6) (30.0 mg, yield: 21%).
MS m/z (ESI): 466.2 [M+H]⁺.
¹H NMR (400 MHz, DMSO-*d₆*) δ: 10.21 (s, 1H), 9.74 (t, *J* = 5.4 Hz, 1H), 9.12 (s, 1H), 8.85 (s, 1H), 8.51-8.50 (m, 1H), 8.34 (d, *J* = 1.6 Hz, 1H), 7.86-7.77 (m, 2H), 7.61-7.59 (m, 1H), 7.51-7.44 (m, 2H), 7.42 (d, *J* = 8.0 Hz, 1H), 7.33-7.30 (m, 1H), 7.18-7.14 (m, 2H), 4.61 (d, *J* = 5.2 Hz, 2H), 2.88 (s, 3H).

### Example 7: Preparation of 1-(4-(4-fluorophenyl)-5-(4-methylquinazolin-6-yl)pyrimidin-2-yl)-3-(2-hydroxy-2-methylpropyl)urea (Compound 7)

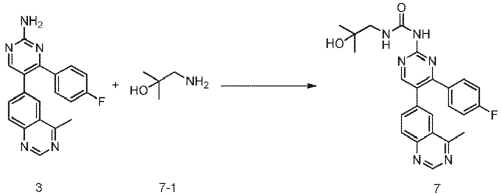

1-Amino-2-methyl-2-propanol (7-1) was used to replace the solution of methylamine in tetrahydrofuran in Example 5, and a method similar to that of Example 5 was adopted to synthesize and obtain the title compound (7) (35.0 mg, yield: 28%).
MS m/z (ESI): 447.2 [M+H]⁺.
¹H NMR (400 MHz, DMSO-*d₆*) δ: 10.02 (s, 1H), 9.27 (t, *J* = 5.6 Hz, 1H), 9.12 (s, 1H), 8.84 (s, 1H), 8.36 (d, *J* = 1.6 Hz, 1H), 7.85 (d, *J* = 8.4 Hz, 1H), 7.67-7.43 (m, 3H), 7.15 (t, *J* = 8.8 Hz, 2H), 4.60 (s, 1H), 3.23 (d, *J* = 5.6 Hz, 2H), 2.89 (s, 3H), 1.13 (s, 6H).

### Example 8: Preparation of 1-[4-(4-fluorophenyl)-5-(4-methylquinazolin-6-yl)pyrimidin-2-yl]-2-(2-methoxyethyl)urea (Compound 8)

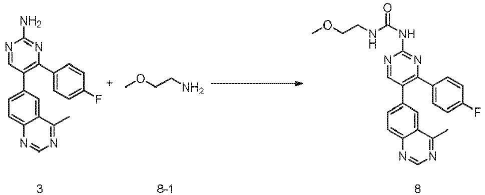

2-Methoxyethylamine (8-1) was used replace the solution of methylamine in tetrahydrofuran in Example 5, and a method similar to that of Example 5 was adopted to synthesize and obtain the title compound (8) (50.0 mg, yield: 38%).
MS m/z (ESI): 433.2 [M+H]⁺.
¹H NMR (400 MHz, DMSO-*d₆*) δ: 10.09 (s, 1H), 9.24 (s, 1H), 9.12 (s, 1H), 8.84 (s, 1H), 8.34 (d, *J* = 1.6 Hz, 1H), 7.85 (d, *J* = 8.8 Hz, 1H), 7.60-7.58 (m, 1H), 7.55-7.40 (m, 2H), 7.18 (t, *J* = 8.8 Hz, 2H), 3.46-3.43 (m, 4H), 3.26 (s, 3H), 2.89 (s, 3H).

### Example 9: Preparation of 3-(4-(4-fluorophenyl)-5-(4-methylquinazolin-6-yl)pyrimidin-2-yl)- 1-methyl-1-(pyridine-2-methyl)urea (Compound 9)

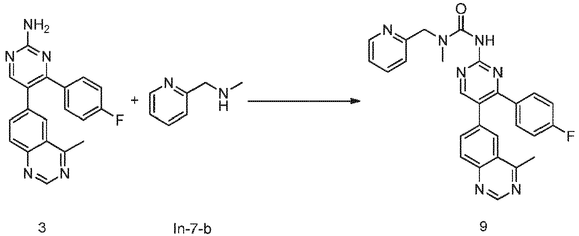

*N*-methyl-1-pyridine-2-methylamine (In-7-b) was used to replace the solution of methylamine in tetrahydrofuran in Example 5, and a method similar to that of Example 5 was adopted to synthesize and obtain the title compound (9) (50.0 mg, yield: 36%).
MS m/z (ESI): 480.2 [M+H]⁺.
¹H NMR (400 MHz, DMSO-*d₆*) δ: 10.0 (s, 1H), 9.12 (s, 1H), 8.80 (s, 1H), 8.59 (d, *J* = 4.4 Hz, 1H), 8.32 (d, *J* = 1.6 Hz, 1H), 7.98-7.68 (m, 2H), 7.62-7.61 (m, 1H), 7.51-7.27 (m, 4H), 7.17 (t, *J* = 8.8 Hz, 2H), 4.69 (s, 2H), 3.03 (s, 3H), 2.89 (s, 3H).

### Example 10: Preparation of 4-cyano-N-(4-(4-fluorophenyl)-5-(4-methylquinazolin-6-yl)pyrimidin-2-yl) piperidine-1-carboxamide (Compound 10)

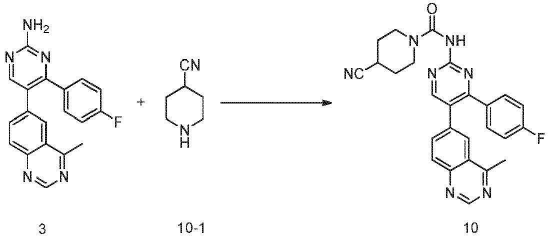

4-Cyanopiperidine (10-1) was used to replace the solution of methylamine in tetrahydrofuran in Example 5, and a method similar to that of Example 5 was adopted to synthesize and obtain the title compound (10) (32.0 mg, yield: 23%).
MS m/z (ESI): 468.2 [M+H]⁺.
¹H NMR (400 MHz, DMSO-*d₆*) δ: 9.90 (s, 1H), 9.12 (s, 1H), 8.79 (s, 1H), 8.32 (d, *J* = 2.0 Hz, 1H), 7.85 (d, *J* = 8.8 Hz, 1H), 7.61-7.59 (m, 1H), 7.52-7.36 (m, 2H), 7.16 (t, *J* = 8.4 Hz, 2H), 3.81-3.65 (m, 2H), 3.34-3.30 (m, 2H), 3.13 (dt, *J* = 12.8, 4.8 Hz, 1H), 3.04-2.82 (m, 3H), 2.00-1.83 (m, 2H), 1.81-1.61 (m, 2H).

### Example 11: Preparation of N-(4-(4-fluorophenyl)-5-(4-methylquinazolin-6-yl)pyrimidin-2-yl) morpholine-4-carboxamide (Compound 11)

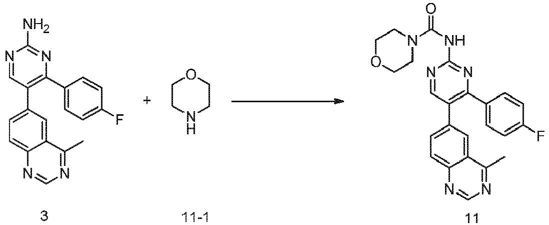

Morpholine (11-1) was used to replace the solution of methylamine in tetrahydrofuran in Example 5, and a method similar to that of Example 5 was adopted to synthesize and obtain the title compound (11) (50.0 mg, yield: 39%).
MS m/z (ESI): 445.2 [M+H]⁺.
¹H NMR (400 MHz, DMSO-*d₆*) δ: 9.89 (s, 1H), 9.12 (s, 1H), 8.79 (s, 1H), 8.32 (d, *J* = 1.6 Hz, 1H), 7.85 (d, *J* = 8.4 Hz, 1H), 7.62-7.59 (m, 1H), 7.46-7.43 (m, 2H), 7.17 (t, *J* = 8.8 Hz, 2H), 3.68-3.56 (m, 4H), 3.56-3.43 (m, 4H), 2.89 (s, 3H).

### Example 12: Preparation of 3-(4-(4-fluorophenyl)-5-(4-methylquinazolin-6-yl)pyrimidin-2-yl)-1-methyl-1-phenylurea (Compound 12)

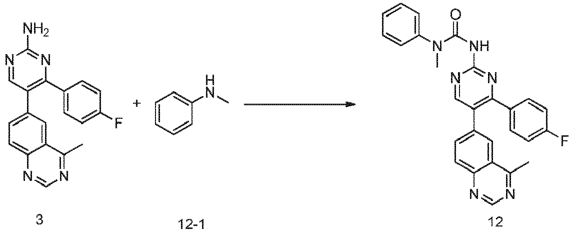

*N*-methyl aniline (12-1) was used to replace the solution of methylamine in tetrahydrofuran in Example 5, and a method similar to that of Example 5 was adopted to synthesize and obtain the title compound (12) (50.0 mg, yield: 36%).
MS m/z (ESI): 465.2 [M+H]⁺.
¹H NMR (400 MHz, DMSO-*d₆*) δ: 9.36 (s, 1H), 9.12 (s, 1H), 8.76 (s, 1H), 8.31 (d, *J* = 1.6 Hz, 1H), 7.85 (d, *J* = 8.4 Hz, 1H), 7.60-7.58 (m, 1H), 7.49-7.32 (m, 6H), 7.31-7.22 (m, 1H), 7.20-7.07 (m, 2H), 3.34(s, 3H), 2.88 (s, 3H).

### Example 13: Preparation of N-(4-(4-fluorophenyl)-5-(4-methylquinazolin-6-yl)pyrimidin-2-yl)-4-methoxypiperidine-1-carboxamide (Compound 13)

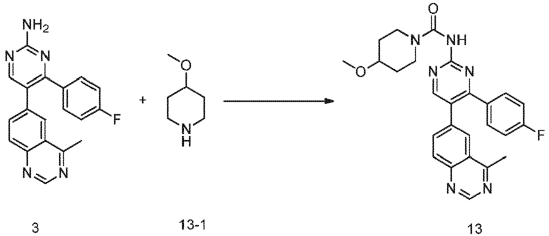

4-Methoxypiperidine (13-1) was used to replace the solution of methylamine in tetrahydrofuran in Example 5, and a method similar to that of Example 5 was adopted to synthesize and obtain the title compound (13) (55.0 mg, yield: 38%).
MS m/z (ESI): 473.2 [M+H]⁺.
¹H NMR (400 MHz, DMSO-*d₆*) δ: 9.82 (s, 1H), 9.12 (s, 1H), 8.77 (s, 1H), 8.31 (d, *J* = 1.6 Hz, 1H), 7.85 (d, *J* = 8.8 Hz, 1H), 7.61-7.59 (m, 1H), 7.52-7.36 (m, 2H), 7.16-7.14 (m, 2H), 3.80-3.75 (m, 2H), 3.49-3.37 (m, 1H), 3.27 (s, 3H), 3.25-3.13 (m, 2H), 2.88 (s, 3H), 1.94-1.77 (m, 2H), 1.48-1.20 (m, 2H).

### Example 14: Preparation of 3-(5-(2-chloro-6-methylpyridin-4-yl)-4-(4-fluorophenyl)pyrimidin-2-yl)-1-methyl-1-(pyridin-2-ylmethyl)urea (Compound 14)

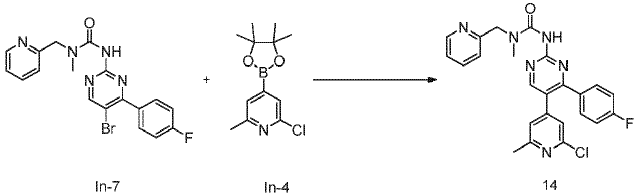

3-(5-Bromo-4-(4-fluorophenyl)pyrimidin-2-yl)-1-methyl-1-(pyridin-2-ylmethyl)-urea (In-7) (50 mg, 0.12 mmol) was dissolved in a mixed solvent of 1,4-dioxane (5 mL) and water (1 mL), then 2-chloro-6-methyl-4-(4,4,5,5-tetramethyl-1,3,2-dioxaborolan-2-yl)pyridine (In-4) (46 mg, 0.18 mmol), potassium carbonate (33 mg, 0.24 mmol) and Pd(dppf)Cl₂ (5 mg, 0.006 mmol) were added sequentially, the atmosphere of which was replaced with nitrogen gas for three times. The obtained mixture was warmed up to 80°C, and reacted overnight. After the reaction was completed, the reaction solution was cooled to room temperature and poured into water, and extracted with ethyl acetate. The organic phases were combined, dried over anhydrous sodium sulfate, and filtered. The filtrate was concentrated, and the residue was purified by preparative high performance liquid chromatography to obtain the title compound (14) (10 mg, yield: 18%).
MS m/z (ESI): 463.1 [M+H]⁺.
¹H NMR (400 MHz, DMSO-*d₆*) δ: 10.06 (s, 1H), 8.65 (s, 1H), 8.57 (d, *J* = 4.4 Hz, 1H), 7.82-7.79 (m, 1H), 7.46-7.43 (m, 2H), 7.38 (d, *J* = 8.0 Hz, 1H), 7.34-7.31 (m, 1H), 7.25-7.23 (m, 2H), 7.16 (d, *J* = 1.2 Hz, 2H), 4.67 (s, 2H), 3.01 (s, 3H), 2.39 (s, 3H).

### Example 15: Preparation of 3-(5-(3-chloro-5-methylphenyl)-4-(4-fluorophenyl)pyrimidin-2-yl)-1-methyl-1-(pyridin-2-ylmethyl)urea (Compound 15)

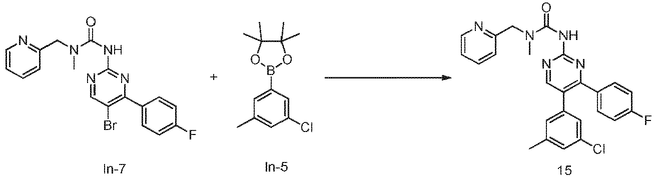

2-(3-Chloro-5-methylphenyl)-4,4,5,5-tetramethyl-1,3,2-dioxaborolane (In-5) was used to replace 2-chloro-6-methyl-4-(4,4,5,5-tetramethyl-l,3,2-dioxaborolan-2-yl)pyridine (In-4) in Example 14, and a method similar to that of Example 14 was adopted to synthesize and obtain the title compound (15) (15 mg, yield: 11%).
MS m/z (ESI): 462.1 [M+H]⁺.
¹H NMR (400 MHz, DMSO-*d₆*) δ: 9.95 (s, 1H), 8.58 (d, *J* = 5.6 Hz, 2H), 7.83-7.79 (m, 1H), 7.45-7.38 (m, 3H), 7.34-7.31 (m, 1H), 7.23-7.19 (m, 3H), 7.05 (d, *J* = 11.6 Hz, 2H), 4.67 (s, 2H), 3.01 (s, 3H), 2.25 (s, 3H).

### Example 16: Preparation of 3-(5-(2-chloro-6-(trifluoromethyl)pyridin-4-yl)-4-(4-fluorophenyl) pyrimidin-2-yl)-1-methyl-1-(pyridin-2-ylmethyl)urea (Compound 16)

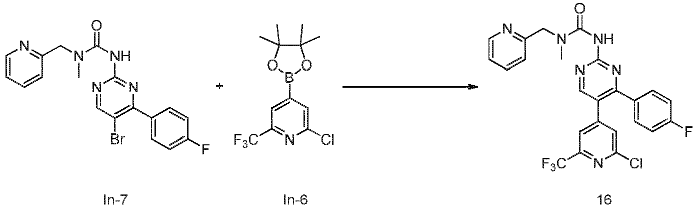

2-Chloro-6-trifluoromethyl-4-(4,4,5,5-tetramethyl-1,3,2-dioxaborolan-2-yl)pyridine (In-6) was used to replace 2-chloro-6-methyl-4-(4,4,5,5-tetramethyl-1,3,2-dioxaborolan-2-yl)pyridine (In-4) in Example 14, and a method similar to that of Example 14 was adopted to synthesize and obtain the title compound (16) (6 mg, yield: 5%).
MS m/z (ESI): 517.1 [M+H]⁺.
¹H NMR (400 MHz, DMSO-*d*₆) δ: 10.15 (s, 1H), 8.80 (s, 1H), 8.57 (d, *J* = 4.4 Hz, 1H), 7.83-7.80(m, 2H), 7.66 (s, 1H), 7.47-7.44 (m, 2H), 7.39 (d, *J* = 7.6 Hz, 1H), 7.34-7.31 (m, 1H), 7.26 (t, *J* = 8.8 Hz, 2H), 4.68 (s, 2H), 3.02 (s, 3H).

### Example 17: Preparation of 5-(2-chloro-6-methylpyridin-4-yl)-4-(4-fluorophenyl)pyrimidin-2-amine (Compound 17)

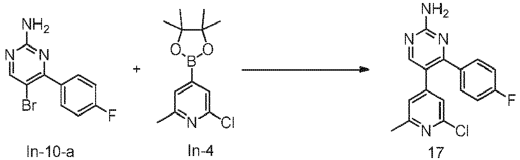

5-Bromo-4-(4-fluorophenyl)pyrimidin-2-amine (In-10-a) (200 mg, 0.75 mmol), 2-chloro-6-methyl-4-(4,4,5,5-tetramethyl-1,3,2-dioxaborolan-2-yl)pyridine (In-4) (283 mg, 1.12 mmol) and potassium carbonate (206 mg, 1.49 mmol) were added to a mixed solvent of 1,4-dioxane (5 mL) and water (1 mL), the atmosphere of which was replaced with nitrogen gas for three times. [1,1'-Bis(diphenylphosphino)ferrocene]dichloropalladium (30 mg, 0.037 mmol) was added, and the obtained mixture was reacted at 80°C for 12 hours. After the reaction was complete, the reaction solution was cooled to room temperature, and filtered. The filtrate was concentrated, and the residue was purified by silica gel column chromatography (eluent: ethyl acetate/petroleum ether = 1/1 (v/v)) to obtain the title compound (17) (220 mg, yield: 93.7%).
MS m/z (ESI): 315.1 [M+H]⁺.
¹H NMR (400 MHz, DMSO-*d*₆) δ: 8.37 (s, 1H), 7.39-7.35 (m, 2H), 7.21 (t, *J* = 8.8 Hz, 2H), 7.12 (s, 2H), 7.04 (s, 1H), 6.99 (s, 1H), 2.35 (s, 3H).

### Example 18: Preparation of N-(5-(2-chloro-6-methylpyridin-4-yl)-4-(4-fluorophenyl)pyrimidin-2-yl)-4-cyanopiperidine-1-carboxamide (Compound 18)

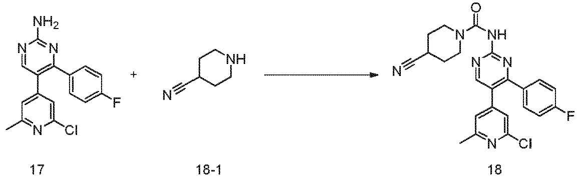

4-Cyanopiperidine (18-1) was used to replace the solution of methylamine in tetrahydrofuran in Example 5, 5-(2-chloro-6-methylpyridin-4-yl)-4-(4-fluorophenyl)pyrimidin-2-amide (17) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (18) (20 mg, yield: 16.6%).
MS m/z (ESI): 451.1 [M+H]⁺.
¹H NMR (400 MHz, DMSO-*d*₆) δ: 9.94 (s, 1H), 8.63 (s, 1H), 7.45-7.41 (m, 2H), 7.24 (t, *J* = 8.8 Hz, 2H), 7.15 (s, 2H), 3.73-3.69 (m, 2H), 3.31-3.27 (m, 2H), 3.11-3.09 (m, 1H), 2.39 (s, 3H), 1.92-1.88 (m, 2H), 1.73-1.68 (m, 2H).

### Example 19: Preparation of N-(5-(2-chloro-6-methylpyridin-4-yl)-4-(4-fluorophenyl)pyrimidin-2-yl) morpholine-4-carboxamide (Compound 19)

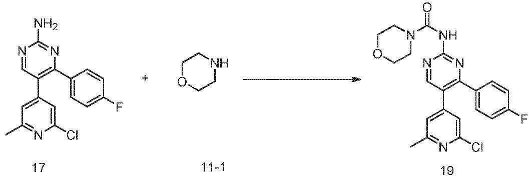

Morpholine (11-1) was used to replace the solution of methylamine in tetrahydrofuran in Example 5, 5-(2-chloro-6-methylpyridin-4-yl)-4-(4-fluorophenyl)pyrimidin-2-amine (17) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (19) (40 mg, yield: 14.0%).
MS m/z (ESI): 427.1 [M+H]⁺.
¹H NMR (400 MHz, DMSO-*d*₆) δ: 9.94 (s, 1H), 8.64 (s, 1H), 7.45-7.42 (m, 2H), 7.24 (t, *J* = 8.8 Hz, 2H), 7.15 (s, 2H), 3.61-3.58 (m, 4H), 3.47-3.45 (m, 4H), 2.39 (s, 3H).

### Example 20: Preparation of N-(5-(3-chloro-5-methylphenyl)-4-(4-fluorophenyl)pyrimidin-2-yl)-4-cyanopiperidine-1-carboxamide (Compound 20)

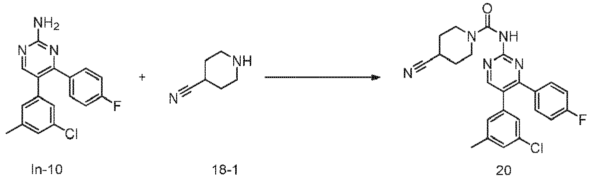

4-Cyanopiperidine (18-1) was used to replace the solution of methylamine in tetrahydrofuran in Example 5, 5-(3-chloro-5-methylphenyl)-4-(4-fluorophenyl)pyrimidin-2-amine (In-10) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (20) (50 mg, yield: 43.7%).
MS m/z (ESI): 450.1 [M+H]⁺.
¹H NMR (400 MHz, DMSO-*d*₆) δ: 9.82 (s, 1H), 8.56 (s, 1H), 7.43-7.40 (m, 2H), 7.23-7.20 (m, 3H), 7.04 (d, *J* = 11.2 Hz, 2H), 3.74-3.68 (m, 2H), 3.31-3.29 (m, 2H), 3.11-3.10 (m, 1H), 2.25 (s, 3H), 1.92-1.88 (m, 2H), 1.73-1.68 (m, 2H).

### Example 21: Preparation of N-(5-(3-chloro-5-methylphenyl)-4-(4-fluorophenyl)pyrimidin-2-yl)-[1,4'-dipiperidine]-1'-carboxamide (Compound 21)

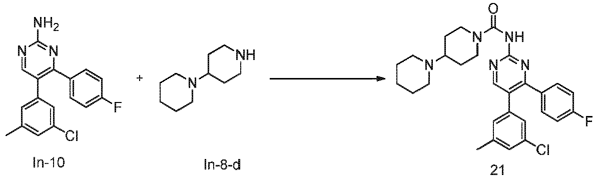

1,4'-Dipiperidine (In-8-d) was used to replace the solution of methylamine in tetrahydrofuran in Example 5, 5-(3-chloro-5-methylphenyl)-4-(4-fluorophenyl)pyrimidin-2-amine (In-10) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (21) (25 mg, yield: 15.7*%*).
MS m/z (ESI): 508.2 [M+H]⁺.
¹H NMR (400 MHz, DMSO-*d*₆) δ: 9.69 (s, 1H), 8.54 (s, 1H), 8.19 (s, 1H), 7.43-7.39 (m, 2H), 7.23-7.18 (m, 2H), 7.03 (d, *J* = 10.8 Hz, 2H), 4.12 (d, *J* = 13.2 Hz, 2H), 2.83-2.76 (m, 2H), 2.50-2.44 (m, 5H), 2.25 (s, 3H), 1.70 (d, *J* = 12.4 Hz, 2H), 1.53-1.45 (m, 4H), 1.41-1.32 (m, 4H).

### Example 22: Preparation of N-(5-(3-chloro-5-methylphenyl)-4-(5-methylfuran-2-yl)pyrimidin-2-yl)-[1,4'-bipiperidine]-1'-carboxamide (Compound 22)

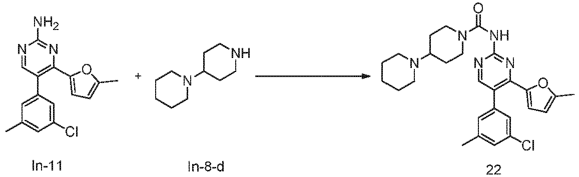

1,4'-Dipiperidine (In-8-d) was used to replace the solution of methylamine in tetrahydrofuran in Example 5, 5-(3-chloro-5-methylphenyl)-4-(5-methylfuran-2-yl)pyrimidin-2-amine (In-11) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (22) (15 mg, yield: 7.9*%*).
MS m/z (ESI): 494.2 [M+H]⁺.
¹H NMR (400 MHz, DMSO-*d*₆) δ: 9.60 (s, 1H), 8.35 (s, 1H), 7.35 (s, 1H), 7.21 (s, 1H), 7.13 (s, 1H), 6.32 (d, *J* = 3.6 Hz, 1H), 6.19 (dd, *J* = 3.2, 0.8 Hz, 1H), 4.13 (d, *J* = 12.8 Hz, 2H), 2.81 (t, *J* = 12.0 Hz, 2H), 2.65-2.54 (m, 5H), 2.34 (s, 3H), 2.20 (s, 3H), 1.76 (d, *J* = 11.6 Hz, 2H), 1.58-1.45 (m, 4H), 1.44-1.35 (m, 4H).

### Example 23: Preparation of 3-(5-(4-fluorophenyl)-6-(4-methylquinazolin-6-yl)-1,2,4-triazin-3-yl)-1,1-dimethylurea (Compound 23)

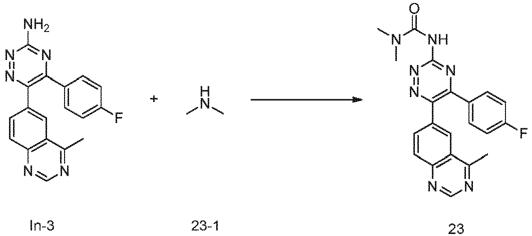

5-(4-Fluorophenyl)-6-(4-methylquinazolin-6-yl)-1,2,4-triazine-3-amine (In-3) (100.0 mg, 0.3 mmol) was dissolved in tetrahydrofuran (4 mL), DIPEA (0.6 mL, 3.2 mmol) and triphosgene (89.0 mg, 0.3 mmol) were added at 0°C, and stirred at 0°C for 0.5 hours, then a solution of dimethylamine (23-1) in tetrahydrofuran (0.3 mL, 2 mol/L, 0.6 mmol) was added, and reacted at 0°C for 1 hour. After the reaction was complete, water was added to the reaction solution, the mixture was extracted with ethyl acetate. The organic phases were combined, dried over anhydrous sodium sulfate, and filtered. The filtrate was concentrated, and the residue was purified by silica gel column chromatography (eluent: methanol/dichloromethane=1/10 (v/v)) to obtain the title compound (23) (50 mg, yield: 42%).
MS m/z (ESI): 404.2 [M+H]⁺.
¹H NMR (400 MHz, MeOD) δ: 9.10 (s, 1H), 8.49 (s, 1H), 8.03-7.95 (m, 2H), 7.66-7.61 (m, 2H), 7.10 (t, *J* = 8.8 Hz, 2H), 3.15 (s, 6H), 2.88 (s, 3H).

### Example 24: Preparation of 3-(5-(4-fluorophenyl)-6-(4-methylquinazolin-6-yl)-1,2,4-triazolin-3-yl)-1-methyl-1-(pyridine-2-methyl)urea (Compound 24)

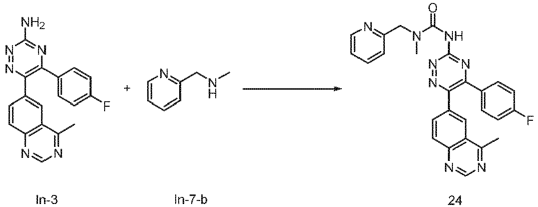

*N*-methyl-1-(pyridin-2-yl)methylamine (In-7-b) was used to replace the solution of dimethylamine in tetrahydrofuran in Example 23, and a method similar to that of Example 23 was adopted to synthesize and obtain the title compound (24) (40 mg, yield: 28*%*).
MS m/z (ESI): 480.2 [M+H]⁺.
¹H NMR (400 MHz, MeOD) δ: 9.10 (s, 1H), 8.61 (s, 1H), 8.49 (s, 1H), 8.04-7.95 (m, 2H), 7.87 (t, *J* = 8.0 Hz, 1H), 7.66-7.63 (m, 2H), 7.52 (t, *J* = 8.4 Hz, 1H), 7.39 (t, *J* = 8.0 Hz, 1H), 7.10 (t, *J* = 8.0 Hz, 2H), 4.79 (s, 2H), 3.15 (s, 3H), 2.88 (s, 3H).

### Example 25: Preparation of 1-methyl-3-(5-(5-methylfuran-2-yl)-6-(4-methylquinazolin-6-yl)-1,2,4-triazin-3-yl)-1-(pyridin-2-yl-methyl)urea (Compound 25)

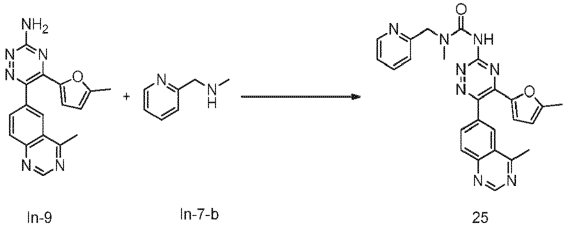

5-(5-Methylfuran-2-yl)-6-(4-methylquinazolin-6-yl)-1,2,4-triazine-3-amine (In-9) was used to replace 5-(4-fluorophenyl)-6-(4-methylquinazolin-6-yl)-1,2,4-triazine-3-amine (In-3) in Example 23, *N*-methyl-1-(pyridin-2-yl)methylamine (In-7-b) was used to replace the solution of dimethylamine in tetrahydrofuran in Example 23, and a method similar to that of Example 23 was adopted to synthesize and obtain the title compound (25) (40 mg, yield: 28%).
MS m/z (ESI): 467.2 [M+H]⁺.
¹H NMR (400 MHz, MeOD) δ: 9.15 (s, 1H), 8.61-8.59 (m, 2H), 8.17-8.09 (m, 2H), 7.90-7.86 (m, 1H), 7.53 (d, *J* = 7.6 Hz, 1H), 7.39 (dd, *J* = 7.2, 5.2 Hz, 1H), 7.05 (d, *J* = 3.6 Hz, 1H), 6.22 (dd, *J* = 3.6, 0.8 Hz, 1H), 4.78 (s, 2H), 3.15 (s, 3H), 3.00 (s, 3H), 2.10 (s, 3H).

### Example 26: Preparation of N-(6-(2-chloro-6-methylpyridin-4-yl)-5-(5-methylfuran-2-yl)-1,2,4-triazin-3-yl)-[1,4'-bipiperidine]-1'-carboxamide (Compound 26)

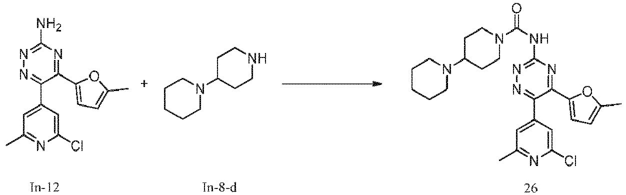

6-(2-Chloro-6-methylpyridin-4-yl)-5-(5-methylfuran-2-yl)-1,2,4-triazine-3-amine (In-12) was used to replace 5-(4-fluorophenyl)-6-(4-methylquinazolin-6-yl)-1,2,4-triazine-3-amine (In-3) in Example 23, 1,4'-dipiperidine (In-8-d) was used to replace the solution of dimethylamine in tetrahydrofuran in Example 23, and a method similar to that of Example 23 was adopted to synthesize and obtain the title compound (26) (100 mg, yield: 30%).
MS m/z (ESI): 496.2 [M+H]⁺.
¹H NMR (400 MHz, MeOD) δ: 8.50 (s, 1H), 7.47 (d, *J* = 9.6 Hz, 2H), 7.20 (d, *J* = 3.6 Hz, 1H), 6.29 (d, *J* = 3.6 Hz, 1H), 4.42 (d, *J* = 13.2 Hz, 2H), 3.36-3.20 (m, 5H), 3.05 (t, *J* = 12.8 Hz, 2H), 2.58 (s, 3H), 2.21 (s, 3H), 2.15 (d, *J* = 12.8 Hz, 2H), 1.86-1.66 (m, 8H).

### Example 27: Preparation of N-(5-(5-methylfuran-2-yl)-6-(4-methylquinazolin-6-yl)-1,2,4-triazin-3-yl)-[1,4'-bipiperidine]-1'-carboxamide (Compound 27)

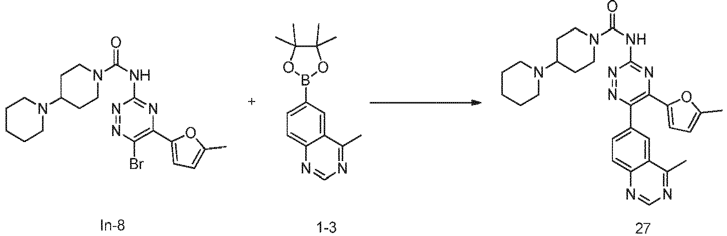

*N*-(6-Bromo-5-(5-methylfuran-2-yl)-1,2,4-triazin-3-yl)-[1,4'-bipiperidine]-1'-carboxamide (In-8) (30 mg, 0.07 mmol), 4-methyl-6-(4,4,5,5-tetramethyl-1,3,2-dioxaborolan-2-yl)quinazoline (1-3) (27.05 mg, 0.10 mmol) and cesium carbonate (43.53 mg, 0.13 mmol) were added to 1,4-dioxane (5 mL), the atmosphere of which was replaced with nitrogen gas for three times, then tetrakis(triphenylphosphine)palladium (3.86 mg, 3.34 µmol) was added, the obtained mixture was reacted at 80°C for 12 hours. After the reaction was complete, the reaction solution was cooled to room temperature, and filtered, and the filtrate was concentrated. The residue was purified by silica gel column chromatography (eluent: methanol/dichloromethane =1/15 (v/v)) to obtain the title compound (27) (10.0 mg, yield: 29%).
MS m/z (ESI): 513.3 [M+H]⁺.
¹H NMR (400 MHz, DMSO-*d*₆) δ: 10.16 (s, 1H), 9.20 (s, 1H), 8.54 (d, *J* = 1.2 Hz, 1H), 8.17-8.05 (m, 2H), 6.48 (d, *J* = 3.6 Hz, 1H), 6.24 (d, *J* = 3.2 Hz, 1H), 4.17 (d, *J* = 12.8 Hz, 2H), 2.98-2.80 (m, 5H), 2.46-2.43 (m, 5H), 2.19 (s, 3H), 1.75 (d, *J* = 11.2 Hz, 2H), 1.56-1.29 (m, 8H).

### Example 28: N-(6-(3-chloro-5-methylphenyl)-5-(5-methylfuran-2-yl)-1,2,4-triazin-3-yl)-[1,4'-bipiperidine]-1'-carboxamide (Compound 28)

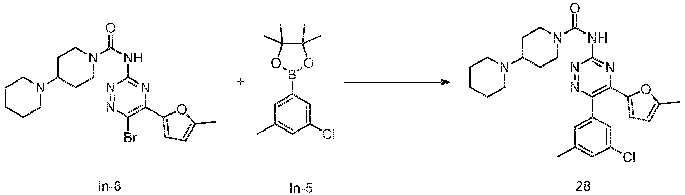

2-(3-Chloro-5-methylphenyl)-4,4,5,5-tetramethyl-1,3,2-dioxaborolane (In-5) was used to replace 4-methyl-6-(4,4,5,5-tetramethyl-1,3,2-dioxaborolan-2-yl)quinazoline (1-3) in Example 27, and a method similar to that of Example 27 was adopted to synthesize and obtain the title compound (28) (9 mg, yield: 11.8%).
MS m/z (ESI): 495.2 [M+H]⁺.
¹H NMR (400 MHz, DMSO-*d*₆) δ: 10.10 (s, 1H), 7.46-7.34 (m, 3H), 6.41 (d, *J* = 3.6 Hz, 1H), 6.29-6.28 (m, 1H), 4.15 (d, *J* = 13.2 Hz, 2H), 2.88-2.82 (m, 2H), 2.47-2.45 (m, 5H), 2.41 (s, 3H), 2.27 (s, 3H), 1.75-1.72 (m, 2H), 1.48-1.38 (m, 8H).

### Example 29: Preparation of 3-(6-(4-fluorophenyl)-5-(4-methylquinazolin-6-yl)pyridin-2-yl)-1-methyl-1-(pyridine-2-methyl)urea (Compound 29)

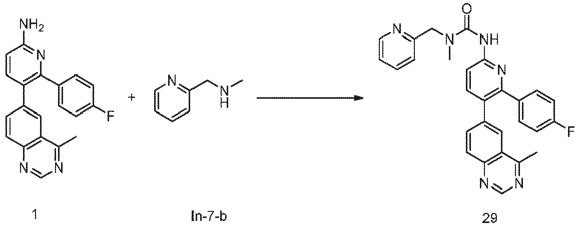

6-(4-Fluorophenyl)-5-(4-methylquinazolin-6-yl)pyridin-2-amine (1) was used to replace 3-fluoro-6-(4-fluorophenyl)-5-(4-methylquinazolin-6-yl)pyridin-2-amine (2) in Example 4, and a method similar to that of Example 4 was adopted to synthesize and obtain the title compound (29) (45 mg, yield: 31*%*).
MS m/z (ESI): 479.2 [M+H]⁺.
¹H NMR (400 MHz, MeOD) δ: 9.03 (s, 1H), 8.56 (d, *J* = 4.4 Hz, 1H), 8.12 (s, 1H), 8.06-7.95 (m, 2H), 7.87-7.82 (m, 2H), 7.70 (d, *J* = 8.4 Hz, 1H), 7.45 (d, *J* = 8.0 Hz, 1H), 7.38-7.33 (m, 3H), 6.99 (t, *J* = 8.0 Hz, 2H), 4.74 (s, 2H), 3.14 (s, 3H), 2.85 (s, 3H).

### Example 30: Preparation of N-(4-(4-fluorophenyl)-5-(4-methylquinazolin-6-yl)pyrimidin-2-yl)-4-methylpiperazine-1-carboxamide (Compound 30)

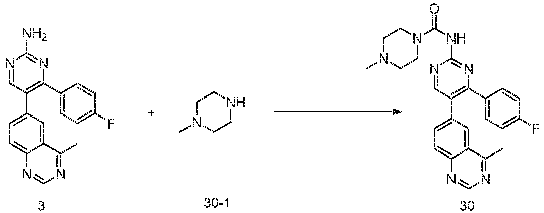

N-methylpiperazine (30-1) was used to replace the solution of methylamine in tetrahydrofuran in Example 5, and a method similar to that of Example 5 was adopted to synthesize and obtain the title compound (30) (61 mg, yield: 21%).
MS m/z (ESI): 462.2 [M+H]⁺.
¹H NMR (400 MHz, DMSO-*d*₆) δ: 9.81 (s, 1H), 9.12 (s, 1H), 8.78 (s, 1H), 8.30 (d, *J* = 1.6 Hz, 1H), 7.85 (d, *J* = 8.8 Hz, 1H), 7.60 (dd, *J* = 8.8, 2.0 Hz, 1H), 7.52-7.38 (m, 2H), 7.16 (t, *J* = 8.8 Hz, 2H), 3.58-3.39 (m, 4H), 2.88 (s, 3H), 2.41-2.27 (m, 4H), 2.20 (s, 3H).

### Example 31: Preparation of 3-(4-(4-fluorophenyl)-5-(4-methylquinazolin-6-yl)pyrimidin-2-yl)-1-(2-methoxyethyl)-1-methylurea (Compound 31)

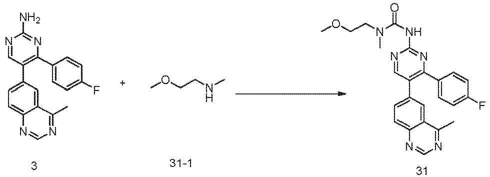

2-Methoxy-*N*-methyl-ethylamine (31-1) was used to replace the solution of methylamine in tetrahydrofuran in Example 5, and a method similar to that of Example 5 was adopted to synthesize and obtain the title compound (31) (63 mg, yield: 22%).
MS m/z (ESI): 447.2 [M+H]⁺.
¹H NMR (400 MHz, DMSO-*d*₆) δ: 9.48 (s, 1H), 9.12 (s, 1H), 8.78 (s, 1H), 8.30 (d, *J* = 1.6 Hz, 1H), 7.85 (d, *J* = 8.8 Hz, 1H), 7.61 (dd, *J* = 8.8, 2.0 Hz, 1H), 7.52-7.38 (m, 2H), 7.24-7.09 (m, 2H), 3.54 (s, 4H), 3.32 (s, 3H), 3.00 (s, 3H), 2.88 (s, 3H).

### Example 32: Preparation of N-(3-fluoro-6-(4-fluorophenyl)-5-(4-methylquinazolin-6-yl)pyridin-2-yl) morpholine-4-carboxamide (Compound 32)

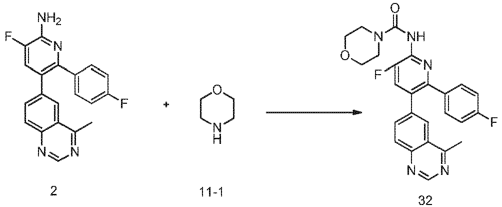

Morpholine (11-1) was used to replace *N*-methyl-1-pyridine-2-methylamine (In-7-b) in Example 4, and a method similar to that of Example 4 was adopted to synthesize and obtain the title compound (32) (625 mg, yield: 64*%*).
MS m/z (ESI): 462.2 [M+H]⁺.
¹H NMR (400 MHz, DMSO-*d*₆) δ: 9.41 (s, 1H), 9.10 (s, 1H), 8.26 (d, *J* = 1.6 Hz, 1H), 8.05 (d, *J* = 10.4 Hz, 1H), 7.82 (d, *J* = 8.8 Hz, 1H), 7.64-7.61 (m, 1H), 7.35-7.31 (m, 2H), 7.10 (t, *J* = 8.8 Hz, 2H), 3.63 (t, *J* = 4.6 Hz, 4H), 3.49 (t, *J* = 4.4 Hz, 4H), 2.85 (s, 3H).

### Example 33: Preparation of N-(5-(2-chloro-6-methylpyridin-4-yl)-4-(4-fluorophenyl)pyrimidin-2-yl)-4-methylpiperazine-1-carboxamide (Compound 33)

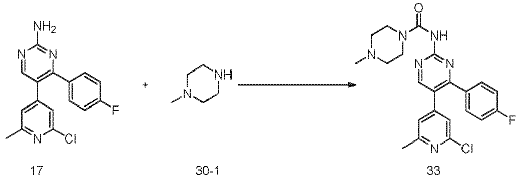

*N*-methyl piperazine (30-1) was used to replace the solution of methylamine in tetrahydrofuran in Example 5, 5-(2-chloro-6-methylpyridin-4-yl)-4-(4-fluorophenyl)pyrimidine-2-amine (17) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (33) (73 mg, yield: 25*%*).
MS m/z (ESI): 441.2 [M+H]⁺.
¹H NMR (400 MHz, DMSO-*d*₆) δ: 9.87 (s, 1H), 8.63 (s, 1H), 7.49-7.37 (m, 2H), 7.31-7.19 (m, 2H), 7.15 (s, 2H), 3.54-3.38 (m, 4H), 2.39 (s, 3H), 2.32 (dd, *J* = 13.6, 8.8 Hz, 4H), 2.24-2.12 (m, 3H).

### Example 34: Preparation of (S)-N-(4-(4-fluorophenyl)-5-(4-methylquinazolin-6-yl)pyrimidin-2-yl)-2-methylmorpholine-4-carboxamide (Compound 34)

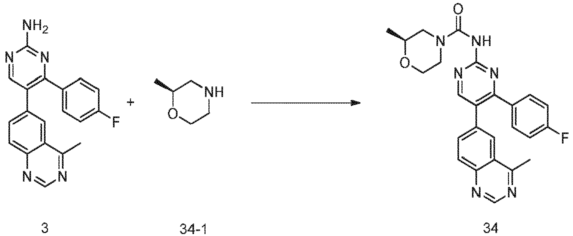

(S)-2-methylmorpholine (34-1) was used to replace the solution of methylamine in tetrahydrofuran in Example 5, and a method similar to that of Example 5 was adopted to synthesize and obtain the title compound (34) (52 mg, yield: 34%).
MS m/z (ESI): 459.2 [M+H]⁺.
¹H NMR (400 MHz, MeOD) δ: 9.07 (s, 1H), 8.73 (s, 1H), 8.27 (d, *J* = 1.6 Hz, 1H), 7.89 (d, *J* = 8.8 Hz, 1H), 7.69-7.67 (m, 1H), 7.51-7.48 (m, 2H), 7.06-6.99 (m, 2H), 4.10-4.02 (m, 2H), 3.94-3.90 (m, 1H), 3.66-3.60 (m, 2H), 3.15-3.08 (m, 1H), 2.92 (s, 3H), 2.80-2.74 (m, 1H), 1.23-1.16 (m, 3H).

### Example 35: Preparation of N-(5-(2-chloro-6-methylpyridin-4-yl)-4-(4-fluorophenyl)pyrimidin-2-yl) piperidine-1-carboxamide (Compound 35)

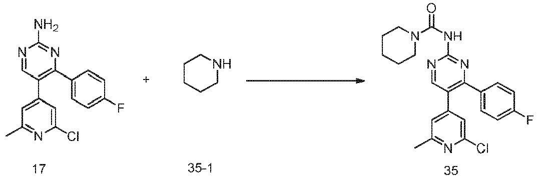

Piperidine (35-1) was used to replace the solution of methylamine in tetrahydrofuran in Example 5, 5-(2-chloro-6-methylpyridin-4-yl)-4-(4-fluorophenyl)pyrimidin-2-amine (17) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (35 ) (40 mg, yield: 21*%*).
MS m/z (ESI): 426.1 [M+H]⁺.
¹H NMR (400 MHz, MeOD) δ: 8.55 (s, 1H), 7.51-7.47 (m, 2H), 7.18-7.05 (m, 4H), 3.57 (d, *J* = 5.2 Hz, 4H), 2.43 (s, 3H), 1.78-1.56 (m, 6H).

### Example 36: Preparation of (S)-N-(5-(2-chloro-6-methylpyridin-4-yl)-4-(4-fluorophenyl)pyrimidin-2-yl)-2-methylmorpholine-4-carboxamide (Compound 36)

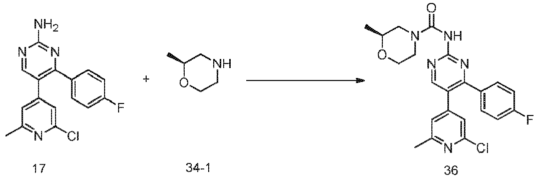

(S)-2-methylmorpholine (34-1) was used to replace the solution of methylamine in tetrahydrofuran in Example 5, 5-(2-chloro-6-methylpyridin-4-yl)-4-(4-fluorophenyl)pyrimidin-2-amine (17) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (36) (40 mg, yield: 21%).
MS m/z (ESI): 442.1 [M+H]⁺.
¹H NMR (400 MHz, MeOD) δ: 8.57 (s, 1H), 7.54-7.45 (m, 2H), 7.16-7.06 (m, 4H), 4.08-4.03 (m, 2H), 3.92-3.88 (m, 1H), 3.64-3.58 (m, 2H), 3.15-3.05 (m, 1H), 2.78-2.76 (m, 1H), 2.42 (d, *J* = 14.0 Hz, 3H), 1.18 (d, *J* = 6.0 Hz, 3H).

### Example 37: Preparation of (2R,6S)-N-(5-(2-chloro-6-methylpyridin-4-yl)-4-(4-fluorophenyl) pyrimidin-2-yl)-2,6-dimethylmorpholine-4-carboxamide (Compound 37)

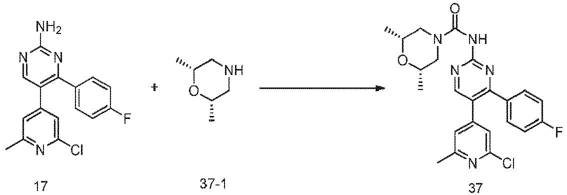

(2*R*,6*S*)-2,6-dimethylmorpholine (37-1) was used to replace the solution of methylamine in tetrahydrofuran in Example 5, 5-(2-chloro-6-methylpyridin-4-yl)-4-(4-fluorophenyl)pyrimidin-2-amine (17) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (37) (40 mg, yield: 14%).
MS m/z (ESI): 456.2 [M+H]⁺.
¹H NMR (400 MHz, DMSO-*d*₆) δ: 9.90 (s, 1H), 8.64 (s, 1H), 7.45-7.42 (m, 2H), 7.24 (t, *J* = 8.8 Hz, 2H), 7.15 (s, 2H), 3.99 (d, *J* = 12.8 Hz, 2H), 3.55-3.50 (m, 2H), 2.55-2.51 (m, 2H), 2.39 (s, 3H), 1.09 (d, *J* = 6.0 Hz, 6H).

### Example 38: Preparation of (R)-N-(5-(2-chloro-6-methylpyridin-4-yl)-4-(4-fluorophenyl)pyrimidin-2-yl)-2-methylmorpholine-4-carboxamide (Compound 38)

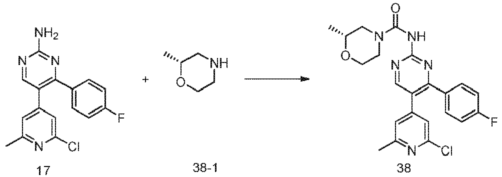

(*R*)-2-methylmorpholine (38-1) was used to replace the solution of methylamine in tetrahydrofuran in Example 5, 5-(2-chloro-6-methylpyridin-4-yl)-4-(4-fluorophenyl)pyrimidin-2-amine (17) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (38) (100 mg, yield: 35%).
MS m/z (ESI): 442.1 [M+H]⁺.
¹H NMR (400 MHz, DMSO-*d*₆) δ: 9.92 (s, 1H), 8.64 (s, 1H), 7.45-7.42 (m, 2H), 7.27-7.22 (m, 2H), 7.15 (s, 2H), 4.00-3.80 (m, 3H), 3.49-3.33 (m, 2H), 2.94 (t, *J* = 9.6 Hz, 1H), 2.64-2.58 (m, 1H), 2.39 (s, 3H), 1.09 (d, *J* = 6.0 Hz, 3H).

### Example 39: Preparation of (2R,6S)-N-(4-(4-fluorophenyl)-5-(4-methylquinazolin-6-yl)pyrimidin-2-yl)-2,6-dimethylmorpholine-4-carboxamide (Compound 39)

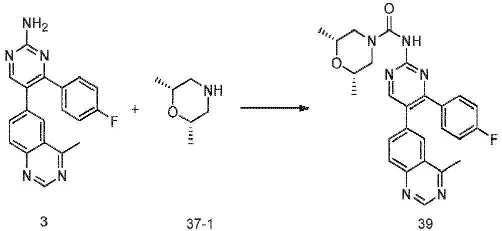

(2*R*,6*S*)-2,6-dimethylmorpholine (37-1) was used to replace the solution of methylamine in tetrahydrofuran in Example 5, and a method similar to that of Example 5 was adopted to synthesize and obtain the title compound (39) (55 mg, yield: 19*%*).
MS m/z (ESI): 473.2 [M+H]⁺.
¹H NMR (400 MHz, DMSO-*d*₆) δ: 9.84 (s, 1H), 9.12 (s, 1H), 8.79 (s, 1H), 8.31 (d, *J* = 1.6 Hz, 1H), 7.85 (d, *J* = 8.8 Hz, 1H), 7.60 (dd, *J* = 8.8, 1.6 Hz, 1H), 7.45-7.43 (m, 2H), 7.16 (dd, *J* = 12.4, 5.6 Hz, 2H), 4.02 (d, *J* = 12.8 Hz, 2H), 3.57-3.52 (m, 2H), 2.88 (s, 3H), 2.57-2.51 (m, 2H), 1.11 (d, *J* = 6.4 Hz, 6H).

### Example 40: Preparation of (R)-N-(4-(4-fluorophenyl)-5-(4-methylquinazolin-6-yl)pyrimidin-2-yl)-2-methylmorpholine-4-carboxamide (Compound 40)

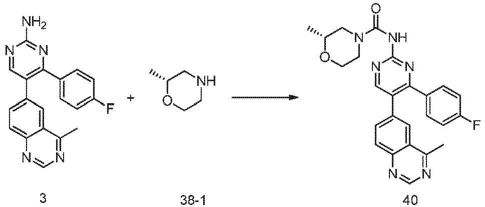

(*R*)-2-methylmorpholine (38-1) was used to replace the solution of methylamine in tetrahydrofuran in Example 5, and a method similar to that of Example 5 was adopted to synthesize and obtain the title compound (40) (55 mg, yield: 19*%*).
MS m/z (ESI): 459.2 [M+H]⁺.
¹H NMR (400 MHz, DMSO-*d*₆) δ: 9.85 (s, 1H), 9.12 (s, 1H), 8.79 (s, 1H), 8.31 (d, *J* = 1.6 Hz, 1H), 7.85 (d, *J* = 8.8 Hz, 1H), 7.61 (dd, *J* = 8.4, 3.2 Hz, 1H), 7.46-7.43 (m, 2H), 7.16 (dd, *J* = 12.4, 5.6 Hz, 2H), 4.02-3.81 (m, 3H), 3.50-3.44 (m, 2H), 2.99-2.93 (m, 1H), 2.88 (s, 3H), 2.66-2.60 (m, 1H), 1.11 (d, *J* = 6.4 Hz, 3H).

### Example 41: Preparation of N-(6-(4-fluorophenyl)-5-(4-methylquinazolin-6-yl)pyridin-2-yl)morpholine-4-carboxamide (Compound 41)

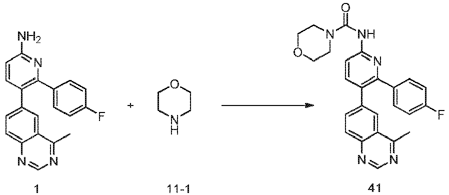

6-(4-Fluorophenyl)-5-(4-methylquinazolin-6-yl)pyridin-2-amine (1) was used to replace 5-(4-fluorophenyl)-6-(4-methylquinazolin-6-yl)-1,2,4-triazin-3-amine (In-3) in Example 23, morpholine (11-1) was used to replace the solution of dimethylamine in tetrahydrofuran in Example 23, and a method similar to that of Example 23 was adopted to synthesize and obtain the title compound (41) (95 mg, yield: 35%).
MS m/z (ESI): 462.2 [M+H]⁺.
¹H NMR (400 MHz, DMSO-*d*₆) δ: 9.46 (s, 1H), 9.09 (s, 1H), 8.15 (d, *J* = 1.8 Hz, 1H), 7.96 (dd, *J* = 26.0, 8.6 Hz, 2H), 7.81 (d, *J* = 8.8 Hz, 1H), 7.60 (dd, *J* = 8.8, 2.0 Hz, 1H), 7.41-7.32 (m, 2H), 7.11 (dd, *J* = 12.4, 5.6 Hz, 2H), 3.68-3.59 (m, 4H), 3.54-3.47 (m, 4H), 2.83 (s, 3H).

### Example 42: Preparation of N-(4-chloro-6-(4-fluorophenyl)-5-(4-methylquinazolin-6-yl)pyridin-2-yl) morpholine-4-carboxamide (Compound 42)

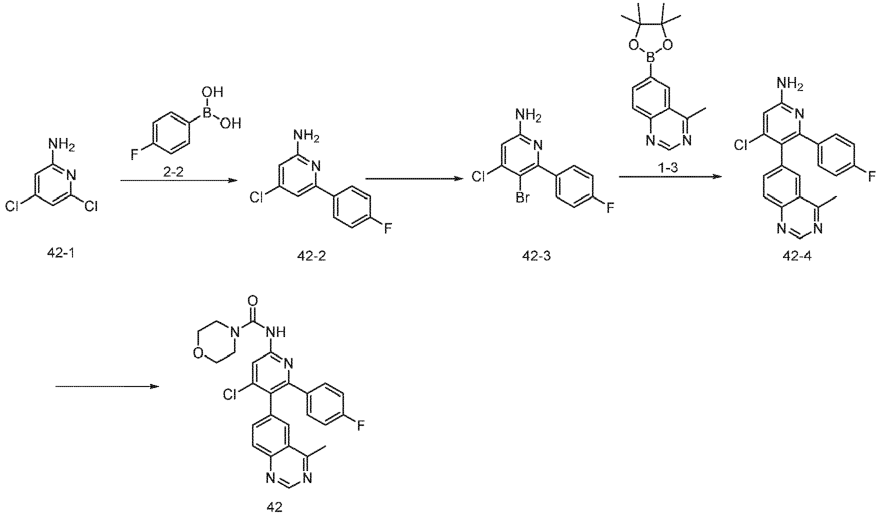

### Step 1: Preparation of 4-chloro-6-(4-fluorophenyl)pyridin-2-amine (42-2)

4,6-Dichloropyridin-2-amine (42-1) was used to replace 6-chloro-3-fluoropyridin-2-amine (2-1) in Step 1 of Example 2, and a method similar to that of Step 1 of Example 2 was adopted to synthesize and obtain the title compound (42-2) (0.5 g, yield: 45%).
MS m/z (ESI): 223.1 [M+H]⁺.

### Step 2: Preparation of 5-bromo-4-chloro-6-(4-fluorophenyl)pyridin-2-amine (42-3)

4-Chloro-6-(4-fluorophenyl)pyridin-2-amine (42-2) was used to replace 3-fluoro-6-(4-fluorophenyl)pyridine-2-amine (2-3) in Step 2 of Example 2, and a method similar to that of Step 2 of Example 2 was adopted to synthesize and obtain the title compound (42-3) (0.3 g, yield: 60%).
MS m/z (ESI): 300.8 [M+H]⁺.

### Step 3: Preparation of 4-chloro-6-(4-fluorophenyl)-5-(4-methylquinazolin-6-yl)pyridin-2-amine (42-4)

4-Chloro-6-(4-fluorophenyl)-5-(4-methylquinazolin-6-yl)pyridin-2-amine (42-4) was used to replace 5-bromo-3-fluoro-6-(4-fluorophenyl)pyridin-2-amine (2-4) in Step 3 of Example 2, and a method similar to that of Step 3 of Example 2 was adopted to synthesize and obtain the title compound (42-3) (0.3 g, yield: 60*%*).
MS m/z (ESI): 365.1 [M+H]⁺.

### Step 4: Preparation of N-(4-chloro-6-(4-fluorophenyl)-5-(4-methylquinazolin-6-yl)pyridin-2-yl) morpholine-4-carboxamide (42)

Morpholine (11-1) was used to replace the solution of methylamine in tetrahydrofuran in Example 5, 4-chloro-6-(4-fluorophenyl)-5-(4-methylquinazolin-6-yl)pyridin-2-amine (42-4) was used to place 4-(4-fluorophenyl)-5-(4-methylquinazolin-6-yl)pyrimidin-2-amine (3) in Example 5, and a method similar to that of Example 5 was adopted to synthesize and obtain the title compound (42) (30 mg, yield: 34%).
MS m/z (ESI): 478.1 [M+H]⁺.
¹H NMR (400 MHz, DMSO-*d*₆) δ: 9.72 (s, 1H), 9.10 (s, 1H), 8.13 (d, *J* = 1.6 Hz, 1H), 8.09 (s, 1H), 7.90 (d, *J* = 8.8 Hz, 1H), 7.78 (dd, *J* = 8.8, 1.6 Hz, 1H), 7.34-7.27 (m, 2H), 7.03-6.98 (m, 2H), 3.65-3.57 (m, 4H), 3.55-3.48 (m, 4H), 2.78 (s, 3H).

### Example 43: Preparation of N-(4-cyano-6-(4-fluorophenyl)-5-(4-methylquinazolin-6-yl)pyridin-2-yl) morpholine-4-carboxamide (Compound 43)

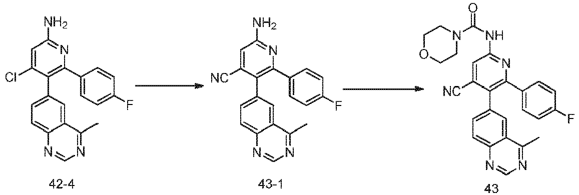

### Step 1: Preparation of 6-amino-2-(4-fluorophenyl)-3-(4-methylquinazolin-6-yl)isonicotinonitrile (43-1)

4-Chloro-6-(4-fluorophenyl)-5-(4-methylquinazolin-6-yl)pyridin-2-amine (42-4) (170 mg, 0.47 mmol), zinc powder (3.0 mg, 0.47 mmol), zinc cyanide (55 mg, 0.47 mmol) and DMF (2 mL) were added sequentially into a 10ml microwave tube. Nitrogen gas was blown into the reaction mixture for 5 minutes, tris(dibenzylideneacetone) dipalladium (46 mg, 0.05 mmol) and dppf (56 mg, 0.1 mmol) were added to the reaction mixture, then the reaction mixture was heated to 140°C with microwaves to perform reaction for 5 hours. The reaction mixture was cooled to room temperature, poured into 20 mL of water, stirred for 5 minutes, and extracted three times with ethyl acetate. The organic phase was washed with saturated brine for three times, dried over anhydrous sodium sulfate, and filtered. The filtrate was concentrated, and the residue was purified by silica gel column chromatography (eluent: ethyl acetate/petroleum ether = 1/1 (v/v)) to obtain the title compound (43-1) (160 mg, yield: 96*%*).
MS m/z (ESI): 356.1 [M+H]⁺.

### Step 2: Preparation of N-(4-cyano-6-(4-fluorophenyl)-5-(4-methylquinazolin-6-yl)pyridin-2-yl) morpholine-4-carboxamide (43)

Morpholine (11-1) was used to replace the solution of methylamine in tetrahydrofuran in Example 5, 6-amino-2-(4-fluorophenyl)-3-(4-methylquinazolin-6-yl)isonicotinonitrile (43-1) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (43) (35 mg, yield: 18%).
MS m/z (ESI): 469.2 [M+H]⁺.
¹H NMR (400 MHz, DMSO-*d*₆) δ: 9.96 (s, 1H), 9.15 (s, 1H), 8.38 (d, *J* = 1.6 Hz, 1H), 8.27 (s, 1H), 7.92 (d, *J* = 8.8 Hz, 1H), 7.76 (dd, *J* = 8.8, 2.0 Hz, 1H), 7.35-7.32 (m, 2H), 7.10-7.05 (m, 2H), 3.64-3.62 (m, 4H), 3.54-3.52 (m, 4H), 2.83 (s, 3H).

### Example 44: Synthesis of N-(4-cyclopropyl-6-(4-fluorophenyl)-5-(4-methylquinazolin-6-yl) pyrimidin-2-yl)morpholine-4-carboxamide (44)

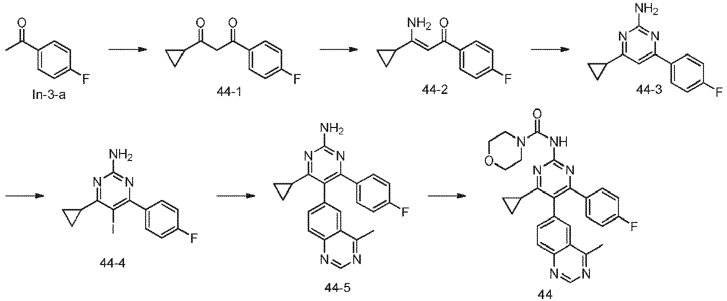

### Step 1: Preparation of 1-cyclopropyl-3-(4-fluorophenyl)propane-1,3-dione (44-1)

4-Fluoroacetophenone (In-3-a) (3 g, 21.7 mmol) and tetrahydrofuran (60 mL) was added sequentially into a 100 mL single-necked flask, the atmosphere of which was replaced with nitrogen for three times. The mixture was cooled to 0°C, then sodium hydride (2.37 g, 59.3 mmol, 60*%*) was added, and stirred for 1 hour, and then methyl cyclopropane carboxylate (4.35 g, 43.5 mmol) was added, and the mixture was reacted at 25°C for 16 hours. Saturated aqueous solution of ammonium chloride was added to the reaction solution to quench the reaction, then the reaction solution was extracted three times with ethyl acetate. The organic phase was washed with saturated brine, dried over anhydrous sodium sulfate, and filtered. The filtrate was concentrated, and the residue was purified by silica gel column chromatography (eluent: ethyl acetate/petroleum ether = 1/10 (v/v)) to obtain the title compound (44-1) of this step (2.8 g, yield: 63%).
MS m/z (ESI): 207.1 [M+H]⁺.

### Step 2: Preparation of (Z)-3-amino-3-cyclopropyl-1-(4-fluorophenyl)prop-2-en-1-one (44-2)

1-Cyclopropyl-3-(4-fluorophenyl)propane-1,3-dione (44-1) (2.0 g, 9.7 mmol), ammonium acetate (7.47 g, 97.1 mmol) and anhydrous methanol (25 mL) were added sequentially into a 100 mL single-necked flask, reacted at 65°C for 16 hours. The reaction mixture was cooled to room temperature, and concentrated, water and ethyl acetate were added to the resulting residue. The aqueous phase was extracted three times with ethyl acetate. The resulting organic phases were combined, washed twice with saturated brine, dried over anhydrous sodium sulfate, and filtered, and the filtrate was concentrated to obtain the title compound (44-2) of this step (1.8 g, yield: 90*%*).
MS m/z (ESI): 206.1 [M+H]⁺.

### Step 3: Preparation of 4-cyclopropyl-6-(4-fluorophenyl)pyrimidin-2-amine (44-3)

(Z)-3-amino-3-cyclopropyl-1-(4-fluorophenyl)prop-2-en-1-one (44-2) (1.8 g, 8.8 mmol) and 50*%* aqueous solution of cyanamide (20 mL) were added into a 50 mL round bottom flask, reacted at 120°C for 2 hours. The reaction solution was cooled to room temperature, and concentrated, and the residue was purified by silica gel column chromatography (eluent: ethyl acetate/petroleum ether=1/ 3 (v/v)) to obtain the title compound (44-3) of this step (1.4 g, yield: 70*%*).
MS m/z (ESI): 230.1 [M+H]⁺.

### Step 4: Preparation of 4-cyclopropyl-6-(4-fluorophenyl)-5-iodopyrimidin-2-amine (44-4)

4-Cyclopropyl-6-(4-fluorophenyl)pyrimidin-2-amine (44-3) (1.2 g, 5.2 mmol) and glacial acetic acid (10 mL) were added into a 100 mL round bottom flask, a solution of iodine chloride in glacial acetic acid (2.2 g, 13.1 mmol, 2 mL) was slowly added dropwise, reacted at 25°C for 16 hours. The reaction solution was poured into water, extracted three times with ethyl acetate. The organic phase was washed three times with saturated aqueous solution of sodium sulfite, washed twice with saturated brine, dried over anhydrous sodium sulfate, and filtered. The filtrate was concentrated, and the residue was purified by silica gel column chromatography (eluent: ethyl acetate/petroleum ether = 1/5 (v/v)) to obtain the title compound (44-4) of this step (1.5 g, yield: 82*%*).
MS m/z (ESI): 356.0 [M+H]⁺.

### Step 5: Preparation of 4-cyclopropyl-6-(4-fluorophenyl)-5-(4-methylquinazolin-6-yl)pyrimidin-2-amine (44-5)

4-Cyclopropyl-6-(4-fluorophenyl)-5-iodopyrimidin-2-amine (44-4) (1.0 g, 2.8 mmol), 4-methyl-6-(4,4,5,5-tetramethyl-1,3,2-dioxaborolan-2-yl)quinazoline (1-3) (0.9 g, 3.4 mmol), potassium carbonate (0.8 g, 5.6 mmol), 1,4-dioxane (10 mL) and water (1 mL) were added sequentially into a 100 mL round bottom flask, the atmosphere of which was replaced with nitrogen gas three times. Pd(dppf)Cl₂ (115 mg, 0.14 mmol) was added to the mixture, and the reaction was carried out at 80°C for 8 hours. The reaction solution was cooled to room temperature, concentrated, and the residue was purified by silica gel column chromatography (eluent: ethyl acetate/petroleum ether = 1/1 (v/v)) to obtain the title compound (44-5) of this step (0.9 g, yield: 85*%*).
MS m/z (ESI): 372.2 [M+H]⁺.

### Step 6: Preparation of N-(4-cyclopropyl-6-(4-fluorophenyl)-5-(4-methylquinazolin-6-yl)pyrimidin-2-yl) morpholine-4-carboxamide (44)

Morpholine (11-1) was used to replace the solution of methylamine in tetrahydrofuran in Example 5, 4-cyclopropyl-6-(4-fluorophenyl)-5-(4-methylquinazolin-6-yl)pyrimidin-2-amine (44-5) was used to place 4-(4-fluorophenyl)-5-(4-methylquinazolin-6-yl)pyrimidin-2-amine (3) in Example 5, and a method similar to that of Example 5 was adopted to synthesize and obtain the title compound (44) (50 mg, yield: 25%).
MS m/z (ESI): 485.2 [M+H]⁺.
¹H NMR (400 MHz, CDCl₃) δ: 9.21 (s, 1H), 8.06 (d, *J* = 8.8 Hz, 1H), 7.89 (s, 1H), 7.75 (dd, *J* = 8.8, 1.6 Hz, 1H), 7.32-7.27 (m, 2H), 6.88 (t, *J* = 8.8 Hz, 2H), 3.77-3.74 (m, 4H), 3.65-3.63 (m, 4H), 2.84 (s, 3H), 1.82-1.79 (m, 1H), 1.41 (bs, 2H), 1.03 (br, 2H).

### Example 45: Preparation of N-(2'-chloro-4-cyano-2-(4-fluorophenyl)-6'-methyl-[3,4'-bipyridyl]-6-yl) morpholine-4-carboxamide (Compound 45)

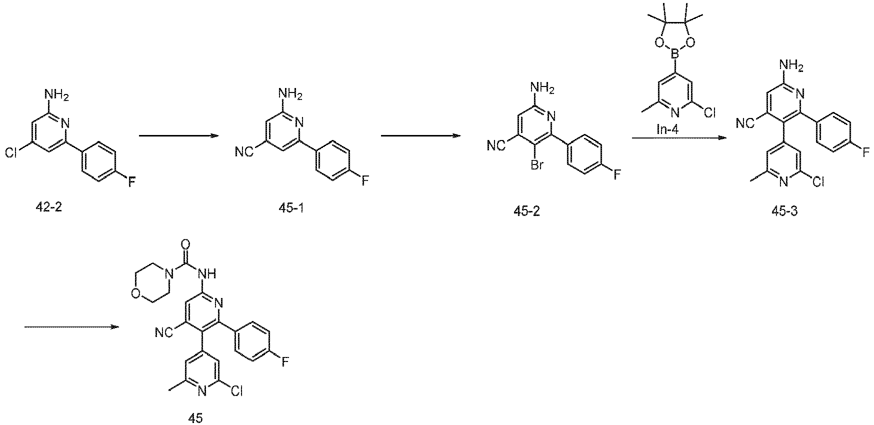

### Step 1: Preparation of 2-amino-6-(4-fluorophenyl)isonicotinonitrile (45-1)

4-Chloro-6-(4-fluorophenyl)pyridine-2-amine (42-2) was used to replace 4-chloro-6-(4-fluorophenyl)-5-(4-methylquinazolin-6-yl)pyridine-2-amine (42-4) in Step 1 of Example 43, and a method similar to that of Step 1 of Example 43 was adopted to synthesize and obtain the title compound (45-1) of this step (1.2 g, yield: 60%). MS m/z (ESI): 214.1 [M+H]⁺.

### Step 2: Preparation of 6-amino-3-bromo-2-(4-fluorophenyl)isonicotinonitrile (45-2)

2-Amino-6-(4-fluorophenyl)isonicotinonitrile (45-1) was used to replace 3-fluoro-6-(4-fluorophenyl)pyridin-2-amine (2-3) in Step 2 of Example 2, and a method similar to that of Step 2 of Example 2 was adopted to synthesize and obtain the title compound (45-2) of this step (0.8 g, yield: 63*%*).
MS m/z (ESI): 292.0 [M+H]⁺.

### Step 3: Preparation of 6-amino-2'-chloro-2-(4-fluorophenyl)-6'-methyl-[3,4'-bipyridine]-4-carbonitrile (45-3)

6-Amino-3-bromo-2-(4-fluorophenyl)isonicotinonitrile (45-2) was used to replace 5-bromo-3-fluoro-6-(4-fluorophenyl)pyridin-2-amine (2-4) in Step 3 of Example 2, 2-chloro-6-methyl-4-(4,4,5,5-tetramethyl-1,3,2-dioxaborolan-2-yl)pyridine (In-4) was used to replace 4-methyl-6-(4,4,5,5-tetramethyl-1,3,2-dioxaborolan-2-yl)quinazoline (1-3) in Step 3 of Example 2, and a method similar to that of Step 3 of Example 2 was adopted to synthesize and obtain the title compound (45-3) of this step (0.4 g, yield: 72*%*).
MS m/z (ESI): 339.1 [M+H]⁺.

### Step 4: Preparation of N-(2'-chloro-4-cyano-2-(4-fluorophenyl)-6'-methyl-[3,4'-bipyridyl]-6-yl) morpholine-4-carboxamide (45)

Morpholine (11-1) was used to replace the solution of methylamine in tetrahydrofuran in Example 5, 6-amino-2'-chloro-2-(4-fluorophenyl)-6'-methyl-[3,4'-bipyridine]-4-nitrile (45-3) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (45) (90 mg, yield: 43.0*%*).
MS m/z (ESI): 452.1 [M+H]⁺.
¹H NMR (400 MHz, DMSO-*d*₆) δ: 10.0 (s, 1H), 8.22 (s, 1H), 7.33 (t, *J* = 7.2 Hz, 3H), 7.23 (s, 1H), 7.17 (t, *J* = 8.8 Hz, 2H), 3.65-3.58 (m, 4H), 3.54-3.48 (m, 4H), 2.40 (s, 3H).

### Example 46: Preparation of N-(4-(4-fluorophenyl)-5-(4-methylquinolin-6-yl)pyrimidin-2-yl) morpholine-4-carboxamide (Compound 46)

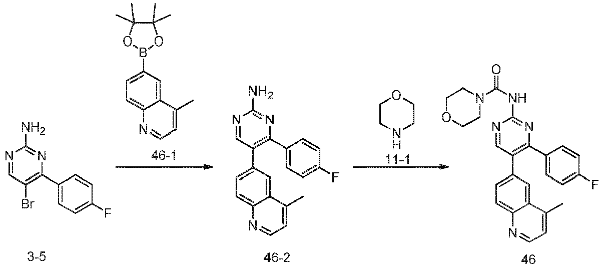

### Step 1: Preparation of 4-(4-fluorophenyl)-5-(4-methylquinolin-6-yl)pyrimidin-3-amine (46-2)

4-Methyl-6-(4,4,5,5-tetramethyl-1,3,2-dioxaborolan-2-yl)quinoline (46-1) was used to replace 4-methyl-6-(4,4,5,5-tetramethyl-1,3,2-dioxaborolan-2-yl)quinazoline (1-3) in Step 4 of Example 3, and a method similar to that of Step 4 of Example 3 was adopted to synthesize and obtain the title compound (46-2) of this step (150 mg, yield: 63*%*).
MS m/z (ESI): 331.1 [M+H]⁺.

### Step 2: Preparation of N-(4-(4-fluorophenyl)-5-(4-methylquinolin-6-yl)pyrimidin-2-yl)morpholine-4-carboxamide (46)

Morpholine (11-1) was used to replace the solution of methylamine in tetrahydrofuran in Example 5, 4-(4-fluorophenyl)-5-(4-methylquinolin-6-yl)pyrimidin-3-amine (46-2) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (46) (30 mg, yield: 18%).
MS m/z (ESI): 444.2 [M+H]⁺.
¹H NMR (400 MHz, DMSO-*d*₆) δ: 9.85 (s, 1H), 8.77 (s, 1H), 8.76 (d, *J* = 8.8 Hz, 1H), 8.70 (s, 1H), 7.90 (s, 1H), 7.46-7.39 (m, 4H), 7.18-7.14 (m, 2H), 3.62-3.60 (m, 4H), 3.49-3.47 (m, 4H), 2.62 (s, 3H).

### Example 47: Preparation of N-(4-cyclopropyl-6-(4-fluorophenyl)-5-(4-methylquinolin-6-yl) pyrimidin-2-yl)morpholine-4-carboxamide (Compound 47)

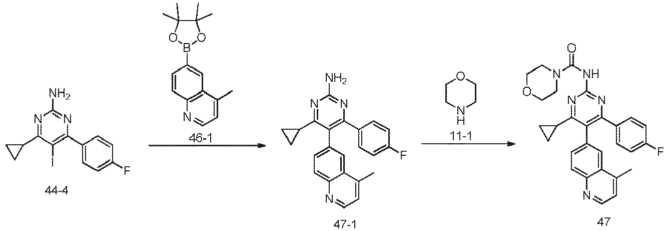

### Step 1: Preparation of 4-cyclopropyl-6-(4-fluorophenyl)-5-(4-methylquinolin-6-yl)pyrimidin-2-amine (47-1)

4-Methyl-6-(4,4,5,5-tetramethyl-1,3,2-dioxaborolan-2-yl)quinoline (46-1) was used to replace 4-methyl-6-(4,4,5,5-tetramethyl-1,3,2-dioxaborolan-2-yl)quinazoline (1-3) in Step 5 of Example 44, and a method similar to that of Step 5 of Example 44 was adopted to synthesize and obtain the title compound (47-1) of this step (260 mg, yield: 63*%*).
MS m/z (ESI): 371.2 [M+H]⁺.

### Step 2: Preparation of N-(4-cyclopropyl-6-(4-fluorophenyl)-5-(4-methylquinolin-6-yl)pyrimidin-2-yl) morpholine-4-carboxamide (48)

Morpholine (11-1) was used to replace the solution of methylamine in tetrahydrofuran in Example 5, 4-cyclopropyl-6-(4-fluorophenyl)-5-(4-methylquinolin-6-yl)pyrimidin-2-amine (47-1) was used to replace 4-(4-fluorophenyl)-5-(4-methylquinazolin-6-yl)pyrimidin-2-amine (3) in Example 5, and a method similar to that of Example 5 was adopted to synthesize and obtain the title compound (47) (45 mg, yield: 50%).
MS m/z (ESI): 484.2 [M+H]⁺.
¹H NMR (400 MHz, DMSO-*d*₆) δ: 9.50 (s, 1H), 8.76 (d, *J* = 8.8 Hz, 1H), 7.97 (s, 2H), 7.59 (d, *J* = 8.8 Hz, 1H), 7.38 (d, *J* = 4.0 Hz, 1H), 7.33-7.30 (m, 2H), 7.05 (t, *J* = 8.8 Hz, 2H), 3.61 (t, *J* = 8.8 Hz, 4H), 3.46 (t, *J* = 8.8 Hz, 4H), 2.56 (s,3H), 1.78 (s, 1H), 1.13 (s, 2H), 0.90 (s, 2H).

### Example 48: Preparation of N-(6-(4-fluorophenyl)-5-(4-methylquinolin-6-yl)pyridin-2-yl)morpholine-4-carboxamide (Compound 48)

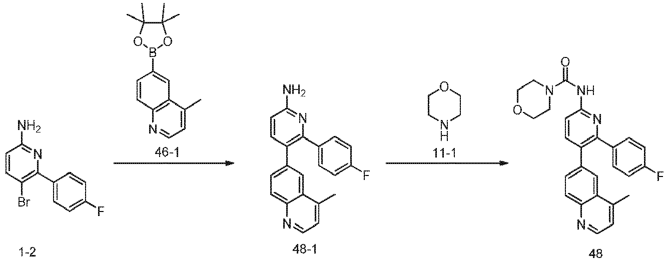

### Step 1: Preparation of 6-(4-fluorophenyl)-5-(4-methylquinolin-6-yl)pyridin-2-amine (48-1)

4-Methyl-6-(4,4,5,5-tetramethyl-1,3,2-dioxaborolan-2-yl)quinoline (46-1) was used to replace 4-methyl-6-(4,4,5,5-tetramethyl-1,3,2-dioxaborolan-2-yl)quinazoline (1-3) in Step 2 of Example 1, and a method similar to that of Step 2 of Example 1 was adopted to synthesize and obtain the title compound (48-1) of this step (1.2 g, yield: 51*%*).
MS m/z (ESI): 330.1 [M+H]⁺.

### Step 2: Preparation of N-(6-(4-fluorophenyl)-5-(4-methylquinolin-6-yl)pyridin-2-yl)morpholine-4-carboxamide (48)

Morpholine (11-1) was used to replace *N*-methyl-1-pyridine-2-methylamine (In-7-b) in Example 4,6-(4-fluorophenyl)-5-(4-methylquinolin-6-yl)pyridin-2-amine (48-1) was used to replace 3-fluoro-6-(4-fluorophenyl)-5-(4-methylquinazolin-6-yl)pyridin-2-amine (2) in Example 4, and a method similar to that of Example 4 was adopted to synthesize and obtain the title compound (48) (12 mg, yield: 15*%*).
MS m/z (ESI): 443.2 [M+H]⁺.
¹H NMR (400 MHz, DMSO-*d*₆) δ: 8.78 (s, 1H), 8.24-8.17 (m, 2H), 7.93 (d, *J* = 8.8 Hz, 1H), 7.84 (m, 1H), 7.50 (d, *J* = 8.8 Hz, 1H), 7.35-7.30 (m, 3H), 6.97 (t, *J* = 8.8 Hz, 2H), 3.80 (t, *J* = 8.8 Hz, 4H), 3.62 (s,4H), 2.64 (s, 3H).

### Example 49: Preparation of N-(2'-chloro-2-(4-fluorophenyl)-6'-methyl-[3,4-bipyridine]-6-yl) morpholine-4-carboxamide (Compound 49)

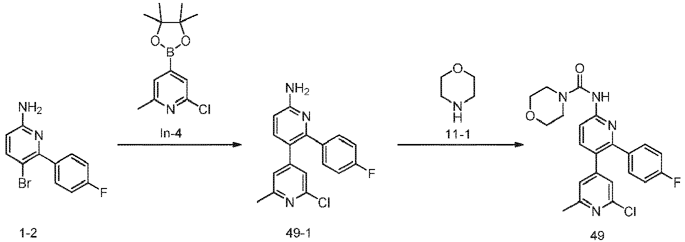

### Step 1: Preparation of 2'-chloro-2-(4-fluorophenyl)-6'-methyl-[3,4-bipyridin]-6-amine (49-1)

2-Chloro-6-methyl-4-(4,4,5,5-tetramethyl-1,3,2-dioxaborolan-2-yl)pyridine (In-4) was used to replace 4-methyl-6-(4,4,5,5-tetramethyl-1,3,2-dioxaborolan-2-yl)quinazoline (1-3) in Step 2 of Example 1, and a method similar to that of Step 2 of Example 1 was adopted to synthesize and obtain the title compound (50-1) of this step (170 mg, yield: 56*%*).
MS m/z (ESI): 314.1 [M+H]⁺.

### Step 2: Preparation of N-(2'-chloro-2-(4-fluorophenyl)-6'-methyl-[3,4-bipyridin]-6-yl)morpholine-4-carboxamide (49)

Morpholine (11-1) was used to replace *N*-methyl-1-pyridine-2-methylamine (In-7-b) in Example 4, 2'-chloro-2-(4-fluorophenyl)-6'-methyl-[3,4-bipyridin]-6-amine (49-1) was used to replace 3-fluoro-6-(4-fluorophenyl)-5-(4-methylquinazolin-6-yl)pyridin-2-amine (2) in Example 4, and a method similar to that of Example 4 was adopted to synthesize and obtain the title compound (49) (100 mg, yield: 42*%*).
MS m/z (ESI): 427.1 [M+H]⁺.
¹H NMR (400 MHz, DMSO-*d*₆) δ: 9.50 (s, 1H), 7.87 (d, *J* = 6.4 Hz, 2H), 7.40-7.32 (m, 2H), 7.20-7.16 (m, 2H), 7.08 (s, 1H), 7.01 (s, 1H), 3.59 (d, *J* = 4.8 Hz, 4H), 3.49 (d, *J* = 4.4 Hz, 4H), 2.36 (s, 3H).

### Biological tests

Test Example 1: Determination of competitive inhibition constant (Kᵢ) for adenosine A1 and A2a receptors Reagents used included:
[³H]-DPCPX (PerkinElmer, NET974250UC),
[³H]-CGS-21680 (PerkinElmer, NET1021250UC),
Adenosine A1 receptor (human) membrane (PerkinElmer, ES-010-M400UA),
Adenosine A2a receptor (human) membrane (PerkinElmer, RBHA2AM400UA),
Microscint 20 liquid scintillation cocktail (PerkinElmer, 6013329),
PEI (Poly ethyleneimine) (Sigma, P3143), and
CGS-15943 (Sigma, C199).

Buffers used included:
assay buffer for A1 (25mM HEPES, 5mM MgCl₂, 1mM CaCl₂, 100mM NaCl, pH7.4), used to dilute adenosine A1 receptor (human) membrane and [³H]-DPCPX;
wash buffer for A1 (25mM HEPES, 5mM MgCl₂, 1mM CaCl₂, 100mM NaCl, pH7.4);
assay buffer for A2a (50mM Tris-HCl, 10mM MgCl₂, 1mM EDTA, pH7.4), used to dilute adenosine A2a receptor (human) membrane (human origin) and [³H]-CGS-21680; and
wash buffer for A2a (50mM Tris-HCl, 154mM NaCl, pH7.4).

### Test method

Adenosine A1 receptor membrane was diluted to 0.025 µg/µl with assay buffer for A1, and Adenosine A2a receptor membrane was diluted to 0.05 µg/µl with assay buffer for A2a, so as to obtain Adenosine A1 receptor membrane dilution and Adenosine A2a receptor membrane dilution.

The compound to be tested and CGS-15943 were diluted with DMSO, and 1 µl of the compound to be tested, high value control (0.5*%* DMSO), low value control (1000 nM CGS-15943) were added to a 96-well plate, and 100 µl of Adenosine A1 receptor membrane dilution (containing 2.5 µg of membrane) was added to each well of the 96-well plate to obtain A1 detection plate; following the same steps, 100 µl of Adenosine A2a receptor membrane dilution (containing 5.0 µg membrane) was added to each well of the 96-well plate to obtain A2a detection plate.

To the A1 detection plate, 100µl of radioisotope-labeled ligand [³H]-DPCPX (diluted with assay buffer for A1, working concentrationof which was 1.0nM) was added; and to the A2a detection plate, 100µl of radioisotope-labeled ligand [³H]-CGS-21680 (diluted with assay buffer for A2a, working concentration of which was 6.0nM) was added. The A1 and A2a detection plates were sealed with tape, and incubated at room temperature for 1h and 2h, respectively.

Unifilter-96 GF/C filter plates were prepared, 50 µl of 0.3*%* PEI was added to each well of the Unifilter-96 GF/C filter plates, and the resulting filter plates were incubated at room temperature for not less than 0.5h.

After the incubation was completed, the reaction solution was transferred from the A1 detection plate and the A2a detection plate to two Unifilter-96 GF/C filter plates, and washed with pre-cooled wash buffer for A1 and wash buffer for A2a, and then the filter plates were dried, sealed at bottom, then 50 µl of Microscint 20 fluid scintillation cocktail was added, and then the filter plates were sealed at top, and counted by MicroBeta2 Reader.

### Data analysis

The following formula was used to calculate inhibition rate:
Inhibition rate *%* = 100 - (signal value of test well - signal average value of low value control) / (signal average value of high value control - signal average value of low value control)^{∗}100.

EXCEL XLfit was used to fit IC₅₀.

The calculation formula for competitive inhibition constant (Kᵢ) was: Kᵢ = IC₅₀/ (1 + concentration of isotope-labeled ligand / K_{d}), wherein K_{d} was the dissociation constant of the isotope-labeled ligand.

The test results of competitive inhibition constants (Kᵢ) of the compounds of the present application for adenosine A2a and A1 receptors were shown in Table 1.

**Table 1: Competitive inhibition constants (Ki) of the compounds of the present application for adenosine A2a and A1 receptors**

| Compound No. | Ki_{(A1)} (nM) | Ki_{(A2a)} (nM) | Ki_{(A1)}/Ki_{(A2a)} |
|---|---|---|---|
| 1 | 3254.5 | 9.6 | 339.0 |
| 2 | 698.2 | 2.7 | 258.6 |
| 3 | 178.1 | 1.6 | 111.3 |
| 4 | 5675.2 | 13.6 | 417.3 |
| 5 | >7916.7 | 15.6 | >507.5 |
| 6 | >7916.7 | 14.3 | >553.6 |
| 9 | 1227.7 | 3.7 | 331.8 |
| 10 | 690.1 | 4.7 | 146.8 |
| 11 | 203.8 | 3.4 | 59.9 |
| 12 | 1211.1 | 8.8 | 137.6 |
| 13 | 1318.5 | 9.8 | 134.5 |
| 14 | 5314.3 | 8.1 | 656.1 |
| 16 | 7774.8 | 21.5 | 361.6 |
| 17 | 204.8 | 4.6 | 44.5 |
| 18 | 3072.1 | 18.9 | 162.5 |
| 19 | 1404.3 | 9.6 | 146.3 |
| 21 | 4665.1 | 7.3 | 639.1 |
| 22 | 1588.6 | 6.6 | 240.7 |
| 24 | 3727.5 | 26.8 | 139.1 |
| 25 | 178.2 | 3.9 | 45.7 |
| 26 | 3998 | 9.9 | 403.8 |
| 27 | 421.8 | 4 | 105.5 |
| 28 | 1777.8 | 9 | 197.5 |
| 29 | 5025.8 | 11.8 | 425.9 |
| 30 | 1591 | 21.2 | 75.0 |
| 31 | 3327.4 | 27.7 | 120.1 |
| 32 | 534.1 | 9.5 | 56.2 |
| 34 | 537.6 | 12.6 | 42.7 |
| 35 | 1865.8 | 14.7 | 126.9 |
| 36 | 583.1 | 10.6 | 55.0 |
| 37 | 4047.2 | 23.3 | 173.7 |
| 38 | 1406.8 | 13.1 | 107.4 |
| 39 | 814 | 11 | 74.0 |
| 40 | 411.4 | 5.4 | 76.2 |
| 41 | 2532.54 | 1.92 | 1319.0 |
| 42 | 332.58 | 2.3 | 144.6 |
| 43 | 133.63 | 1.36 | 98.3 |
| 44 | 931 | 14.5 | 64.2 |
| 45 | 1365.63 | 11.84 | 115.3 |
| 47 | 217.39 | 5.13 | 42.4 |

The data in Table 1 shows that the compounds of the present application have strong affinity for adenosine A2a receptor, and weak affinity for adenosine A1 receptor. The compounds of the present application have a good selectivity for adenosine A2a receptor.

Test Example 2: Study on inhibitory activity for ADORA2a/CHOK1 cells

Reagents used included:
Adenosine (Sigma, A4036),
Ham's F-12 medium (Hyclone, SH30526.01),
Hygromycin B (Invitrogen, 10687010),
Zeocin (Invitrogen, R25001),
cAMP detection kit (Cisbio, 62AM4PEB), and
ADORA2a/CHOK1 cells (Cobai, CBP71017).
Solution preparation
Preparation of cell culture media
FBS was added to Ham's F-12 medium to a concentration of 10% (v/v), Zeocin was added to a final concentration of 200 µg/mL, and Hygromycin B was added to a final concentration of 100 µg/mL, and the resulting mixture was stored at 4°C for later use.

### Preparation of 10µM adenosine solution

A certain amount of adenosine was precisely weighed and placed into a 1.5mL EP tube, then a certain volume of cell culture medium was added, the resulting mixture was incubated at 37°C for dissolution, thereby a 10mM stock solution was prepared. The stock solution was diluted with cell culture medium at 1:1000 to get 10µM adenosine solution.

### Preparation of compound to be tested

The compound to be tested was dissolved in DMSO to form a 10mM stock solution, and the stock solution was stored in a refrigerator at 4°C for later use. The stock solution was diluted with the 10µM of adenosine solution to 2000nM or 200 nM for later test.

### Test method

1.Inoculation of cells to plate
   (1) CHO-K1 cells stably expressing ADORA2A receptor were cultured in a 37°C, 5*%* CO₂ cell incubator. When the cell confluence reached about 80*%*, the cells were digested by trypsin, dispersed and counted; (2) the cell concentration was adjusted to 6 × 10⁵ cells/mL according to the counting results; (3) the cells was inoculated into a white 384-well cell culture plate at 5µL per well (about 3000 cells/well).
2. Co-incubation of compound and cells
   All groups were provided with multiple wells, the adenosine-free cell culture medium was added to the wells of the Blank group (5µL/well); 10µM adenosine solution was added to the wells of the Agonist group (5µL/well); the diluted compound was added to the wells of the test compound group (5µL/well). After the addition of the compound was completed, the 384-well plate was centrifuged at 2500 rpm for 30 seconds to fully mix the compound and the medium, then placed in a 25°C incubator and incubated for 30 min.
3. Adding detection reagents
   (1) Preparation of detection solutions
      Solution A
         A certain volume of cAMP-d2 was taken, and diluted by adding Lysis & Detection Buffer at the ratio of 1:5 to prepare Solution A.
      Solution B
         A certain volume of cAMP-Cryptate was taken, and diluted by adding Lysis & Detection Buffer at the ratio of 1:5 to prepare Solution B.
   (2) After 30min of incubation in step 2 was completed, addition of detection solution was carried out. The 384-well plate was taken out, 5µL of Solution A was added to each well, and then 5µL of Solution B was added to each well, then the 384-well plate was covered with sealing membrane, centrifuged at 2500rpm for 30s (to fully mix the detection solution and medium), and incubated in a thermostat at 25°C for 1h.
4. Reading plate

After the incubation, the 384-well plate was taken out, and detected by PHERA Star FS to read data, detection wavelength: 620nm/665nm.

Data analysis included raw data processing and calcutation of inhibition rate:
Raw data processing
R=(reading at 665nm / reading at 620nm)^{∗}10000.
inhibition rate *%* = (1-(R_{Compound}-R_{Blank}) / (R_{Agonist}-R_{Blank}))^{∗}100*%*
wherein, R_{Compound} represented the R Value of the test compound group, R_{Agonist} represented the R value of the Agonist group, and R_{Blank} represented the R value of the Blank group.

The test results of the inhibitory activity (inhibition rate) of the compounds of the present application for ADORA2a/CHOK1 cells were shown in Table 2.

**Table 2: Inhibitory activity (inhibition rate) of the compounds of the present application for ADORA2a/CHOK1 cells**

| Compound No. | Inhibition rate at 1000nM (*%*) | Inhibition rate at 100nM (*%*) |
|---|---|---|
| 11 | 111.8 | 72.0 |
| 19 | 94.4 | 33.9 |
| 43 | 100.2 | 59.1 |
| 44 | 104.0 | 64.8 |

The data in Table 2 show that the compounds of the present application have good antagonistic activity against the adenosine A2a receptor of CHOK1 cells.

Test Example 3: Study on pharmacokinetics (PK) and distribution in brain tissue in rats

The compounds of the present application were administered to male SD rats by intravenous (IV) and intragastric (PO) respectively to investigate the pharmacokinetic characteristics. The dosages of IV and PO were 1 mg/kg and 5 mg/kg, respectively. The solvent for IV was a mixture of 5*%* DMSO, 5*%* Solutol (polyethylene glycol 15-hydroxystearate) and 90*%* saline, and the solvent for PO was 0.5*%* MC (methyl cellulose sodium). Before IV administration (0h) and at 0.083h, 0.25h, 0.5h, 1h, 2h, 4h, 6h, 8h and 24h time points after administration, blood samples were collected; before PO administration (0h) and at 0.25h, 0.5h, 1h, 2h, 4h, 6h, 8h and 24h time points after administration, blood samples were collected; the blood samples were anticoagulated with EDTA.K₂, and centrifuged to obtain plasma samples. For Compound 11, brain tissue samples were taken and homogenized at 0.25h, 0.5h, 1h, 4h and 8h; for Compounds 19 and 32, and brain tissue samples were taken and homogenized at 0.25h, 0.5h, 1h and 8h (three SD rats were selected for the experiment of distribution in brain tissue at each time point), and the resulting brain tissue homogenate samples were stored at -80°C. The plasma samples and brain tissue homogenate samples were subjected to precipitation of protein, and then LC-MS/MS analysis was performed.

Pharmacokinetic parameters were calculated by means of non-compartmental model by using WinNonlin 6.3 software. The results were shown in Table 3 and Table 4.

**Table 3: Pharmacokinetic parameters of compounds administered by IV in rats**

| Compound No. | Administration route | Dose | AUCₗₐₛₜ | Cₘₐₓ |
|---|---|---|---|---|
| | | mg/kg | h^{∗}ng/mL | ng/mL |
| 11 | IV | 1 | 985 | 1311 |
| 19 | IV | 1 | 2631 | 1150 |
| 32 | IV | 1 | 1822 | 1480 |

As shown in Table 3, the exposures (AUCₗₐₛₜ) of Compounds 11, 19 and 32 of the present application in rats by IV administration at a dose of 1 mg/kg were 985 h^{∗}ng/mL, 2631 h^{∗}ng/mL, 1822 h^{∗}ng/mL, respectively, the maximum plasma concentrations (Cₘₐₓ) of Compounds 11, 19 and 32 in rats were 1311 ng/mL, 1150 ng/mL, 1480 ng/mL, respectively, which indicated that the Compounds 11, 19 and 32 of the present application have excellent drug exposure in rats by IV administration.

**Table 4: Pharmacokinetic parameters of compounds administered by PO in rats**

| Compound No. | Administration route | Dose | AUCₗₐₛₜ | Plasma AUCₗₐₛₜ/brain AUCₗₐₛₜ |
|---|---|---|---|---|
| | | mg/kg | | |
| 11 | PO | 5 | 3218 h^{∗}ng/mL (plasma) | 282/1 |
| | | | 11.4 h^{∗}ng/g (brain) | |
| 19 | PO | 5 | 11419 h^{∗}ng/mL (plasma) | 163/1 |
| | | | 70.2 h^{∗}ng/g (brain) | |
| 32 | PO | 5 | 7306 h^{∗}ng/mL (plasma) | 138/1 |
| | | | 52.9 h^{∗}ng/g (brain) | |

As shown in Table 4, the AUCₗₐₛₜ of Compounds 11, 19 and 32 of the present application in rat plasma by PO administration at a dose of 5 mg/kg were 3218 h^{∗}ng/mL, 11419 h^{∗}ng/mL and 7306 h^{∗}ng/mL, respectively; the AUCₗₐₛₜ thereof in rat brain tissue were 11.4 h^{∗}ng/g, 70.2 h^{∗}ng/g and 52.9 h^{∗}ng/g, respectively. It could be obtained that for Compounds 11, 19 and 32, the ratios of AUCₗₐₛₜ in rat plasma to AUCₗₐₛₜ in brain tissue were 282/1, 163/1 and 138/1, respectively, indicating that Compounds 11, 19 and 32 of the present application have excellent drug exposure in rat plasma by PO administration, and the proportion crossing the blood-brain barrier is very low.

According to calculations, compared with intravenous administration, the bioavailability of Compounds 11, 19 and 32 orally administrated in rats were 65.3%, 86.8% and 80.2%, respectively, indicating that the compounds of the present application (e.g., Compounds 11, 19 and 32) have excellent absorption effect for oral administration in rats.

Although the specific embodiments of the present application has been described in detail, those skilled in the art will understand that according to all the teachings that have been disclosed, various modifications and changes can be made to the details, and these modifications and changes are within the scope of protection of this application. The full scope of the present application is given by the claims and any equivalents thereof.

## Claims

1. A compound represented by Formula I, a pharmaceutically acceptable salt, a stereoisomer, a polymorph, a solvate, a *N*-oxide, an isotope-labeled compound, a metabolite or a prodrug thereof, 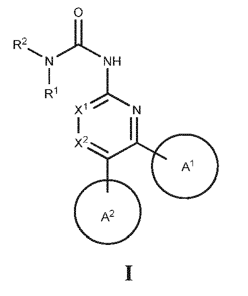 wherein:
X¹ is N or CR³;
X² is N or CR⁴;
and when X¹ is CR³, X² is not N;
ring A¹ is selected from the group consisting of C₃₋₁₀ cycloalkyl, 4- to 12-membered heterocyclyl, C₆₋₁₀ aryl and 5- to 10-membered heteroaryl, the ring A¹ is optionally substituted by one or more substituents independently selected from the group consisting of: halogen, cyano, nitro, C₁₋₆ alkyl, C₁₋₆ haloalkyl, C₃₋₁₀ cycloalkyl, hydroxy, C₁₋₆ alkoxy, amino, C₁₋₆ alkyl-amino-, di(C₁₋₆ alkyl)-amino- and 4- to 12-membered heterocyclyl;
ring A² is selected from the group consisting of C₃₋₁₀ cycloalkyl, 4- to 12-membered heterocyclyl, C₆₋₁₀ aryl and 5- to 10-membered heteroaryl; preferably, the 5- to 10-membered heteroaryl is 5- to 10-membered nitrogen-containing heteroaryl, the ring A² is optionally substituted by one or more substituents independently selected from the group consisting of: halogen, cyano, nitro, C₁₋₆ alkyl, C₁₋₆ haloalkyl, C₃₋₁₀ cycloalkyl, hydroxy, C₁₋₆ alkoxy, amino, C₁₋₆ alkyl-amino-, di(C₁₋₆ alkyl)-amino- and 4- to 12-membered heterocyclyl, and ring A² is not 1-isopropyl-6-oxo-1,6-dihydropyridin-3-yl;
R¹ and R² each are independently selected from the group consisting of hydrogen, C₁₋₆ alkyl, C₃₋₁₀ cycloalkyl, 4- to 12-membered heterocyclyl, C₆₋₁₀ aryl, 5- to 10-membered heteroaryl, 4- to 12-membered heterocyclyl-C₁₋₆ alkyl- and 5- to 10-membered heteroaryl-C₁₋₆ alkyl-, wherein the C₁₋₆ alkyl, C₃₋₁₀ cycloalkyl, 4- to 12-membered heterocyclyl, C₆₋₁₀ aryl, 5- to 10-membered heteroaryl, 4- to 12-membered heterocyclyl-C₁₋₆ alkyl- or 5- to 10-membered heteroaryl-C₁₋₆ alkyl- is optionally substituted by one or more substituents independently selected from the group consisting of: halogen, cyano, hydroxy, C₁₋₆ alkyl, C₁₋₆ haloalkyl, C₁₋₆ alkoxy, C₃₋₁₀ cycloalkyl and 4- to 12-membered heterocyclyl; or,
R¹ and R² together with the nitrogen atom linked to them form a 4- to 12-membered nitrogen-containing heterocyclyl, preferably, the 4- to 12-membered nitrogen-containing heterocyclyl comprises one or more heteroatoms selected from the group consisting of nitrogen atom and oxygen atom; the 4- to 12-membered nitrogen-containing heterocyclyl is optionally substituted by one or more substituents independently selected from the group consisting of: halogen, cyano, hydroxy, C₁₋₆ alkyl, C₁₋₆ haloalkyl, C₁₋₆ alkoxy, C₁₋₆ alkoxy-C₁₋₆ alkyl-, C₁₋₆ alkoxy-C₁₋₆ alkoxy-, C₃₋₁₀ cycloalkyl and 4- to 12-membered heterocyclyl;
R³ and R⁴ each are independently selected from the group consisting of hydrogen, halogen, cyano, hydroxy, amino, C₁₋₆ alkyl, C₁₋₆ haloalkyl, C₁₋₆ alkoxy, C₃₋₆ cycloalkyl, C₁₋₆ alkyl-amino-, di(C₁₋₆ alkyl)-amino-; preferably, R³ and R⁴ each are independently selected from the group consisting of hydrogen, halogen, cyano, hydroxy, amino, C₁₋₆ alkyl, C₁₋₆ haloalkyl, C₁₋₆ alkoxy, C₁₋₆ alkyl-amino- and di(C₁₋₆ alkyl)-amino-,
and
the compound is not the following compounds:
1-(5,6-diphenyl-1,2,4-triazin-3-yl)-3-phenylurea,
1-(5-(2-bromo-5-hydroxyphenyl)-4-ethyl-6-phenylpyrimidin-2-yl)urea,
1-(5-(2-chloro-5-hydroxyphenyl)-4-ethyl-6-phenylpyrimidin-2-yl)urea,
1-(3,5-di(trifluoromethyl)phenyl)-3-(4-(3-hydroxy-5-methylphenyl)-5-(pyridin-4-yl)pyrimidin-2-yl)urea,
1-(4-chloro-3-(trifluoromethyl)phenyl)-3-(4-(3-hydroxy-5-methylphenyl)-5-(pyridin-4-yl)pyrimidin-2-yl)urea,
1-(3,5-di(trifluoromethyl)phenyl)-3-(4-(3-methoxy-5-methylphenyl)-5-(pyridin-4-yl)pyrimidin-2-yl)urea,
1-(4-chloro-3-(trifluoromethyl)phenyl)-3-(4-(3-methoxy5-methylphenyl)-5-(pyridin-4-yl)pyrimidin-2-yl)urea,
1-(4-chloro-3-(trifluoromethyl)phenyl)-3-(4-(2-hydroxyphenyl)-5-(4-methoxyphenyl)pyrimidin-2-yl)urea.

2. The compound, a pharmaceutically acceptable salt, a stereoisomer, a polymorph, a solvate, a *N*-oxide, an isotope-labeled compound, a metabolite or a prodrug thereof according to Claim 1, wherein the compound is not 1-(6-(2-chloro-6-methylpyridin-4-yl)-5-(4-fluorophenyl)-1,2,4-triazin-3-yl)urea.

3. The compound, a pharmaceutically acceptable salt, a stereoisomer, a polymorph, a solvate, a *N*-oxide, an isotope-labeled compound, a metabolite or a prodrug thereof according to Claim 1, wherein the compound has the structure represented by Formula IIa, Formula IIb or Formula IIc, 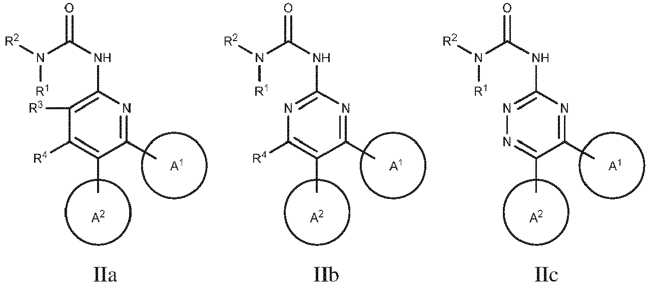 wherein: ring A¹, ring A², R¹, R², R³ and R⁴ have the definitions as described in Claim 1.

4. The compound, a pharmaceutically acceptable salt, a stereoisomer, a polymorph, a solvate, a *N*-oxide, an isotope-labeled compound, a metabolite or a prodrug thereof according to any of Claims 1 to 3, wherein:
ring A¹, ring A², R³ and R⁴ have the definitions as described in Claim 2,
R¹ and R² each are independently selected from the group consisting of hydrogen, C₁₋₆ alkyl, C₃₋₈ cycloalkyl, 4- to 8-membered heterocyclyl, phenyl, 5- to 6-membered heteroaryl, 4- to 7-membered heterocyclyl-C₁₋₆ alkyl- or 5- to 6-membered heteroaryl-C₁₋₆ alkyl-, wherein the C₁₋₆ alkyl, C₃₋₈ cycloalkyl, 4- to 8-membered heterocyclyl, phenyl, 5- to 6-membered heteroaryl, 4- to 7-membered heterocyclyl-C₁₋₆ alkyl- or 5- to 6-membered heteroaryl-C₁₋₆ alkyl- is optionally substituted by one or more substituents independently selected from the group consisting of: halogen, cyano, hydroxy, C₁₋₆ alkyl, C₁₋₆ haloalkyl, C₁₋₆ alkoxy, C₃₋₆ cycloalkyl and 4- to 7-membered heterocyclyl; or,
R¹ and R² together with the nitrogen atom linked to them form a 4- to 12-membered nitrogen-containing heterocyclyl (e.g., 4- to 6-membered nitrogen-containing heterocyclyl), preferably, the 4- to 12-membered nitrogen-containing heterocyclyl comprises one or more heteroatoms selected from the group consisting of nitrogen atom and oxygen atom; the 4- to 12-membered nitrogen-containing heterocyclyl (e.g., 4- to 6-membered nitrogen-containing heterocyclyl) is optionally substituted by one or more substituents independently selected from the group consisting of: halogen, cyano, hydroxy, C₁₋₆ alkyl, C₁₋₆ haloalkyl, C₁₋₆ alkoxy, C₁₋₆ alkoxy-C₁₋₆ alkyl-, C₁₋₆ alkoxy-C₁₋₆ alkoxy-, C₃₋₁₀ cycloalkyl and 4- to 12-membered heterocyclyl;
preferably, R³ is fluoro;
preferably, R⁴ is hydrogen.

5. The compound, a pharmaceutically acceptable salt, a stereoisomer, a polymorph, a solvate, a N-oxide, an isotope-labeled compound, a metabolite or a prodrug thereof according to any of Claims 1 to 4, wherein:
ring A¹ is selected from the group consisting of phenyl and 5- to 6-membered heteroaryl, the ring A¹ is optionally substituted by one or more substituents independently selected from the group consisting of: halogen, cyano, nitro, C₁₋₆ alkyl, C₁₋₆ haloalkyl, C₃₋₆ cycloalkyl and 4- to 7-membered heterocyclyl;
preferably, ring A¹ is selected from the group consisting of phenyl and furyl, the ring A¹ is optionally substituted by one or more substituents independently selected from the group consisting of: fluoro, chloro, bromo, cyano, nitro, methyl, ethyl, n-propyl, isopropyl, trifluoromethyl, cyclopropyl, cyclobutyl and cyclopentyl;
preferably, ring A¹ is selected from the group consisting of phenyl, furan-2-yl and furan-3-yl, the ring A¹ is optionally substituted by one or more substituents independently selected from the group consisting of: fluoro, chloro, bromo, cyano, nitro, methyl, ethyl, n-propyl, isopropyl, trifluoromethyl, cyclopropyl, cyclobutyl and cyclopentyl;
preferably, ring A¹ is selected from the group consisting of phenyl and furan-2-yl, the ring A¹ is optionally substituted by one or more substituents independently selected from the group consisting of: fluoro, chloro, bromo, cyano, nitro, methyl, ethyl, n-propyl, isopropyl and trifluoromethyl;
preferably, ring A¹ is selected from the group consisting of phenyl, 4-fluorophenyl, 4-ethylphenyl, 4-nitrophenyl, 2-fluorophenyl, 3-fluorophenyl, 4-methylphenyl, 2-methylphenyl, 3-methylphenyl, 5-methylfuran-2-yl, 3-methyl-furan-2-yl and 4-methyl-furan-2-yl;
preferably, ring A¹ is selected from the group consisting of 4-fluorophenyl and 5-methyl-furan-2-yl;
preferably, ring A¹ is 4-fluorophenyl.

6. The compound, a pharmaceutically acceptable salt, a stereoisomer, a polymorph, a solvate, a *N*-oxide, an isotope-labeled compound, a metabolite or a prodrug thereof according to any of Claims 1 to 5, wherein:
ring A² is selected from the group consisting of C₆₋₁₀ aryl and 5- to 10-membered heteroaryl, the ring A² is optionally substituted by one or more substituents independently selected from the group consisting of: halogen, cyano, nitro, C₁₋₆ alkyl, C₁₋₆ haloalkyl, C₃₋₆ cycloalkyl and 4- to 7-membered heterocyclyl;
preferably, ring A² is selected from the group consisting of phenyl, pyridyl and quinazolinyl, the ring A² is optionally substituted by one or more substituents independently selected from the group consisting of: halogen, cyano, nitro, C₁₋₆ alkyl, C₁₋₆ haloalkyl, C₃₋₆ cycloalkyl and 4- to 7-membered heterocyclyl;
preferably, ring A² is selected from the group consisting of phenyl, pyridin-2-yl, pyridin-3-yl, pyridin-4-yl, quinazolin-2-yl, quinazolin-4-yl, quinazolin-5-yl, quinazolin-6-yl, quinazolin-7-yl and quinazolin-8-yl, the ring A² is optionally substituted by one or more substituents independently selected from the group consisting of:
halogen, cyano, nitro, C₁₋₆ alkyl, C₁₋₆ haloalkyl, C₃₋₆ cycloalkyl and 4- to 7-membered heterocyclyl;
preferably, ring A² is selected from the group consisting of phenyl, pyridin-4-yl and quinazolin-6-yl, the ring A² is optionally substituted by one or more substituents independently selected from the group consisting of: fluoro, chloro, bromo, cyano, nitro, methyl, ethyl, n-propyl, isopropyl and trifluoromethyl;
preferably, ring A² is selected from the group consisting of 3-chloro-5-methylphenyl, 2-chloro-6-methylpyridin-4-yl, 2-chloro-6-(trifluoromethyl)-pyridin-4-yl and 4-methylquinazolin-6-yl; preferably, ring A² is 4-methylquinazolin-6-yl;
preferably, ring A² is 2-chloro-6-methylpyridin-4-yl.

7. The compound, a pharmaceutically acceptable salt, a stereoisomer, a polymorph, a solvate, a *N*-oxide, an isotope-labeled compound, a metabolite or a prodrug thereof according to any of Claims 1 to 6, wherein:
R¹ and R² each are independently selected from the group consisting of hydrogen, C₁₋₆ alkyl, C₃₋₆ cycloalkyl, 4- to 7-membered heterocyclyl, phenyl, 5- to 6-membered heteroaryl, 4- to 7-membered heterocyclyl-C₁₋₆ alkyl- and 5- to 6-membered heteroaryl-C₁₋₆ alkyl-, wherein the C₁₋₆ alkyl, C₃₋₆ cycloalkyl, 4- to 7-membered heterocyclyl, phenyl, 5- to 6-membered heteroaryl, 4- to 7-membered heterocyclyl-C₁₋₆ alkyl- or 5- to 6-membered heteroaryl-C₁₋₆ alkyl- is optionally substituted by one or more substituents independently selected from the group consisting of: halogen, cyano, hydroxy, C₁₋₆ alkyl, C₁₋₆ haloalkyl, C₁₋₆ alkoxy, C₃₋₆ cycloalkyl and 4- to 7-membered heterocyclyl;
preferably, R¹ and R² each are independently selected from the group consisting of hydrogen, C₁₋₆ alkyl, C₃₋₆ cycloalkyl, 4- to 7-membered heterocyclyl, phenyl, 5- to 6-membered heteroaryl, 4- to 7-membered heterocyclyl-C₁₋₆ alkyl- and 5- to 6-membered heteroaryl-C₁₋₆ alkyl-, wherein the C₁₋₆ alkyl, C₃₋₆ cycloalkyl, 4-to 7-membered heterocyclyl, phenyl, 5- to 6-membered heteroaryl, 4- to 7-membered heterocyclyl-C₁₋₆ alkyl- or 5- to 6-membered heteroaryl-C₁₋₆ alkyl- is optionally substituted by one or more substituents independently selected from the group consisting of: fluoro, chloro, bromo, cyano, hydroxy, methyl, ethyl, n-propyl, isopropyl, trifluoromethyl, methoxy, ethoxy, propoxy, cyclopropyl, cyclobutyl, cyclopentyl, cyclohexyl, piperidin-1-yl, piperidin-2-yl, piperidin-3-yl and piperidin-4-yl;
preferably, R¹ and R² each are independently selected from the group consisting of hydrogen, methyl, ethyl, n-propyl, isopropyl, n-butyl, sec-butyl, isobutyl, tert-butyl, cyclopropyl, cyclobutyl, cyclopentyl, cyclohexyl, piperidinyl, phenyl, pyridyl, pyridin-2-ylmethyl, pyridin-2-ylethyl, pyridin-2-ylpropyl, methoxyethyl and 2-hydroxy-2-methyl-propyl;
preferably, R¹ and R² each are independently selected from the group consisting of hydrogen, methyl, methoxyethyl, 2-hydroxy-2-methyl-propyl, phenyl and pyridin-2-ylmethyl;
preferably, R¹ is hydrogen or methyl, and R² is hydrogen, methyl, phenyl, pyridin-2-ylmethyl, 2-hydroxy-2-methyl-propyl or 2-methoxyethyl.

8. The compound, a pharmaceutically acceptable salt, a stereoisomer, a polymorph, a solvate, a *N*-oxide, an isotope-labeled compound, a metabolite or a prodrug thereof according to any of Claims 1 to 7, wherein:
R¹ and R² together with the nitrogen atom linked to them form a 4- to 7-membered nitrogen-containing heterocyclyl, the 4- to 7-membered nitrogen-containing heterocyclyl is optionally substituted by one or more substituents independently selected from the group consisting of: halogen, cyano, hydroxy, C₁₋₆ alkyl, C₁₋₆ haloalkyl, C₁₋₆ alkoxy, C₁₋₆ alkoxy-C₁₋₆ alkyl-, C₁₋₆ alkoxy-C₁₋₆ alkoxy-, C₃₋₆ cycloalkyl and 4- to 7-membered heterocyclyl;
preferably, R¹ and R² together with the nitrogen atom linked to them form a 4- to 7-membered nitrogen-containing heterocyclyl, the 4- to 7-membered nitrogen-containing heterocyclyl comprises one or more heteroatoms selected from the group consisting of nitrogen atom and oxygen atom; the 4- to 7-membered nitrogen-containing heterocyclyl is optionally substituted by one or more substituents independently selected from the group consisting of: halogen, cyano, hydroxy, C₁₋₆ alkyl, C₁₋₆ haloalkyl, C₁₋₆ alkoxy, C₁₋₆ alkoxy-C₁₋₆ alkyl-, C₁₋₆ alkoxy-C₁₋₆ alkoxy-, C₃₋₆ cycloalkyl and 4- to 7-membered heterocyclyl;
preferably, R¹ and R² together with the nitrogen atom linked to them form a 6-membered nitrogen-containing heterocyclyl, the 6-membered nitrogen-containing heterocyclyl comprises one or more heteroatoms selected from the group consisting of nitrogen atom and oxygen atom; the 6-membered nitrogen-containing heterocyclyl is optionally substituted by one or more substituents independently selected from the group consisting of: halogen, cyano, hydroxy, C₁₋₆ alkyl, C₁₋₆ haloalkyl, C₁₋₆ alkoxy, C₁₋₆ alkoxy-C₁₋₆ alkyl-, C₁₋₆ alkoxy-C₁₋₆ alkoxy-, C₃₋₆ cycloalkyl and 4- to 7-membered heterocyclyl;
preferably, R¹ and R² together with the nitrogen atom linked to them form a piperidinyl or morpholinyl, the piperidinyl or morpholinyl is optionally substituted by one or more substituents independently selected from the group consisting of: fluoro, chloro, bromo, cyano, hydroxy, methyl, ethyl, n-propyl, isopropyl, n-butyl, sec-butyl, isobutyl, tert-butyl, cyclopropyl, cyclobutyl, cyclopentyl, cyclohexyl, trifluoromethyl, methoxy, ethoxy, propoxy, piperidin-1-yl, piperidin-1-yl, piperidin-2-yl, piperidin-3-yl and piperidin-4-yl; or, R¹ and R² together with the nitrogen atom linked to them form a piperazinyl, the piperazinyl is optionally substituted by one or more substituents independently selected from the group consisting of: fluoro, chloro, bromo, cyano, hydroxy, methyl, ethyl, n-propyl, isopropyl, n-butyl, sec-butyl, isobutyl, tert-butyl, cyclopropyl, cyclobutyl, cyclopentyl, cyclohexyl, trifluoromethyl, methoxy, ethoxy, propoxy, piperidin-1-yl, piperidin-1-yl, piperidin-2-yl, piperidin-3-yl and piperidin-4-yl;
preferably, R¹ and R² together with the nitrogen atom linked to them form a piperidinyl, morpholinyl, 4-cyanopiperidinyl, 4-methoxypiperidinyl or 1,4'-bipiperidyl;
preferably, R¹ and R² together with the nitrogen atom linked to them form a morpholinyl, the morpholinyl is optionally substituted by one or more substituents independently selected from the group consisting of: methyl, ethyl, n-propyl, isopropyl, n-butyl, sec-butyl, isobutyl and tert-butyl;
preferably, R¹ and R² together with the nitrogen atom linked to them form a morpholinyl, 2-methylmorpholinyl or 2,6-dimethylmorpholinyl.

9. The compound, a pharmaceutically acceptable salt, a stereoisomer, a polymorph, a solvate, a *N*-oxide, an isotope-labeled compound, a metabolite or a prodrug thereof according to any of Claims 1 to 8, wherein:
R³ and R⁴ each are independently selected from the group consisting of hydrogen, halogen, cyano, hydroxy, amino, C₁₋₆ alkyl, C₁₋₆ haloalkyl, C₁₋₆ alkoxy, C₁₋₆ alkyl-amino- and di(C₁₋₆ alkyl)-amino-;
preferably, R³ and R⁴ each are independently selected from the group consisting of hydrogen, fluoro, chloro, bromo, cyano, hydroxy, amino, methyl, ethyl, n-propyl, isopropyl, trifluoromethyl, methoxy, ethoxy, propoxy, methylamino, ethylamino, dimethylamino, diethylamino, cyclopropyl, cyclobutyl, cyclopentyl and cyclohexyl;
preferably, R³ and R⁴ each are independently selected from the group consisting of hydrogen, fluoro, chloro, bromo, cyano, hydroxy, amino, methyl, ethyl, n-propyl, isopropyl, trifluoromethyl, methoxy, ethoxy, propoxy, methylamino, ethylamino, dimethylamino and diethylamino;
preferably, R³ is selected from the group consisting of hydrogen, fluoro, chloro, bromo, methyl, ethyl, n-propyl, isopropyl, trifluoromethyl, methoxy, ethoxy, propoxy, methylamino, ethylamino, dimethylamino and diethylamino;
preferably, R⁴ is selected from the group consisting of hydrogen, fluoro, chloro, bromo, methyl, ethyl, n-propyl, isopropyl, trifluoromethyl, methoxy, ethoxy, propoxy, methylamino, ethylamino, dimethylamino, diethylamino, cyclopropyl, cyclobutyl, cyclopentyl and cyclohexyl;
preferably, R⁴ is selected from the group consisting of hydrogen, fluoro, chloro, bromo, methyl, ethyl, n-propyl, isopropyl, trifluoromethyl, methoxy, ethoxy, propoxy, methylamino, ethylamino, dimethylamino and diethylamino; preferably, R⁴ is hydrogen, chloro, cyano or cyclopropyl;
preferably, R⁴ is hydrogen.

10. A compound, a pharmaceutically acceptable salt, a stereoisomer, a polymorph, a solvate, a *N*-oxide, an isotope-labeled compound, a metabolite or a prodrug thereof, wherein the compound is selected from the group consisting of:

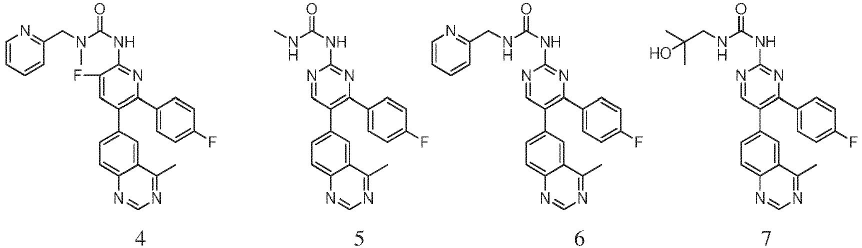

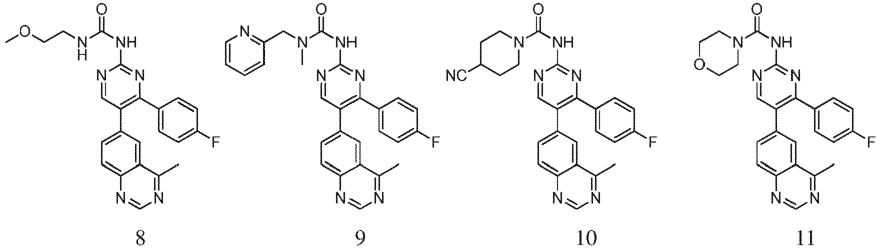

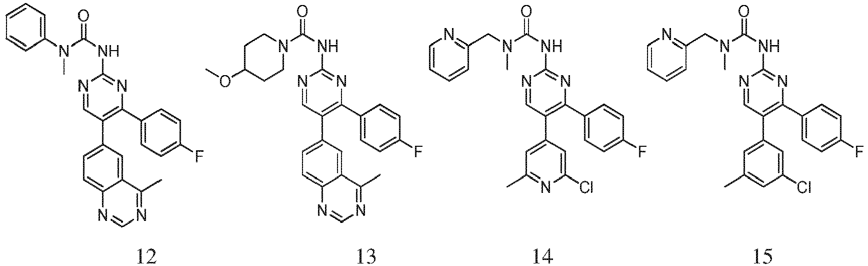

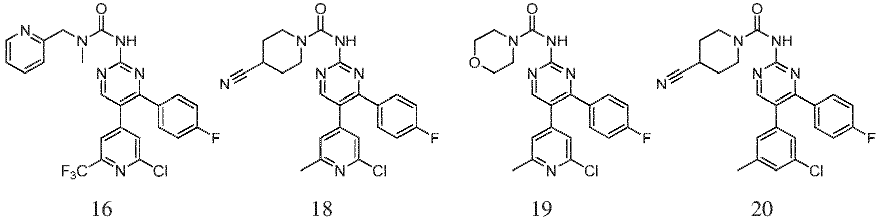

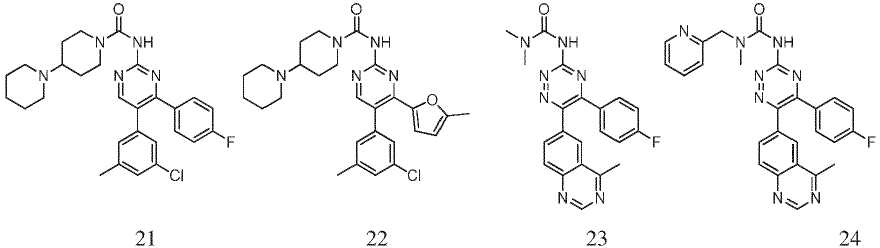

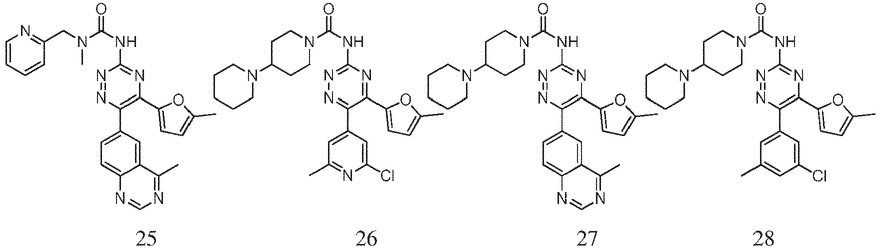

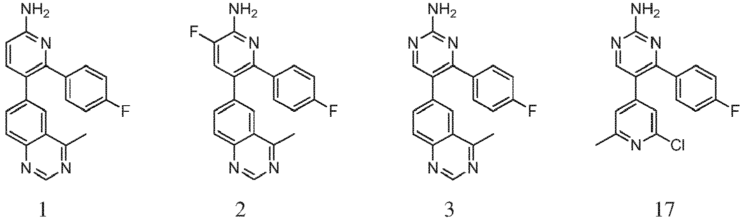

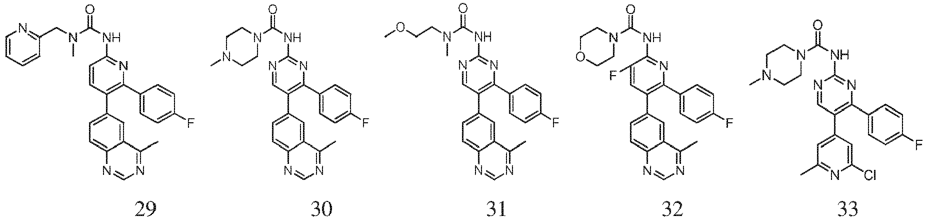

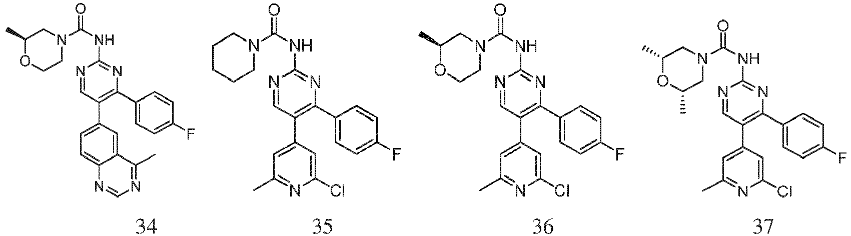

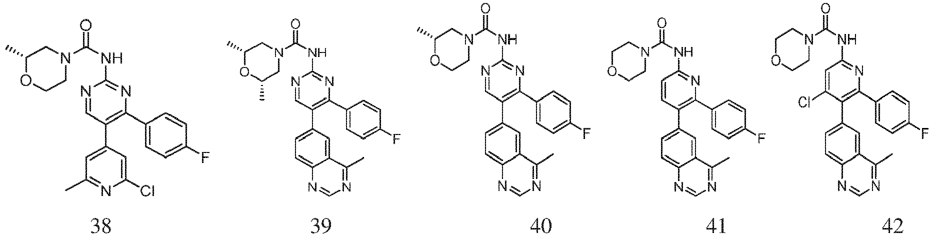

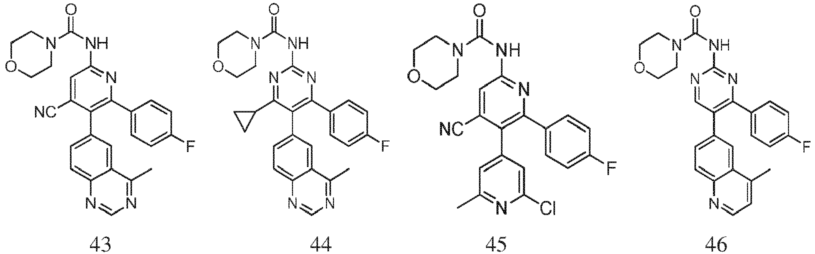

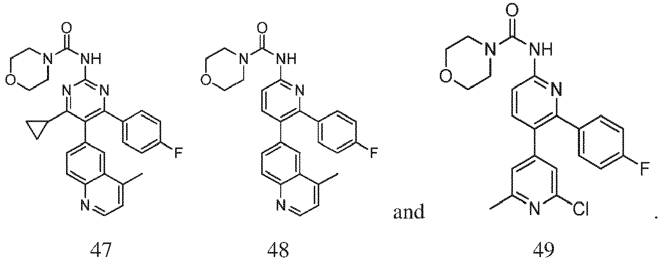

11. A pharmaceutical composition comprising the compound, a pharmaceutically acceptable salt, a stereoisomer, a polymorph, a solvate, a *N*-oxide, an isotope-labeled compound, a metabolite or a prodrug thereof according to any of Claims 1 to 10, and optionally one or more pharmaceutically acceptable carriers or excipients.

12. Use of the compound, a pharmaceutically acceptable salt, a stereoisomer, a polymorph, a solvate, a *N*-oxide, an isotope-labeled compound, a metabolite or a prodrug thereof according to any of Claims 1 to 10, or the pharmaceutical composition according to Claim 10 in manufacture of a medicament as an adenosine A2a receptor antagonist.

13. The compound, a pharmaceutically acceptable salt, a stereoisomer, a polymorph, a solvate, a N-oxide, an isotope-labeled compound, a metabolite or a prodrug thereof according to any of Claims 1 to 10, or the pharmaceutical composition according to Claim 10, for use in the inhibition of the activity of an adenosine A2a receptor.

14. Use of the compound, a pharmaceutically acceptable salt, a stereoisomer, a polymorph, a solvate, a *N*-oxide, an isotope-labeled compound, a metabolite or a prodrug thereof according to any of Claims 1 to 10, or the pharmaceutical composition according to Claim 10 in manufacture of a medicament for the prevention and/or treatment of a disease related to adenosine A2a receptor;
preferably, the disease related to adenosine A2a receptor is a tumor;
preferably, the tumor is selected from the group consisting of: breast cancer, ovarian cancer, colorectal cancer, melanoma, non-small cell lung cancer, small cell lung cancer, gastrointestinal stromal tumor, cervical cancer, pancreatic cancer, prostate cancer, gastric cancer, chronic myeloid leukocytosis, liver cancer, lymphoma, peritoneal cancer and soft tissue sarcoma.

15. A method for the treatment and/or prevention of a disease related to adenosine A2a receptor comprising administering to a subject in need an effective amount of the compound, a pharmaceutically acceptable salt, a stereoisomer, a polymorph, a solvate, a *N*-oxide, an isotope-labeled compound, a metabolite or a prodrug thereof, or pharmaceutical composition thereof according to any of Claims 1 to 10,
preferably, the disease related to adenosine A2a receptor is a tumor,
preferably, the tumor includes but is not limited to: breast cancer, ovarian cancer, colorectal cancer, melanoma, non-small cell lung cancer, small cell lung cancer, gastrointestinal stromal tumor, cervical cancer, pancreatic cancer, prostate cancer, gastric cancer, chronic myeloid leukocytosis, liver cancer, lymphoma, peritoneal cancer and soft tissue sarcoma.

16. The compound, a pharmaceutically acceptable salt, a stereoisomer, a polymorph, a solvate, a *N-*oxide, an isotope-labeled compound, a metabolite or a prodrug thereof, or pharmaceutical composition thereof according to any of Claims 1 to 10, for use in the treatment and/or prevention of a disease related to adenosine A2a receptor,
preferably, the disease related to adenosine A2a receptor is a tumor,
preferably, the tumor includes but is not limited to: breast cancer, ovarian cancer, colorectal cancer, melanoma, non-small cell lung cancer, small cell lung cancer, gastrointestinal stromal tumor, cervical cancer, pancreatic cancer, prostate cancer, gastric cancer, chronic myeloid leukocytosis, liver cancer, lymphoma, peritoneal cancer and soft tissue sarcoma.
